(19) Europäisches Patentamt
European Patent Office
Office européen des brevets

(11) EP 2 582 655 B1

(12) # EUROPEAN PATENT SPECIFICATION

(45) Date of publication and mention of the grant of the patent:
**30.03.2022 Bulletin 2022/13**

(21) Application number: **11795855.3**

(22) Date of filing: **14.06.2011**

(51) International Patent Classification (IPC):
***C07C 69/28*** (2006.01) ***C07C 69/013*** (2006.01)
***C07C 69/63*** (2006.01) ***C07D 495/04*** (2006.01)
***C07D 495/22*** (2006.01) ***H01L 21/336*** (2006.01)
***H01L 29/786*** (2006.01) ***H01L 29/80*** (2006.01)
***H01L 51/05*** (2006.01) ***H01L 51/30*** (2006.01)
***H01L 51/40*** (2006.01)

(52) Cooperative Patent Classification (CPC):
(C-Sets available)
**C07C 69/013; C07C 1/213; C07C 17/357; C07C 17/363; C07C 69/24; C07C 69/28; C07C 69/63; C07D 495/04; C07D 495/22; C09D 5/24; C09D 11/52; H01L 51/0004; H01L 51/0005; H01L 51/0056; H01L 51/0058;**
(Cont.)

(86) International application number:
**PCT/JP2011/063999**

(87) International publication number:
**WO 2011/158953 (22.12.2011 Gazette 2011/51)**

(54) **METHOD FOR PRODUCING FILM-LIKE PRODUCT COMPRISING PI-ELECTRON CONJUGATED COMPOUND**

VERFAHREN ZUR HERSTELLUNG EINES FILM-ÄHNLICHEN PRODUKTS, UMFASSEND PI-ELEKTRONEN-KONJUGIERTE VERBINDUNG

PROCÉDÉ DE PRODUCTION D'UN PRODUIT EN FILM, COMPRENANT UN COMPOSÉ CONJUGUÉ À ÉLECTRONS PI

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **15.06.2010 JP 2010136363**
**31.03.2011 JP 2011080630**
**11.04.2011 JP 2011086973**

(43) Date of publication of application:
**24.04.2013 Bulletin 2013/17**

(73) Proprietor: **Ricoh Company, Ltd.**
**Tokyo 143-8555 (JP)**

(72) Inventors:
- **GOTO, Daisuke**
  **Tokyo 143-8555 (JP)**
- **YAMAMOTO, Satoshi**
  **Tokyo 143-8555 (JP)**
- **SAGISAKA, Toshiya**
  **Tokyo 143-8555 (JP)**
- **KATO, Takuji**
  **Tokyo 143-8555 (JP)**
- **TANO, Takanori**
  **Tokyo 143-8555 (JP)**
- **SHINODA, Masato**
  **Tokyo 143-8555 (JP)**
- **MATSUMOTO, Shinji**
  **Tokyo 143-8555 (JP)**
- **MOHRI, Masataka**
  **Tokyo 143-8555 (JP)**
- **YUTANI, Keiichiro**
  **Tokyo 143-8555 (JP)**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

(74) Representative: **Fairbairn, Angus Chisholm et al**
**Marks & Clerk LLP**
**15 Fetter Lane**
**London EC4A 1BW (GB)**

(56) References cited:
**WO-A1-2004/079834** **WO-A1-2011/030918**
**JP-A- 2006 352 143** **JP-A- 2009 302 407**
**JP-A- 2011 071 501**

- **S NAGARAJA RAO ET AL: "Acid-Catalyzed Aromatizations of Arene Oxides and Arene Hydrates: Are Arene Oxides Homoaromatic?", J. AM. CHEM. SOC, vol. 115, 1 January 1993 (1993-01-01), pages 5458-5465, XP055174745,**
- **Francis Brion: "ON THE LEWIS ACID CATALYZED DIELS-ALDER REACTION OF FURAN. REGIO- AND STEREOSPECIFIC SYNTHESIS OF SUBSTITUTED CYCLOHEXENOLS AND CYCLOHEXADIENOLS.", Tetrahedron Letters,Vol.23,No.5O,pp, 1 January 1982 (1982-01-01), pages 5299-5302, XP055078427, Retrieved from the Internet: URL:http://ac.els-cdn.com/S004040390085823 2/1-s2.0-S0040403900858232-main.pdf?_tid=c ca4e8a4-19f8-11e3-a2ff-00000aab0f6b&acdnat =1378804392_d1aa643d66c2c41530b0e90539ffd 6 1e [retrieved on 2013-09-10]**
- **Yoshiyuki Kikuchi ET AL: "Regiospecific Amination of Veratrole via o-Benzoquinone Bis(dimethy1 acetal )", J. CHEM. SOC., CHEM. COMMUN, 1 January 1982 (1982-01-01), pages 878-879, XP055157365, Retrieved from the Internet: URL:http://pubs.rsc.org/en/content/article pdf/1982/c3/c39820000878 [retrieved on 2014-12-08]**
- **GERALD DYKER ET AL: "Mitt. XV: Struktur und reaktivität von isoanellierten heterocyclischen systemen mit 4n[pi]- und (4n + 2) [pi]-elektronen. 2- t -butyl-4-methyl-2,4-dihydropyrrolo[3,4- b ]indole. reaktionen mit aktivierten CC-dienophilen", JOURNAL OF HETEROCYCLIC CHEMISTRY, vol. 26, no. 4, 1 July 1989 (1989-07-01), pages 941-945, XP055157362, ISSN: 0022-152X, DOI: 10.1002/jhet.5570260411**
- **AXEL JACOBI VON WANGELIN ET AL: "Unusual Coupling Reactions of Aldehydes and Alkynes: A Novel Preparation of Substituted Phthalic Acid Derivatives by Automated Synthesis", CHEMISTRY - A EUROPEAN JOURNAL, vol. 9, no. 10, 23 May 2003 (2003-05-23), pages 2273-2281, XP055174728, ISSN: 0947-6539, DOI: 10.1002/chem.200204668**
- **M.A.CONSTENLA ET AL.: 'Mixed esters of trans-9,10-dihydroxy-9,10-dihydrophenanthre ne' REVISTA LATINOAMERICANA DE QUIMICA vol. 13, no. 3-4, 1982, pages 117 - 119, XP008169228**
- **D.P.KELLY ET AL.: 'Proton-carbon-13 coupling constants in carbocations. 6. Generation and trapping of the (1aalpha,7aalpha)-1a,2,7,7a-tetrahydro -1H-cyclopropa[b]naphthalen-2-yl cation' JOURNAL OF ORGANIC CHEMISTRY vol. 56, no. 6, 1991, pages 2040 - 2045, XP055072406**
- **AKHTAR M.N. ET AL.: 'Anthracene 1,2-oxide: synthesis and role in the metabolism of anthracene by mammals' JOURNAL OF THE CHEMICAL SOCIETY, PERKIN TRANSACTIONS vol. 1, no. 6, 1979, pages 1442 - 1446, XP055072407**
- **Ko Hoon Kim ET AL: "Construction of a Tetracyclic Butterfly-Like Scaffold: Palladium-Catalyzed Heck/Arylation Cascade", Chemistry - A European Journal, 2010, 16, 27 January 2010 (2010-01-27), pages 2375-2380, XP055690871, Retrieved from the Internet: URL:https://chemistry-europe.onlinelibrary .wiley.com/doi/epdf/10.1002/chem.200903029 [retrieved on 2020-04-30]**

(52) Cooperative Patent Classification (CPC): (Cont.)
**H01L 51/0074;** C07C 2602/10; C07C 2603/26; C07C 2603/50; C07C 2603/52; C07C 2603/54; H01L 51/001; H01L 51/0558

C-Sets
**C07C 1/213, C07C 15/20;**
**C07C 1/213, C07C 15/60;**
**C07C 17/357, C07C 25/22;**
**C07C 17/363, C07C 25/22**

## Description

### Technical Field

The present disclosure describes a leaving substituent-containing compound which is synthesized in a simple manner, which has high solubility to an organic solvent, and which can be thermally converted by energy at lower temperatures than in conventional cases; an ink and an organic film each containing the leaving substituent-containing compound; an organic semiconductor material containing a specific compound produced from the leaving substituent-containing compound; and an organic electronic device, an organic thin-film transistor and a display device using the organic semiconductor material.

The present invention relates to a method for producing a film-like product containing a π-electron conjugated compound having an aromatic ring (e.g., a benzene ring), the π-electron conjugated compound being produced by eliminating specific substituents from a π-electron conjugated compound precursor which has a cyclohexadiene ring, which is synthesized in a simple manner, which has high solubility to an organic solvent, and which can be thermally converted by energy at lower temperatures than in conventional cases; and a method for producing the π-electron conjugated compound at high yield in a simple manner. The methods of the present invention are useful in the production of organic electronics such as organic electronic devices (organic electroluminescence (EL) elements, organic semiconductors and organic solar cells) as well as the production of films of organic pigments and organic dyes.

### Background Art

In recent years, organic thin-film transistors using organic semiconductor materials have been intensively studied and developed.

Hitherto, organic semiconducor materials of low molecular weight have been reported, such as acene materials (e.g., pentacene) (see, for example, PTL 1 and NPL 1).

It has been reported that the organic thin-film transistors including an organic semiconductive layer formed of the aforementioned pentacene has relatively high charge mobility. However, these acene materials have extremely low solubility to common solvents. Therefore, these materials need to be vacuum-deposited to form a thin film as an organic semiconductive layer of an organic thin-film transistor. For this reason, these materials do not meet the demand in the art, which is to provide an organic semiconductor material that can be formed into a thin film by a simple wet process such as coating or printing.

As one of the acene-based materials such as pentacene, 2,7-diphenyl[1]benzothieno[3,2-b][1]benzothiophene having the following Structural Formula (1) (see PTL 2 and NPL 2), which is a derivative of benzothieno[3,2-b]benzothiophene, is deposited on a substrate having been treated with octadecyltrichlorosilane, so that the deposited product exhibits a mobility comparable to that of pentacene (approximately 2.0 $cm^2/V \cdot s$) and has prolonged stability in the atmosphere. However, this compound also needs to be vacuum-deposited similar to pentacene. Thus, this material also does not meet the demand in the art, which is to provide an organic semiconductor material that can be formed into a thin film by a simple process such as coating or printing.

The organic semiconductor materials can be easily formed into a thin film by a simple process such as a wet process, for example, printing, spin coating, ink jetting, or the like. The thin-film transistors using organic semiconductor materials also have an advantage over those using inorganic semiconductor materials in that the temperature of the production process can be lowered. Thus, a film can be formed on a plastic substrate having a generally low heat resistance, so that electronic devices such as displays can be reduced in weight and cost. Further, the electronic devices are expected to be widely used by taking advantage of flexibility of the plastic substrate.

Moreover, 2,7-dialkyl[1]benzothieno[3,2-b][1]benzothiophene represented by the following General Formula (2), having liquid crystallinity and high solubility, can be applied by spin coating or casting (see PTL 2 and NPL 3). This compound is also a derivative which exhibits a mobility comparable to that of pentacene (approximately 2.0 $cm^2/V \cdot s$) when thermally treated at a temperature equal to or lower than the temperature at which the compound shows a liquid crystal phase (about 100°C).

However, the temperature at which this compound shows a liquid crystal phase is relatively low; i.e., about 100°C, and the film formed therefrom may be changed through thermal treatment after film formation. Thus, this compound poses a problem in process adaptability in production of organic semiconductor devices.

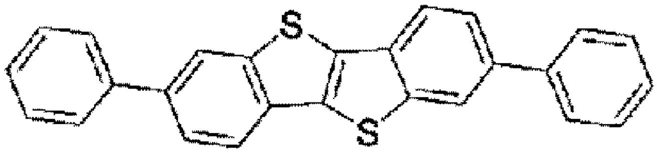

(1)

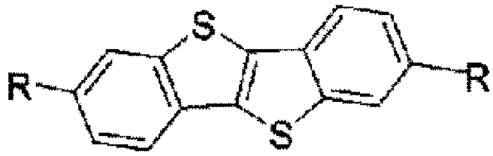

(2)

In recent years, a method of producing a field-effect transistor is reported, wherein a low-molecular-weight compound having high solvent solubility is used as a semiconductor precursor, which is dissolved in a solvent and the like, and applied so as to form a film by a coating process, and then the film is transformed to an organic semiconductor film. Intensive studies have been made on methods of converting the precursor to pentacene, a porphyrin-based compound, and a phthalocyanine-based compound through retro-Diels-Alder reaction (see, for example, PTLs 3 to 9 and NPLs 4 to 7).

As described in NPL 4, the mobility of organic semiconductor materials depends on the orderly molecular arrangement (e.g., crystallization) in organic material films. When a vapor-deposition method is employed, the molecular arrangement of the materials in the films can be surely obtained. Meanwhile, the organic materials with molecular arrangement generally have a low solubility to an organic solvent. That is, in the organic material films, the semiconductive property and film formability (through coating) are generally in a trade-off relation. Thus, in only one possible method for attaining both satisfactorily, after a coating film has been formed from a coating liquid containing a semiconductor precursor having a solvent solubility-imparting group, the precursor in the coating film is converted to an organic semiconductor material.

However, in the above-described example, a tetrachlorobenzene molecule or other molecules are eliminated from the pentacene precursor. Here, tetrachlorobenzene has a high boiling point and is hard to be removed from the reaction system. Additionally, there is concern for its toxicity. Also, both porphylin and phthalocyanine require complicate syntheses, and thus are used in narrow applications.

Therefore, there is a need to develop a substituent-containing compound (semiconductor precursor having a solubility-imparting group) that can be synthesized in a simple manner.

Also, it has been proposed that, by applying an external stimulus to a precursor having a high solvent-solubility and sulfonate-based substituents so that the substituents are eliminated and substituted with hydrogen atoms, the precursor is converted to phthalocyanine (see, for example, PTLs 10 and 11).

However, in this method, the sulfonate-based substituents have a high polarity and thus do not have sufficient solubility to an organic solvent having no polarity. The temperature for conversion of the precursor is relatively high; i.e., at least 250°C to 300°C or higher, which is disadvantageous.

Also, it has been proposed that an alkyl group-containing carboxylate is introduced to the end $\beta$-position of an oligothiophene for imparting solubilization, and then heat is applied to eliminate the carboxylate, thereby obtaining an olefin-substituted oligthiophene or an olefin-substituted [1]benzothieno[3,2-b][1]benzothiophene (see, for example, PTLs 12 and 13 and NPL 7). In this method, the elimination occurs by heating to about 150°C to about 250°C, and the converted compound has at its ends olefin groups (e.g., a vinyl group and a propenyl group) which involve cis-trans isomerization due to heat or light. Thus, the resultant material is problematically degraded in purity and/or crystallinity. In addition, such highly reactive olefin end groups allow the compound to decrease in stability to oxygen or water. Furthermore, one olefin group is thermally polymerized with another olefin group at higher temperatures.

The present inventors conducted extensive studies and have found that the above existing problems can be solved by using a leaving substituent-containing compound with a cyclohexene skeleton having as a leaving substituent an acyloxy group (specifically, carboxylic acid ester). Specifically, after elimination of the substituent, such a leaving substituent-containing compound is converted to have a benzene ring structure instead of the aforementioned olefin group (e.g., an olefin-substituted oligthiophene or an olefin-substituted [1]benzothieno[3,2-b][1]benzothiophene), thereby involving no cis-trans isomerization. However, the temperature (energy) at which the leaving substituent is removed from the leaving substituent-containing compound is typically about 150°C to about 250°C, and thus there are still problems in using low-heat-resistant substrates made of plastics, etc.

The above-described conventional compounds pose problems in solubility of their precursors, stability of eliminated components, conversion temperature, and stability of the compounds obtained after conversion. In addition, it is difficult to obtain a desired intermediate during synthesis thereof.

$\pi$-Electron conjugated compounds, having a moiety in which double bonds and single bonds are alternatingly located, have a highly extended n-electron conjugation system, and thus, are excellent in hole transportability and electron transportability. Thus, such $\pi$-electron conjugated compounds have been used as electroluminescence materials and organic semiconductor materials (see, for example, PTLs 1 and 2 and NPLs 1 and 2) as well as organic dyes and pigments. The $\pi$-electron conjugated compounds widely used involve the following problem, for example. Specifically, most of the $\pi$-electron conjugated compounds are rigid and highly planar, and thus the intermolecular interaction is very strong. As a result, these compounds have poor solubility to water or organic solvents. For example, the organic pigments

made of such conjugated compounds are unstable in dispersion due to aggregation of the pigments. Also, taking an example electroluminescence materials and organic semiconductor materials made of such conjugated compounds, a wet process (using a solution) is difficult to employ since the conjugated compounds are sparingly soluble. As a result, vapor phase-film formation (e.g., vacuum vapor deposition) is required to elevate the production cost and complicate the production process which is disadvantageous. Considering the coating on a larger area and the attainment of higher efficiency, the π-electron conjugated compounds are required to be applicable to wet processes using a coating liquid previously prepared by dissolving materials in a solvent (e.g., spin coating, blade coating, gravure printing, inkjet coating and dip coating). Meanwhile, the fact that intermolecular contact, rearrangement, aggregation and crystallization are easily attained since intermolecular interaction is very strong contributes to conductivity of the compounds. In general, the film-formability and the conductivity of the obtained film are often in a trade-off relation. This is one cause making difficult to employ the π-electron conjugated compounds.

In order to overcome the above-described problems, it has been proposed that an external stimulus is applied to an organic compound precursor (including π-electron conjugated compound precursors) having reactive substituents (which impart the solubility to the precursor) to thereby eliminate the substituents to obtain a compound of interest (see, for example, PTLs 14 and 15 and NPL 8). In this method, for example, a pigment precursor having a structure in which an amino group or an alcoholic or phenolic hydroxyl group is modified with a t-butoxycarbonyl group (a t-Boc group) is heated or treated otherwise to thereby eliminate the t-Boc group. However, some limitation is imposed on the compound employable in this method, since the substituent must be bonded to the nitrogen atom or oxygen atom. In addition, further improvement has been required in terms of stability of the precursor.

Meanwhile, in recent years, intensive studies have been made on a method of applying an external stimulus to a precursor having solvent-soluble bulky substituents so that the solvent-soluble bulky substituents are eliminated, and converting the precursor to a pentacene, a porphyrin-based compound, and a phthalocyanine-based compound (see, for example, PTLs 3, 4, 6, 7, 8 and 9 and NPLs 4, 5, 6 and 7).

However, in the above-described example, a tetrachlorobenzene molecule or other molecules are eliminated from the pentacene precursor. Here, tetrachlorobenzene has a high boiling point and is hard to be removed from the reaction system. Additionally, there is concern for its toxicity. Also, both porphylin and phthalocyanine require complicate syntheses, and thus are used in narrow applications. Therefore, there is a need to develop a substituent-containing compound that can be synthesized in a simple manner.

Also, it has been proposed that, by applying external stimulus to a precursor having a high solvent-solubility and sulfonate-based substituents so that the substituents are eliminated and substituted with hydrogen atoms, whereby the precursor is converted to phthalocyanine (see, for example, PTLs 10 and 11).

However, in this method, the sulfonate-based substituents have a high polarity and thus do not have sufficient solubility to an organic solvent having no polarity. In addition, the temperature for conversion of the precursor is relatively high; i.e., 250°C to 300°C, which is disadvantageous.

Also, it has been proposed that an alkyl group-containing carboxylate is introduced to the end β-position of an oligothiophene for imparting solubilization, and then heat is applied to eliminate the carboxylate, thereby obtaining an olefin-substituted oligthiophene or an olefin-substituted [1]benzothieno[3,2-b][1]benzothiophene (see, for example, PTLs 12 and 13 and NPL 7). In this method, the elimination occurs by heating to about 150°C to about 250°C, and the converted compound has at its ends olefin groups (e.g., a vinyl group and a propenyl group) which involve cis-trans isomerization due to heat or light. Thus, the resultant material is problematically degraded in purity and/or crystallinity. In addition, such highly reactive olefin end groups allow the compound to decrease in stability to oxygen or water. Furthermore, one olefin group is thermally polymerized with another olefin group at higher temperatures.

The above-described conventional compounds pose problems in solubility of their precursors, stability of eliminated components, conversion temperature, and stability of the compounds obtained after conversion. In addition, it is difficult to obtain a desired intermediate during synthesis thereof.

The present inventors conducted extensive studies and have found that the above existing problems can be solved by using a π-electron conjugated compound precursor (precursor) with a cyclohexene skeleton having as a leaving substituent an acyloxy group (specifically, carboxylic acid ester). Specifically, after elimination of the substituent, such a leaving substituent-containing compound is converted to have a benzene ring structure instead of the aforementioned olefin group (e.g., an olefin-substituted oligthiophene or an olefin-substituted [1]benzothieno[3,2-b][1]benzothiophene), thereby involving no cis-trans isomerization. The present inventors have previously disclosed the above described finding in International Publication No. WO2011030918.

However, the temperature (energy) at which the leaving substituent is removed from the precursor is typically about 150°C to about 250°C, and thus there are still problems in using low-heat-resistant substrates made of plastics, etc.

PTL 16 relates to a crystalline organic thin film and a method for producing the same, and to a method for producing a crystalline organic thin film that can be used as an organic semiconductor.

Citation List

Patent Literature

PTL 1: Japanese Patent Application Laid-Open (JP-A) No. 05-055568
PTL 2: International Publication No. WO/2006-077888 PTL 3: JP-A No. 2007-224019
PTL 4: JP-A No. 2008-270843
PTL 5: JP-A No. 2009-105336
PTL 6: JP-A No. 2009-188386
PTL 7: JP-A No. 2009-215547
PTL 8: JP-A No. 2009-239293
PTL 9: JP-A No. 2009-283943
PTL 10: JP-A No. 2009-84555
PTL 11: JP-A No. 2009-88483
PTL 12: JP-A No. 2006-352143
PTL 13: JP-A No. 2009-275032
PTL 14: JP-A No. 07-188234
PTL 15: JP-A No. 2008-226959
PTL 16: JP A No. 2009-302407

Non Patent Literature

NPL 1: Appl. Phys. Lett. 72, p. 1854 (1998)
NPL 2: J. Am. Chem. Soc. 128, p. 12604 (2006)
NPL 3: J. Am. Chem. Soc. 129, p. 15732 (2007)
NPL 4: Adv. Mater., 11, p. 480 (1999)
NPL 5: J. Appl. Phys. 100, p. 034502 (2006)
NPL 6: Appl. Phys. Lett. 84, 12, p. 2085 (2004)
NPL 7: J. Am. Chem. Soc. 126, p. 1596 (2004)
NPL 8: Nature, 388, p. 131, (1997)

Summary of Invention

Technical Problem

Disclosed herein is a leaving substituent-containing compound which is synthesized in a simple manner, which has high solubility to an organic solvent, and whose leaving substituent can be removed by energy (hereinafter may be referred to as "external stimulus) lower than in conventional cases; an ink (including a coating liquid) and an organic film each containing the leaving substituent-containing compound; an organic semiconductor material containing a specific compound (an organic semiconductor compound) produced with high yield from the leaving substituent-containing compound through application of external stimulus (e.g., heat) lower than in conventional cases, while forming a eliminated component and not forming chemically unstable end olefin group; and an organic electronic device (especially, an organic thin-film transistor) and a display device containing a sparingly-soluble continuous film of the organic semiconductor material formed through a wet process by converting a film of the leaving substituent-containing compound (serving as an organic semiconductor precursor) to an organic semiconductor with heat, etc.

Herein disclosed is a method for producing a $\pi$-electron conjugated compound having a benzene ring, while forming an eliminated component and not forming chemically unstable end olefin group, by applying energy (hereinafter may be referred to as "external stimulus), such as heat, to a $\pi$-electron conjugated compound precursor which is synthesized in a simple manner, which has high solubility to an organic solvent, and whose leaving group can be eliminated by energy lower than in conventional cases. Using the above technique, the present invention aims to provide a method for efficiently producing a sparingly-soluble continuous thin film of the $\pi$-electron conjugated compound and application of this thin film to an organic electronic device (especially, an organic thin-film transistor).

Solution to Problem

Means for solving the above existing problems are as follows.

<1> A method for producing a film-like product, comprising:

forming a coating film on a substrate by coating the substrate with a coating liquid containing in a solvent a π-electron conjugated compound precursor represented by A-(B)m, and
eliminating an eliminated component represented by General Formula (II) to form a π-electron conjugated compound represented by A-(C)m in the coating film,

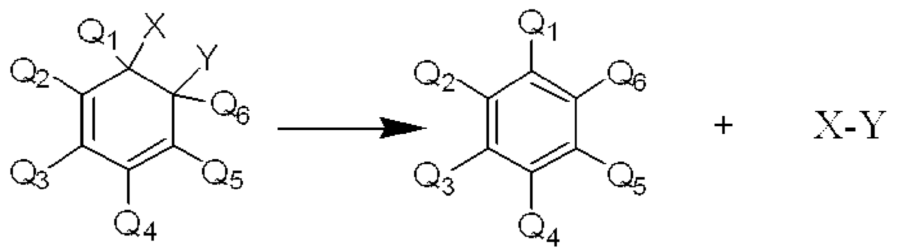

(I) (Ia) (II)

wherein in A-(B)m and A-(C)m, A represents a π-electron conjugated substituent, B represents a solvent-soluble substituent containing a structure represented by General Formula (I) as at least a partial structure, and m is a natural number,
wherein the solvent-soluble substituent represented by B is linked via a covalent bond with the π-electron conjugated substituent represented by A where the covalent bond is formed between an atom present on $Q_1$ to $Q_6$ and an atom present on the π-electron conjugated substituent represented by A or the solvent-soluble substituent represented by B is ring-fused with the π-electron conjugated substituent represented by A via atoms present on the π-electron conjugated substituent represented by A, and C represents a substituent containing a structure represented by General Formula (Ia) as at least a partial structure, and
wherein in General Formulas (I), (Ia) and (II), X and Y each represent a hydrogen atom, a substituted or unsubstituted ether group having 1 to 38 carbon atoms or a substituted or unsubstituted acyloxy group having 1 to 38 carbon atoms, where one of X and Y is the ether or acyloxy group and the other is the hydrogen atom; $Q_2$ to $Q_5$ each represent a hydrogen atom or a monovalent organic group; $Q_1$ and $Q_6$ each represent a hydrogen atom, a halogen atom, an alkyl group, an alkenyl group, an alkynyl group, an aryl group or a heteroaryl group; and among the monovalent organic groups represented by $Q_1$ to $Q_6$, adjacent monovalent organic groups may be linked together to form a ring, and
wherein the monovalent organic group is any one of an alkyl group, an alkenyl group, an alkynyl group, an aryl group and a heteroaryl group, wherein the heteroaryl group is selected from a thiophene ring, a pyridine ring, a pyrazine ring, a pyrimidine ring, a triazine ring, a pyrrol ring, a pyrazole ring, an imidaziole ring, a triazole ring, an oxazole ring, a thiazole ring and a furan ring.

<2> The method for producing a film-like product according to <1>, wherein the substrate is coated with the coating liquid by a method selected from the group consisting of inkjet coating, spin coating, solution casting and dip coating.
<3> The method for producing a film-like product according to <1> or <2>, wherein the substituent represented by A is at least one π-electron conjugated compound selected from the group consisting of (i) compounds in which one or more aromatic hydrocarbon rings are ring-fused with one or more aromatic heterocyclic rings, compounds in which two or more aromatic hydrocarbon rings are ring-fused together, and compounds in which two or more aromatic heterocyclic rings are ring-fused together; and (ii) compounds in which one or more aromatic hydrocarbon rings are linked via a covalent bond with one or more aromatic heterocyclic rings, compounds in which two or more aromatic hydrocarbon rings are linked together via a covalent bond, and compounds in which two or more aromatic heterocyclic rings are linked together via a covalent bond.
<4> The method for producing a film-like product according to any one of <1> to <3>, wherein the eliminated component represented by General Formula (II) and eliminated from the compound represented by A-(B)m includes a hydrogen halide, a substituted or unsubstituted carboxylic acid, a substituted or unsubstituted alcohol or carbon dioxide.
<5> The method for producing a film-like product according to any one of <1> to <4>, wherein the compound represented by A-(B)m has a solvent solubility, and the compound represented by A-(C)m and formed after elimination of the leaving substituent has a solvent insolubility.
<6> The method for producing a film-like product according to any one of claims 1 to 5, wherein the leaving substituent-containing compound represented by General Formula (I) does not have the structure I-(11)

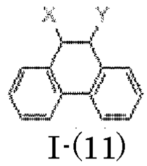

I-(11)

Advantageous Effects of Invention

Disclosed herein is a leaving substituent-containing compound which is synthesized in a simple manner and has high solubility to an organic solvent. Also disclosed herein is an organic semiconductor material having no unstable end-substituent (an olefin group such as a vinyl group or a propenyl group) through elimination reaction of the substituent of the leaving substituent-containing compound. In addition, the elimination reaction of the substituent of the leaving substituent-containing compound can be performed by lower energy (at lower temperatures) than in conventional leaving substituent-containing compounds (e.g., a leaving substituent-containing compound having a cyclohexene skeleton). Also, a solution of the leaving substituent-containing compound (organic semiconductor precursor) and a solvent is coated to form an organic film, followed by elimination reaction, whereby an organic semiconductor film of the organic semiconductor material can be obtained. Use of the organic semiconductor film can provide an organic electronic device (especially, an organic thin-film transistor) and a display device having display pixels driven by the organic thin-film transistor.

The production method of the present invention uses a solvent-soluble $\pi$-electron conjugated compound precursor as a starting material and thus, can be performed through a wet process using a solution. In addition, application of external stimulus such as heat or light to the precursor to eliminate the solvent solubility-imparting substituent can produce a $\pi$-electron conjugated compound having a benzene ring at high yield in a simple manner without forming unstable end substituents. In addition, the elimination reaction of the substituent of the $\pi$-electron conjugated compound precursor can be performed by lower energy (at lower temperatures) than in conventional $\pi$-electron conjugated compound precursors (e.g., a $\pi$-electron conjugated compound precursor having a cyclohexene skeleton). Also, a solution of the $\pi$-electron conjugated compound precursor in a solvent is coated to form an organic film, followed by elimination reaction of the substituent, whereby an organic film of a $\pi$-electron conjugated compound (including an organic semiconductor) can be obtained. Use of the organic film (including an organic semiconductor film) can provide an organic electronic device (especially, an organic thin-film transistor).

Brief Description of Drawings

Fig. 1A schematically illustrates one exemplary configuration of an organic thin-film transistor.
Fig. 1B schematically illustrates another exemplary configuration of an organic thin-film transistor.
Fig. 1C schematically illustrates still another exemplary configuration of an organic thin-film transistor.
Fig. 1D schematically illustrates yet another exemplary configuration of an organic thin-film transistor.
Fig. 2 is a cross-sectional view of one exemplary transistor array for driving a display pixel.
Fig. 3 is a cross-sectional view of another exemplary transistor array for driving a display pixel.
Fig. 4 is a TG-DTA graph showing behaviors of thermal decomposition and phase transition of Ex. 1 compound observed in Example 27.
Fig. 5 is a TG-DTA graph showing behaviors of thermal decomposition and phase transition of Ex. 2 compound observed in Example 28.
Fig. 6A is a photograph of a thin film made of Ex. 1 compound or Ex. 2 compound in Example 29, which is taken with a polarization microscope (open Nicol).
Fig. 6B is a photograph of a thin film made of Ex. 1 compound or Ex. 2 compound in Example 29, which is taken with a polarization microscope (cross Nicol).
Fig. 7 is a current-voltage (I-V) graph of an organic thin-film transistor (FET element) produced in Example 30.

Description of Embodiments

Next, the present invention will be described referring to specific embodiments, which should not be construed as limiting the present invention thereto. The present invention can be variously made without departing the scope of the present invention.

[1. Leaving substituent-containing compound and compound obtained through elimination reaction]

As described above, a leaving substituent-containing compound is represented by the following General Formula (I), and can be converted to a compound represented by the following General Formula (Ia) and a compound represented by the following General Formula (II), by applying energy to the leaving substituent-containing compound.

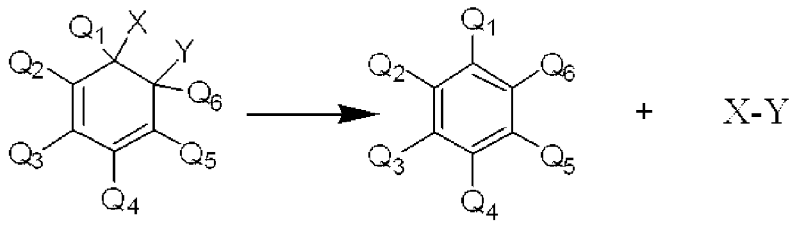

(I) (Ia) (II)

In General Formulas (I), (Ia) and (II), X and Y each represent a hydrogen atom or a leaving substituent, where one of X and Y is the leaving substituent and the other is the hydrogen atom; $Q_2$ to $Q_5$ each represent a hydrogen atom, a halogen atom or a monovalent organic group; $Q_1$ and $Q_6$ each represent a hydrogen atom or a monovalent organic group other than the leaving substituent; and among the monovalent organic groups represented by $Q_1$ to $Q_6$, adjacent monovalent organic groups may be linked together to form a ring.

Specifically, the leaving substituent-containing compound has solubility to a solvent (solvent solubility). When energy (external stimulus) is applied to this compound to eliminate the specific substituent, a target compound can be produced.

The leaving substituent-containing compound represented by the above General Formula (I) contains a cyclohexadiene structure having leaving substituents. By applying energy (external stimulus) thereto, specific leaving substituents X and Y are eliminated from the leaving substituent-containing compound in the form of the compound represented by the above General Formula (II). As a result, the cyclohexadiene structure of the leaving substituent-containing compound is converted to a benzene ring to be the structure represented by the above General Formula (Ia), whereby the corresponding compound can be obtained.

In the above General Formulas (I) and (II), the group represented by X or Y is a hydrogen atom or a leaving substituent. Examples of the leaving substituent include a halogen atom, a hydroxyl group, a substituted or unsubstituted ether group, a substituted or unsubstituted acyloxy group, a substituted or unsubstituted sulfonyloxy group, a nitroxy group, a substituted or unsubstituted phosphooxy group, a substituted or unsubstituted alkylamineoxide group, and groups that are eliminated with the hydrogen atom present on the β carbon (e.g., substituted or unsubstituted polyalkyl quaternary ammonium salts). From the viewpoints of, for example, storage stability of the compound itself, dissolvability to an organic solvent, and conditions for elimination reaction of the substituent (presence or absence of a catalyst, reaction temperature, etc.), preferred are a substituted or unsubstituted ether group, a substituted or unsubstituted acyloxy group and a substituted or unsubstituted sulfonyloxy group. Particularly preferred are a substituted or unsubstituted ether group and a substituted or unsubstituted acyloxy group.

As described above, X and Y are each a hydrogen atom or a substituted or unsubstituted ether group or acyloxy group having 1 or more carbon atoms, and one of X and Y is the leaving substituent (i.e., the substituted or unsubstituted ether group or acyloxy group having 1 or more carbon atoms) and the other is the hydrogen atom.

Examples of the substituted or unsubstituted ether group having 1 or more carbon atoms include ether groups derived from alcohols such as substituted or unsubstituted, linear or cyclic aliphatic alcohols having 1 or more carbon atoms and aromatic alcohols having 4 or more carbon atoms. Further examples include thioether groups obtained by replacing, with a sulfur atom, the oxygen atom in the above ethers. The number of carbon atoms contained in the above ether group is generally 1 to 38, preferably 2 to 22, still more preferably 3 to 18, considering various factors such as dissolvability and the boiling point of an eliminated component.

Specific examples of the ether group include a methoxy group, an ethoxy group, a propoxy group, a butoxy group, an isobutoxy group, a pivaloyl group, a pentoxy group, a hexyloxy group, a lauryloxy group, a trifluoromethoxy group, a 3,3,3-trifluoropropoxy group, a pentafluoropropoxy group, a cyclopropoxy group, a cyclobutoxy group, a cyclohexyloxy group, a trimethylsilyloxy group, a triethylsilyloxy group, a tert-butyldimethylsilyloxy group and a tert-butyldiphenylsilyloxy group. Further examples include thioethers obtained by replacing, with a sulfur atom, the oxygen atom in the ether bonds of the above ether groups.

Examples of the substituted or unsubstituted acyloxy group having 1 or more carbon atoms include a formyloxy group; those derived from linear or cyclic aliphatic carboxylic acids having two or more carbon atoms and optionally

containing a halogen atom and carbonate half esters thereof; and those derived from carboxylic acids such as aromatic carboxylic acids having 4 or more carbon atoms and carbonate half esters thereof. Further examples include acyloxy groups derived from thiocarboxylic acids obtained by replacing, with a sulfur atom, the oxygen atom in the above carboxylic acids. The number of carbon atoms contained in the above acyloxy group is generally 1 to 38, preferably 2 to 22, still more preferably 3 to 18, considering various factors such as dissolvability and the boiling point of an eliminated component.

Specific examples of the acyloxy group include a formyloxy group, an acetoxy group, a propionyloxy group, a butylyloxy group, an isobutylyloxy group, a pivaloyloxy group, a pentanoyloxy group, a hexanoyloxy group, a lauroyloxy group, a stearoyloxy group, a trifluoroacetyloxy group, 3,3,3-trifluoropropionyloxy group, a pentafluoropropionyloxy group, a cyclopropanoyloxy group, a cyclobutanoyloxy group, a cyclohexanoyloxy group, a benzoyloxy group, p-methoxyphenylcarbonyloxy group and a pentafluorobenzoyloxy group.

Additionally, there are exemplified carbonate esters derived from carbonate half esters in which an oxygen atom or sulfur atom is introduced, in the above acyloxy groups, into between their carbonyl groups and their alkyl or aryl groups. Moreover, further examples include acylthiooxy groups and thioacyloxy groups obtained by replacing, with a sulfur atom, one or more oxygen atoms in the ether bonds and carbonyl moieties.

Next will be given some preferred examples of the leaving substituents X and Y as described above.

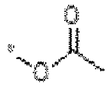 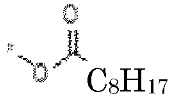 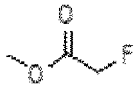 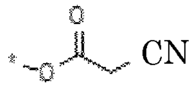

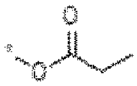 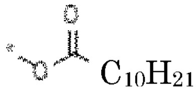 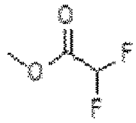 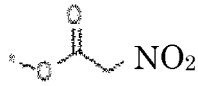

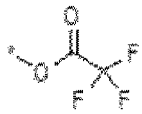 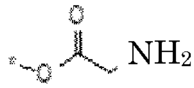 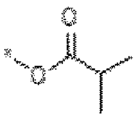 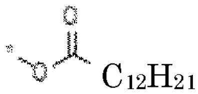 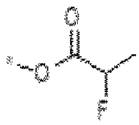 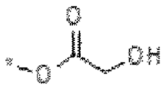

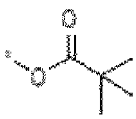 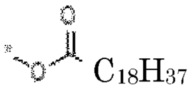 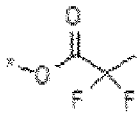 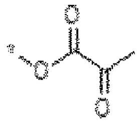

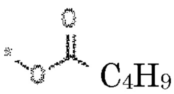 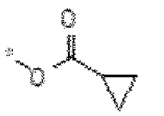 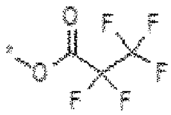 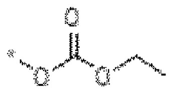

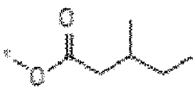 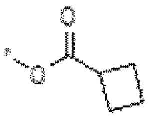 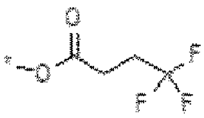 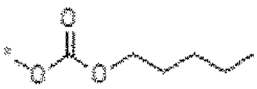

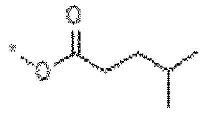
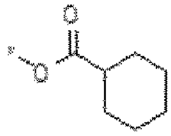
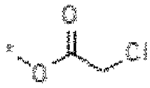
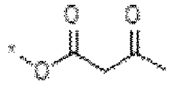
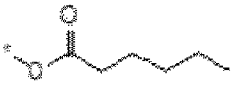
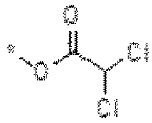
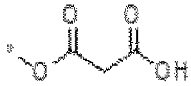
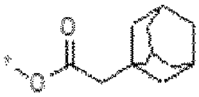
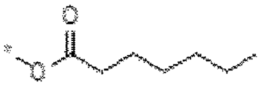
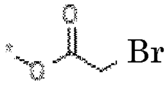
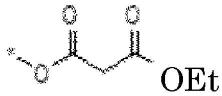
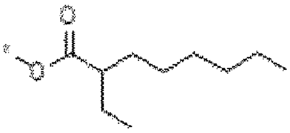
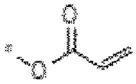
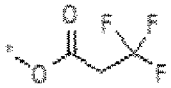
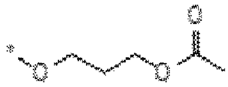
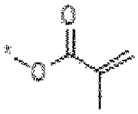
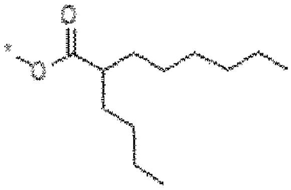
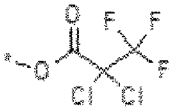
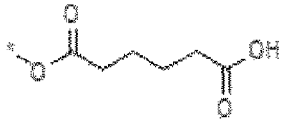
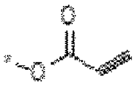
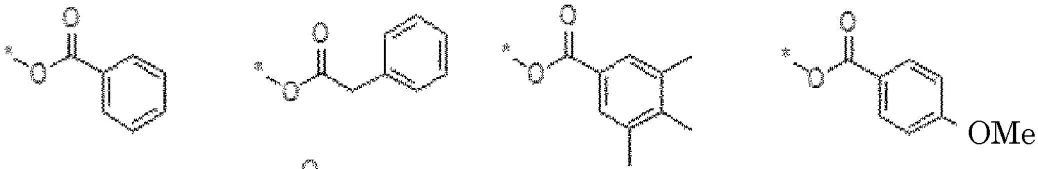
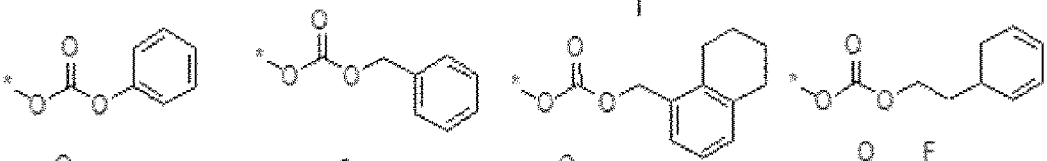
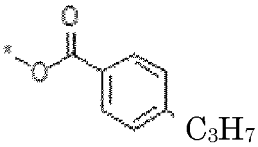
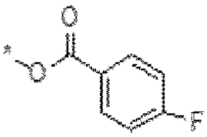
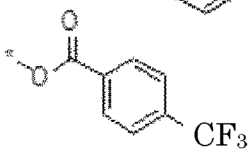
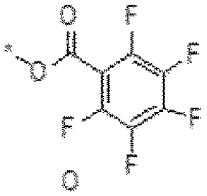
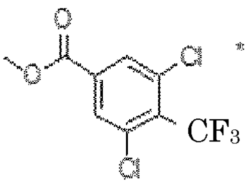
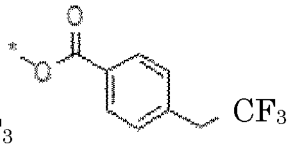
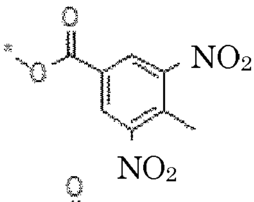
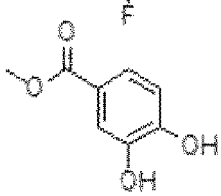
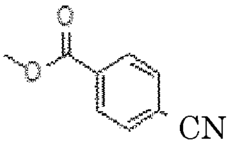
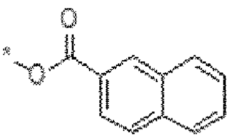
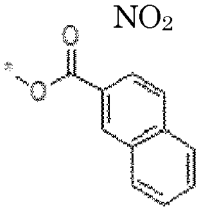
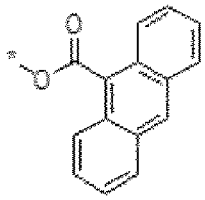
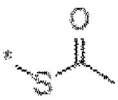
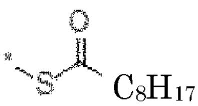
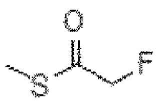
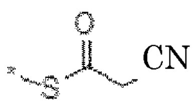

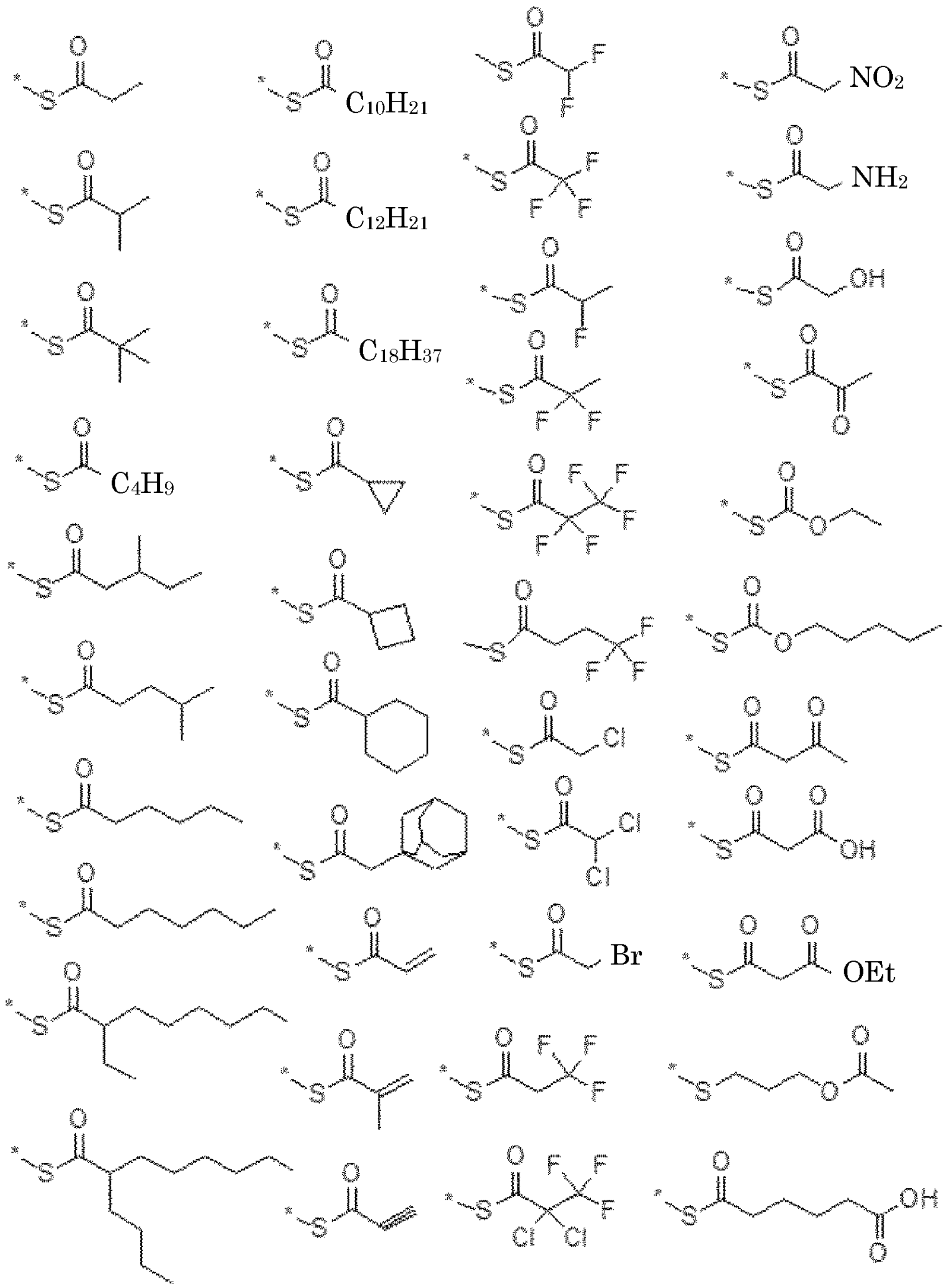

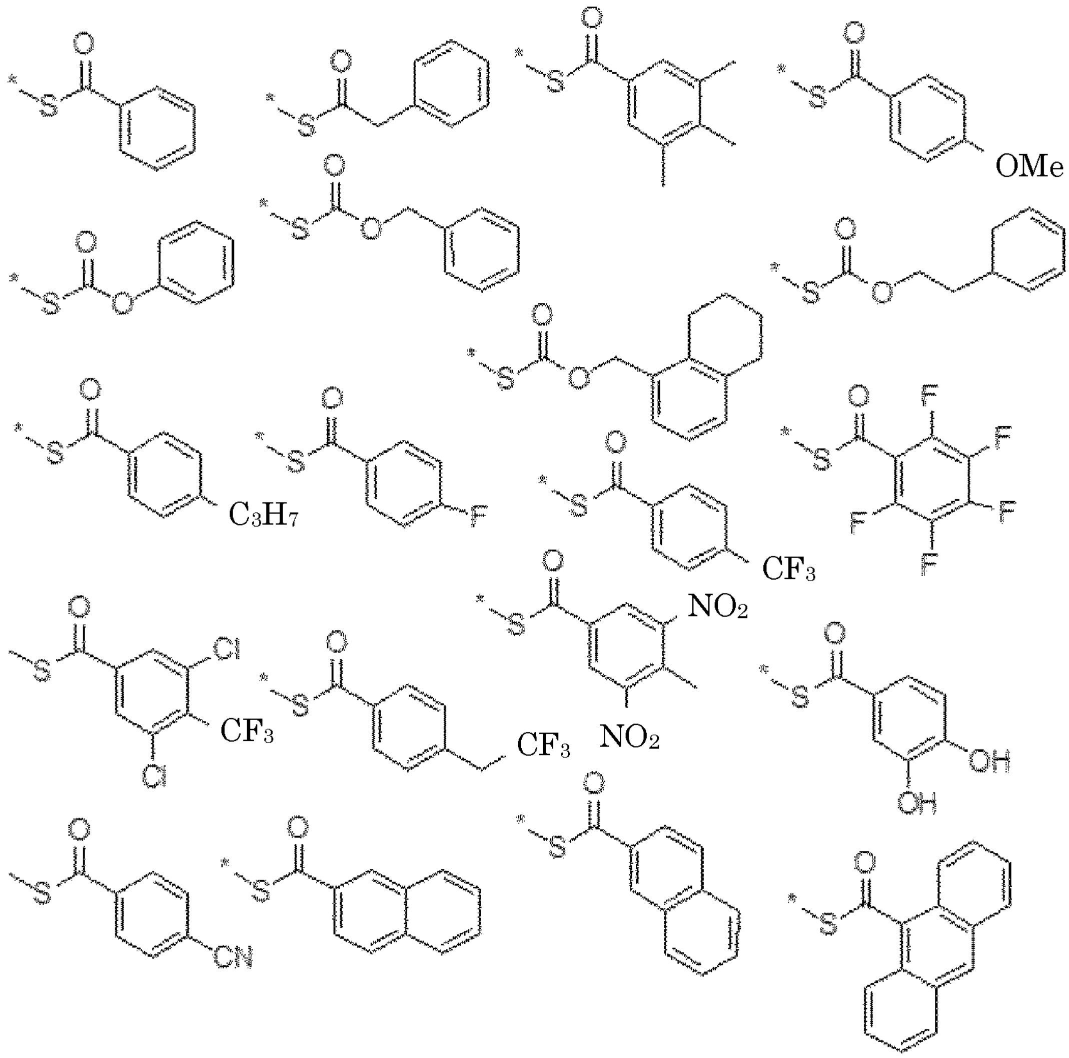

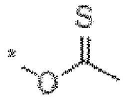

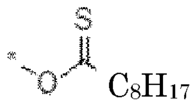

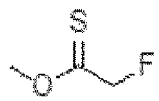

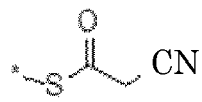

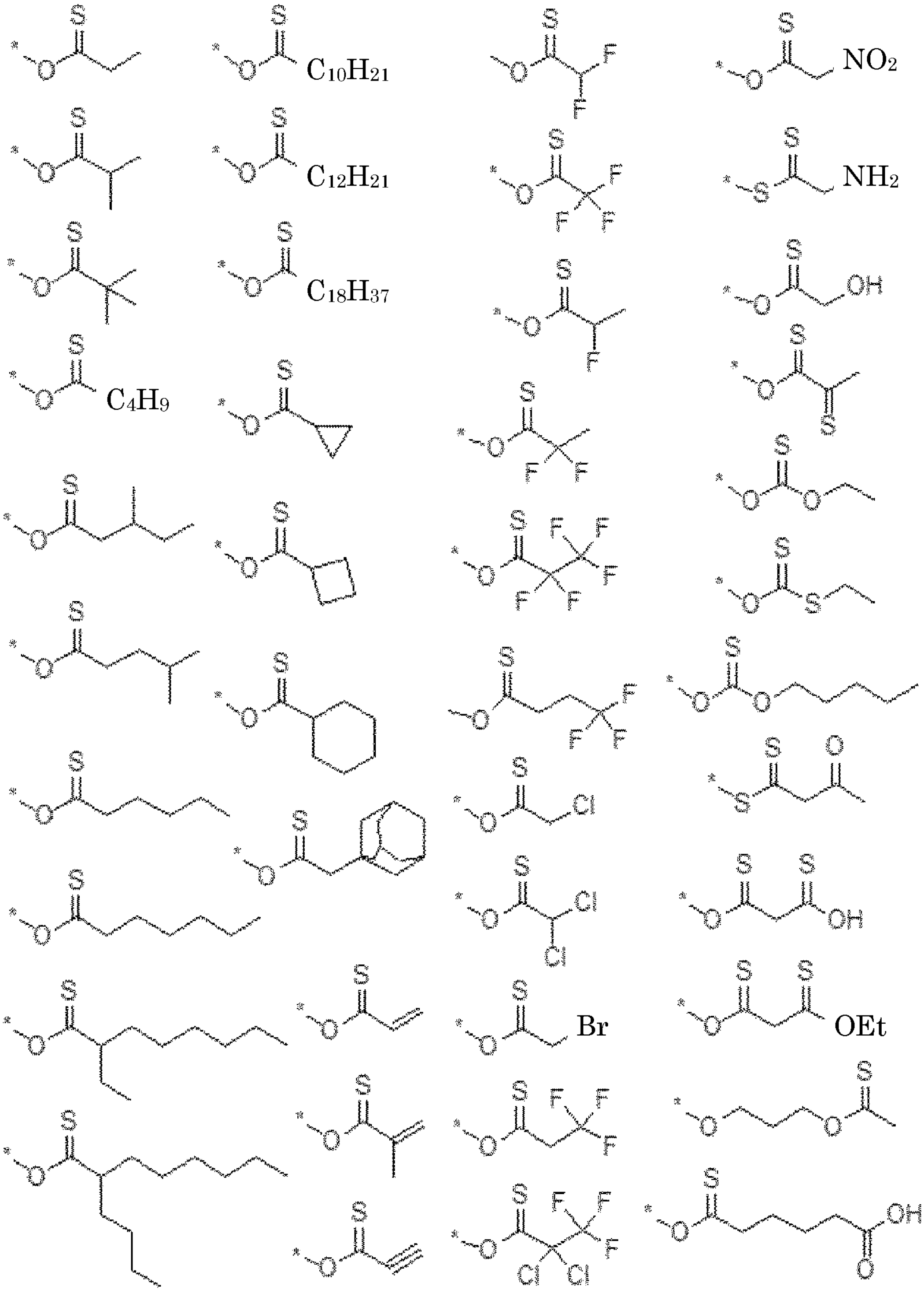

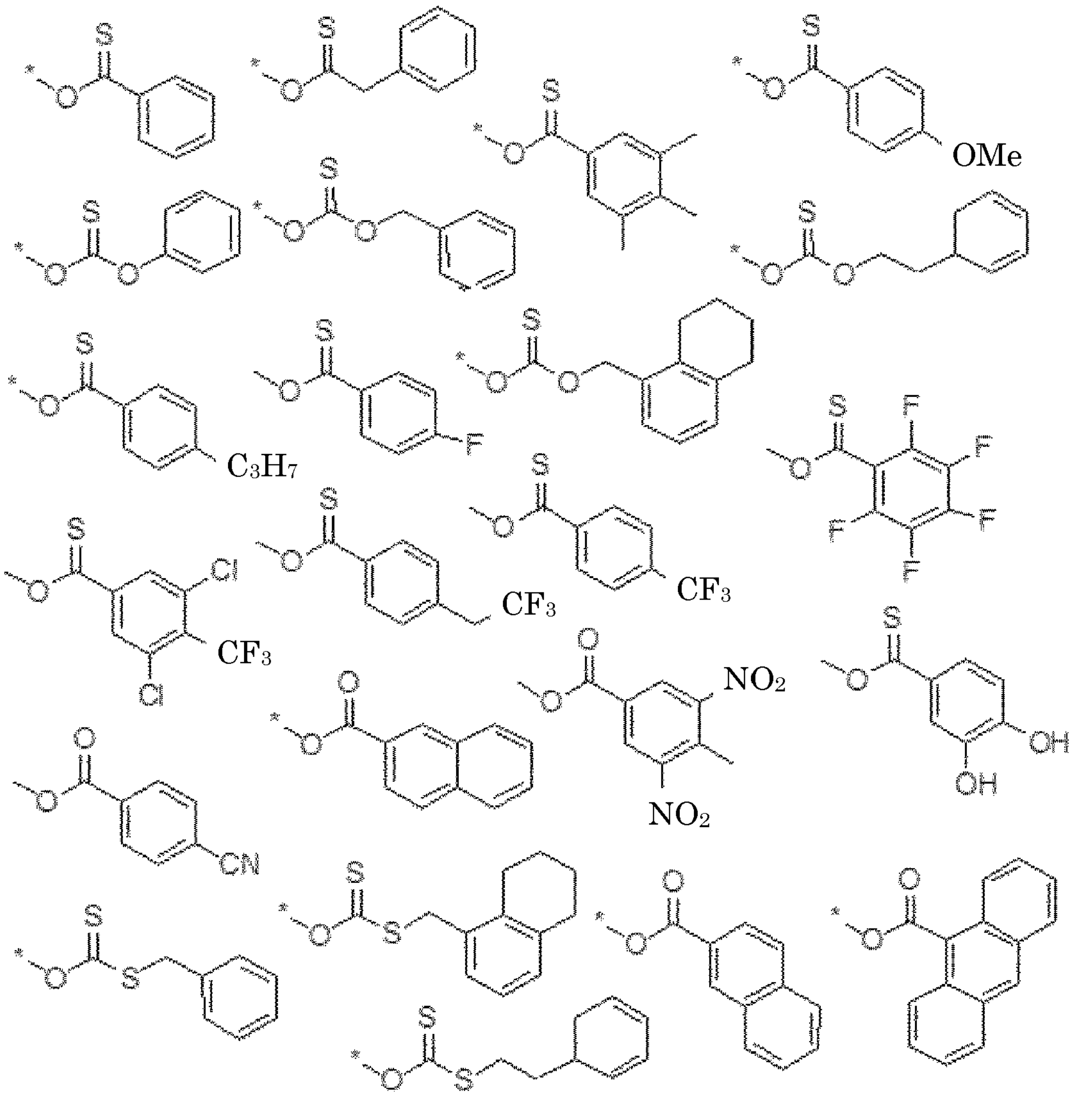

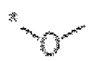

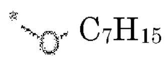

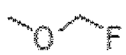

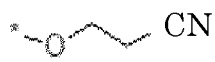

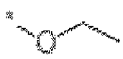

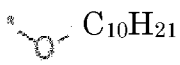

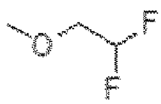

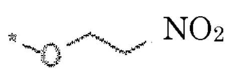

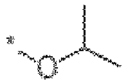

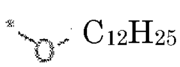

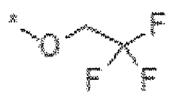

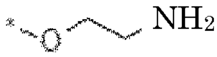
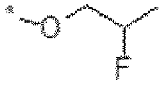
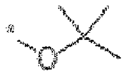
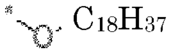
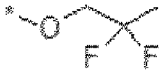
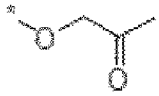
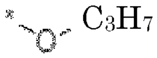
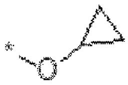
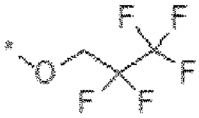
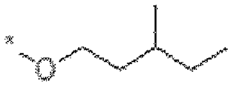
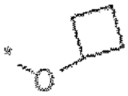
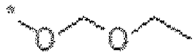
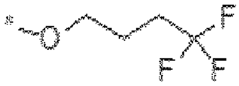
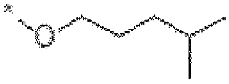
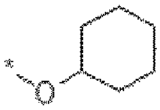
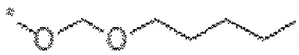
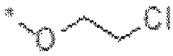
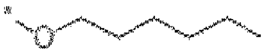
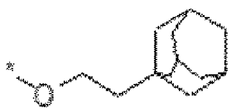
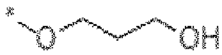
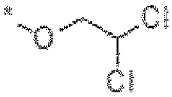
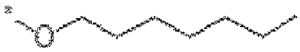
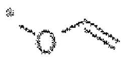
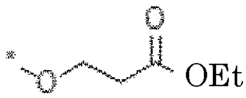
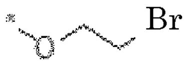
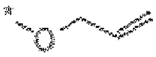
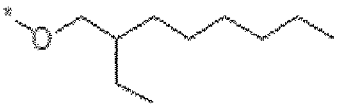
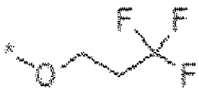
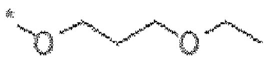
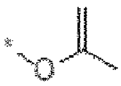
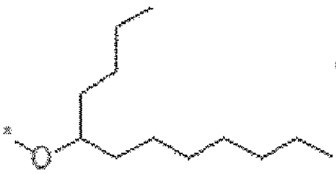
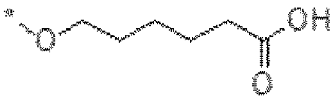
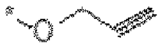
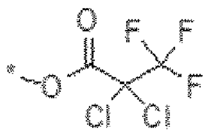
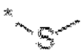
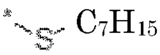
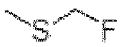
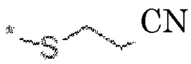

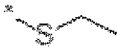
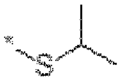
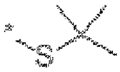
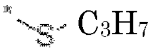
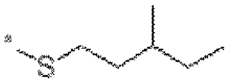
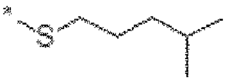
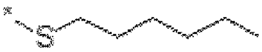
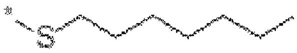
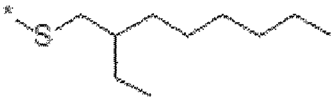
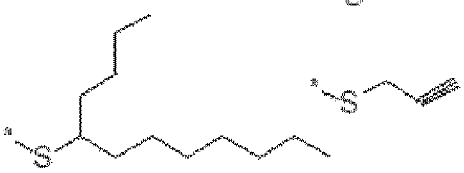
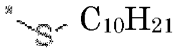
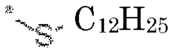
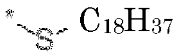
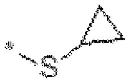
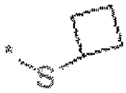
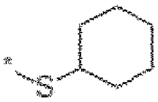
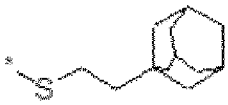
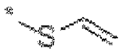
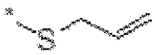
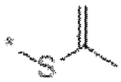
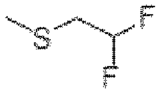
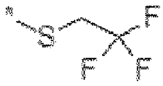
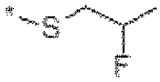
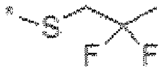
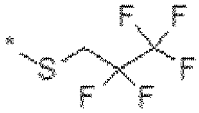
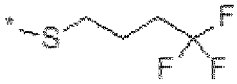
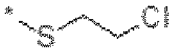
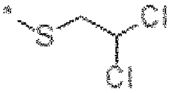
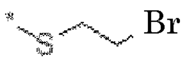
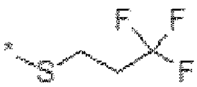
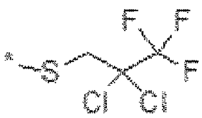
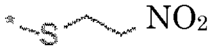
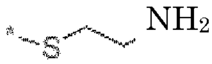
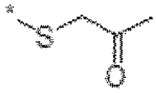
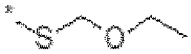
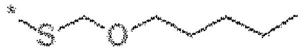
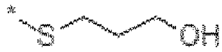
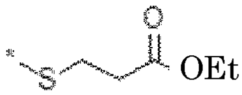
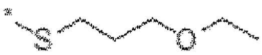
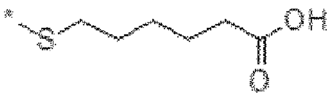

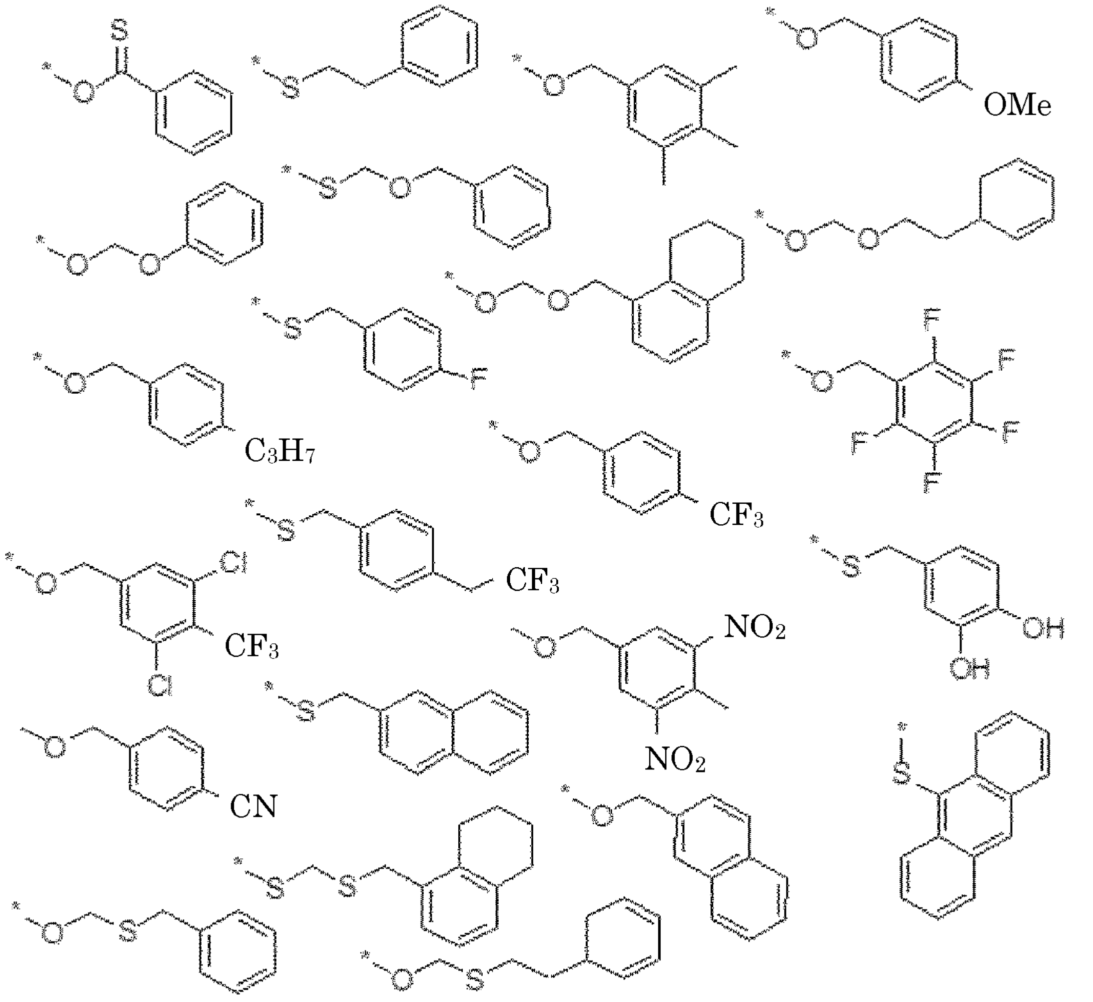

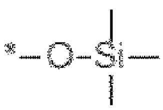 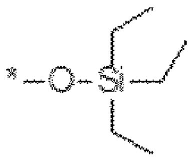 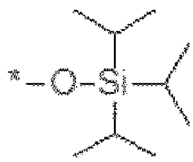 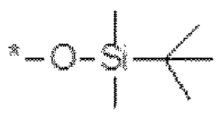 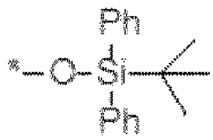

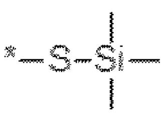 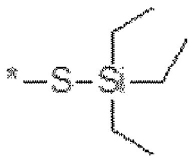 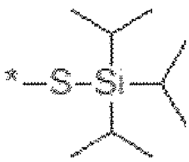 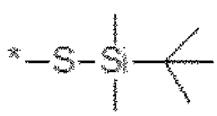 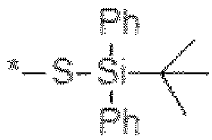

Introduction of the substituted or unsubstituted ether group or acyloxy group having one or more carbon atoms (leaving group) enables the compound to perform elimination reaction of its leaving group by energy (heat) lower than in the conventional compounds while the compound maintains its high dissolvability to an organic solvent and safety.

As another leaving group, a substituted or unsubstituted sulfonyloxy group having one or more carbon atoms may be used instead of the substituted or unsubstituted ether group or acyloxy group having one or more carbon atoms.

Examples of the above substituted or unsubstituted sulfonyloxy group include sulfonyloxy groups derived from sulfonic acids such as linear or cyclic aliphatic sulfonic acids having one or more carbon atoms and aromatic sulfonic acids having four or more carbon atoms. Specific examples thereof include a methylsulfonyloxy group, an ethylsulfonyloxy group, an isopropylsulfonyloxy group, a pivaloylsulfonyloxy group, a pentanoylsulfonyloxy group, a hexanoylsulfonyloxy group, a trifluoromethanesulfonyloxy group, a 3,3,3-trifluoropropionylsulfonyloxy group, a phenylsulfonyloxy group and a p-toluenesulfonyloxy group. Further examples include sulfonylthiooxy groups obtained by replacing, with a sulfur atom,

the oxygen atom in the ether bond of the above sulfonyloxy groups. The number of carbon atoms contained in the above sulfonyloxy group is generally 1 to 38, preferably 2 to 22, still more preferably 3 to 18, considering various factors such as dissolvability and the boiling point of an eliminated component.

As disclosed herein, the groups represented by $Q_1$ to $Q_6$ are, as described above, a hydrogen atom, a halogen atom (e.g., a fluorine atom, a chlorine atom, a bromine atom or an iodine atom), or a monovalent organic group (provided that $Q_1$ and $Q_6$ are monovalent organic groups other than the leaving substituent; i.e., preferably, monovalent organic groups other than the substituted or unsubstituted ether group or acyloxy group having one or more carbon atoms). Examples of the monovalent organic group include alkyl groups, alkenyl groups, alkynyl groups, aryl groups, heteroaryl groups, alkoxyl groups, thioalkoxyl groups, aryloxy groups, thioaryloxy groups, heteroaryloxy groups, heteroarylthiooxy groups, a cyano group, a hydroxyl group, a nitro group, a carboxyl group, a thiol group and an amino group.

When $Q_1$ and $Q_6$ are the leaving substituents, competitive elimination reaction with the leaving substituents X and Y occurs depending on the reaction conditions. As a result, there is a possibility that a single target product cannot be obtained or the reaction does not proceed altogether. Thus, when $Q_1$ and $Q_6$ are the leaving substituents, the usefulness of the compound becomes low.

The above alkyl group is a linear, branched or cyclic, substituted or unsubstituted alkyl group.

Examples of the alkyl group include alkyl groups (preferably, substituted or unsubstituted alkyl groups having one or more carbon atoms such as a methyl group, an ethyl group, a n-propyl group, an i-propyl group, a t-butyl group, a s-butyl group, a n-butyl group, an i-butyl group, a pentyl group, a hexyl group, a heptyl group, an octyl group, a nonyl group, a decyl group, an undecyl group, a dodecyl group, a tridecyl group, a tetradecyl group, a pentadecane group, a hexadecyl group, a heptadecyl group, an octadecyl group, a 3,7-dimethyloctyl group, a 2-ethylhexyl group, a trifluoromethyl group, a trifluorooctyl group, a trifluorododecyl group, a trifluorooctadecyl group and a 2-cyanoethyl group) and cycloalkyl groups (preferably, substituted or unsubstituted alkyl groups having three or more carbon atoms such as a cyclopentyl group, a cyclobutyl group, a cyclohexyl group and a pentafluorocyclohexyl group). The number of carbon atoms contained in the above alkyl group is generally 1 to 38, preferably 2 to 22, still more preferably 3 to 18, considering various factors such as dissolvability and the boiling point of an eliminated component.

The alkyl groups referred to in the other organic groups described below refer to the above-described alkyl groups.

The above alkenyl group is a linear, branched or cyclic, substituted or unsubstituted alkenyl group. Examples of the alkenyl group include alkenyl groups (preferably, substituted or unsubstituted alkenyl groups having two or more carbon atoms such as groups obtained by changing one or more carbon-carbon single bonds to a double bond in the above-exemplified alkyl groups having two or more carbon atoms (e.g., an ethenyl group

(a vinyl group), a propenyl group (an allyl group), a 1-butenyl group, a 2-butenyl group, a 2-methyl-2-butenyl group, a 1-pentenyl group, a 2-pentenyl group, a 3-pentenyl group, a 1-hexenyl group, a 2-hexenyl group, a 3-hexenyl group, a 1-heptenyl group, a 2-heptenyl group, a 3-heptenyl group, a 4-heptenyl group, a 1-octenyl group, a 2-octenyl group, a 3-octenyl group, a 4-octenyl group and a 1,1,1-trifluoro-2-butenyl group)) and cycloalkenyl groups such as groups obtained by changing one or more carbon-carbon single bonds to a double bond in the above-exemplified cycloalkyl groups having two or more carbon atoms (e.g., a 1-cycloallyl group, a 1-cyclobutenyl group, a 1-cyclopentenyl group, a 2-cyclopentenyl group, a 3-cyclopentenyl group, a 1-cyclohexenyl group, a 2-cyclohexenyl group, a 3-cyclohexenyl group, a 1-cycloheptenyl group, a 2-cycloheptenyl group, a 3-cycloheptenyl group, a 4-cycloheptenyl group and a 3-fluoro-1-cyclohexenyl group)). When the alkenyl group has stereoisomers such as a trans (E) form and cis (Z) form, both the stereoisomers may be used, or a mixture containing them at any ratio may be used also.

The above alkynyl group is preferably a substituted or unsubstituted alkynyl group having two or more carbon atoms such as groups obtained by changing one or more carbon-carbon single bonds to a triple bond in the above-exemplified alkyl groups having two or more carbon atoms. Examples thereof include an ethynyl group, a proparygyl group, a trimethylsilylethynyl group and a triisopropylsilylethynyl group.

The above aryl group is preferably a substituted or unsubstituted aryl group having six or more carbon atoms (e.g., a phenyl group, an o-tolyl group, a m-tolyl group, a p-tolyl group, a p-chlorophenyl group, a p-fluorophenyl group, a p-trifluorophenyl group and a naphthyl group).

The above heteroaryl group is preferably 5- or 6-membered substituted or unsubstituted, aromatic or nonaromatic heterocyclic groups (e.g., a 2-furyl group, a 2-thienyl group, a 3-thienyl group, a 2-thienothienyl group, a 2-benzothienyl group and a 2-pyrimidyl group)).

The above alkoxyl group and thioalkoxyl group are preferably substituted or unsubstituted alkoxyl groups and thioalkoxyl groups such as groups obtained by introducing an oxygen atom or a sulfur atom into the binding site of the above-exemplified alkyl, alkenyl and alkynyl groups.

The above aryloxy group and thioaryloxy group are preferably substituted or unsubstituted aryloxy groups and thioaryloxy groups such as groups obtained by introducing an oxygen atom or a sulfur atom into the binding site of the above-exemplified aryl groups.

The above heteroaryloxy group and heterothioaryloxy group are preferably substituted or unsubstituted heteroaryloxy groups and heteroarylthiooxy groups such as groups obtained by introducing an oxygen atom or a sulfur atom into the binding site of the above-exemplified heteroaryl groups.

The above amino group is preferably an amino group, substituted or unsubstituted alkylamino groups, substituted or unsubstituted anilino groups such as an amino group, a methylamino group, a dimethylamino group, an anilino group, an N-methyl-anilino group and a diphenylamino group; an acylamino group (preferably, a formylamino group, a substituted or unsubstituted alkylcarbonylamino group and a substituted or unsubstituted arylcarbonylamino group (e.g., a formylamino group, an acetylamino group, a pivaloylamino group, a lauroylamino group, a benzoylamino group and a 3,4,5-tri-n-octyloxyphenylcarbonylamino group)) and an aminocarbonylamino group (preferably, a carbon-substituted or unsubstituted aminocarbonylamino group (e.g., a carbamoylamino group, an N,N-dimethylaminocarbonylamino group, an N,N-diethylaminocarbonylamino group and a morpholinocarbonylamino group)).

The monovalent organic groups represented by $Q_1$ to $Q_6$ may be those described above. Preferably, they are substituted or unsubstituted aryl groups or heteroaryl groups, or form ring structures together with the adjacent groups. More preferably, the ring structures are formed of substituted or unsubstituted aryl groups or heteroaryl groups

The forms of the bond or ring-fusion of the ring structures are expressed by the following I-(1) to I-(42), for example.

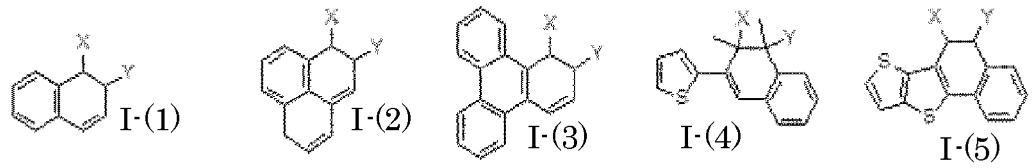
I-(1) I-(2) I-(3) I-(4) I-(5)

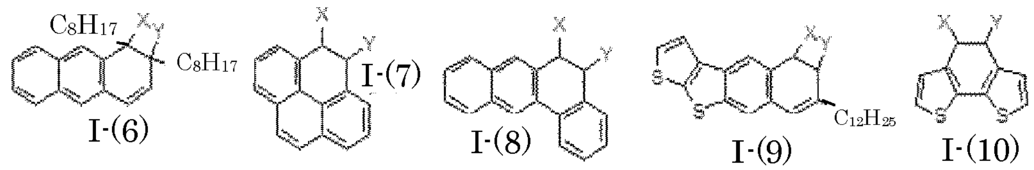
I-(6) I-(7) I-(8) I-(9) I-(10)

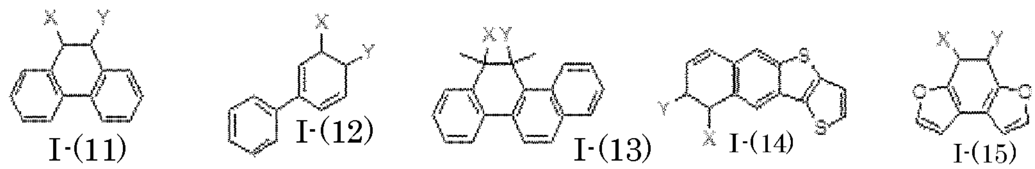
I-(11) I-(12) I-(13) I-(14) I-(15)

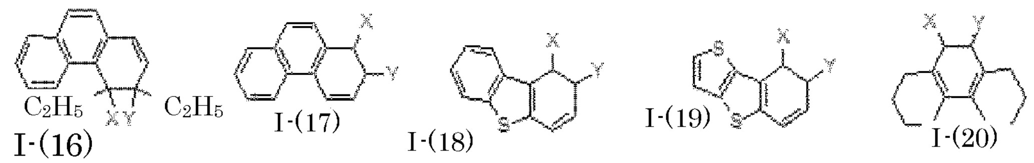
I-(16) I-(17) I-(18) I-(19) I-(20)

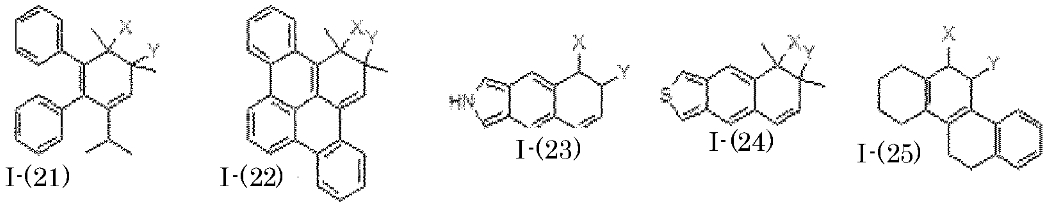
I-(21) I-(22) I-(23) I-(24) I-(25)

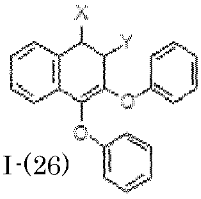
I-(26)

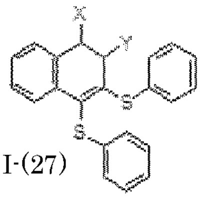
I-(27)

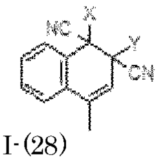
I-(28)

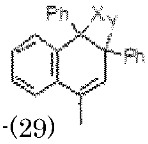
I-(29)

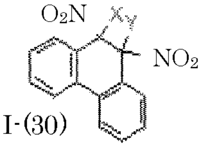
I-(30)

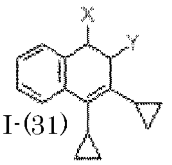
I-(31)

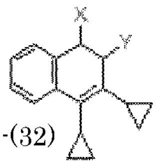
I-(32)

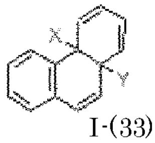
I-(33)

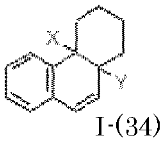
I-(34)

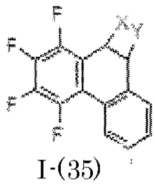
I-(35)

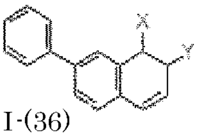
I-(36)

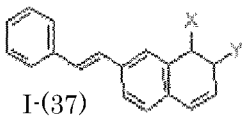
I-(37)

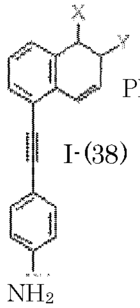
I-(38)

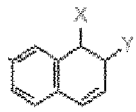
I-(39)

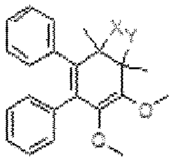
I-(40)

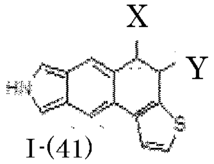
I-(41)

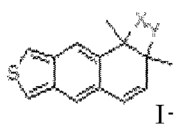
I-(42)

Preferred examples of the substituted or unsubstituted aryl or heteroaryl group include a benzene ring, a thiophene ring, a pyridine ring, a pyrazine ring, a pyrimidine ring, a triazine ring, a pyrrol ring, a pyrazole ring, an imidazole ring, a triazole ring, an oxazole ring, a thiazole ring, a furan ring, a selenophene ring and a silole ring. More preferred are (i) compounds in which one or more of the above aryl groups, heteroaryl groups and rings are ring-fused together and (ii) compounds in which the rings in (i) are linked together via a covalent bond.

Also, preferred is at least one $\pi$-electron conjugated compound selected from the group consisting of the compounds in (i) and the compounds in (ii) Further, $\pi$ electrons contained in the aromatic hydrocarbon rings or aromatic heterocyclic rings are preferably delocalized throughout the ring-fused or linked ring by the interaction as a result of ring-fused linkage or covalently bonding.

Here, the "covalent bond" may be, for example, a carbon-carbon single bond, a carbon-carbon double bond, a carbon-carbon triple bond, an oxyether bond, a thioether bond, an amide bond and an ester bond, with a carbon-carbon single bond, a carbon-carbon double bond and a carbon-carbon triple bond being preferred.

The number of the aromatic hydrocarbon rings or aromatic heterocyclic rings which are ring-fused or linked together via a covalent bond is preferably two or more. Specific examples thereof include naphthalene, anthracene, tetracene (naphthacene), chrycene and pyrene (the following General Formula Ar3), pentacene and thienothiophene (the following General Formula Ar1), thienodithiophene triphenylene, hexabenzocoronene and benzothiophene (the following General Formula Ar2), benzodithiophene and [1]benzothieno[3,2-b][1]benzothiophene (BTBT) (the following General Formula Ar4), dinaphto[2,3-b:2',3'-f][3,2-b]thienothiophene (DNTT) and benzodithienothiophene (TTPTT) (the following General Formula Ar5), fused polycyclic compounds such as naphthodithienothiophene (TTNTT) (the following General Formulas Ar6 and Ar7), and oligomers of aromatic hydrocarbon rings and aromatic heterocyclic rings such as biphenyl, terphenyl, quaterphenyl, bithiophene, terthiophene and quaterthiophene; phthalocyanines; and porphyrins.

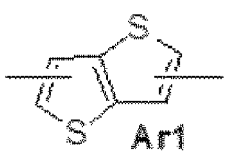
Ar1

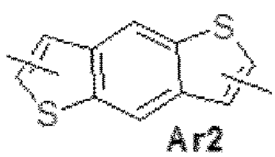
Ar2

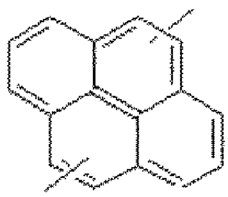
Ar3

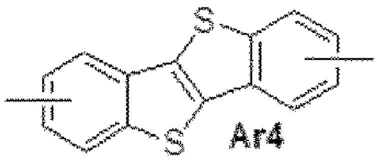
Ar4

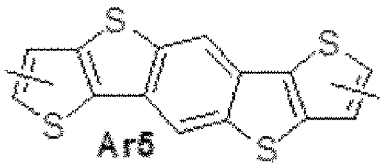
Ar5

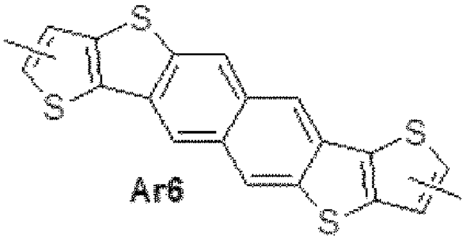
Ar6

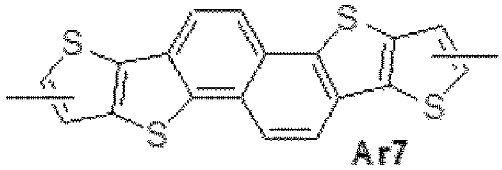
Ar7

As disclosed herein, needless to say, the number of soluble substituents bonded or fused via a covalent bond to the main skeleton depends on the number of atoms on the Ar that can provide sites for substitution or ring fusion. For example, an unsubstituted benzene ring can provide up to six sites for substitution via a covalent bond or for ring fusion. However, considering the size of the main skeleton itself, the number of substituents depending on dissolvability, symmetry of the molecule and easiness of synthesis, the number of soluble substituents contained in one molecule is preferably 2 or more. Meanwhile, when the number of soluble substituents is too large, the soluble substituents sterically interact with each other, which is not preferred. Thus, considering symmetry of the molecule, the number of substituents depending on dissolvability and easiness of synthesis, the number of soluble substituents contained in one molecule is preferably 4 or less.

As described above, one of X and Y is a substituted or unsubstituted [ether group or acyloxy group] having one or more carbon atoms. Such [ether group or acyloxy group] may have a structure expressed by the following General Formula (III) or (IV).

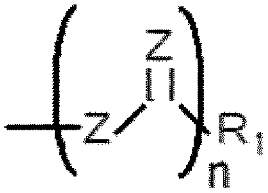
(III)

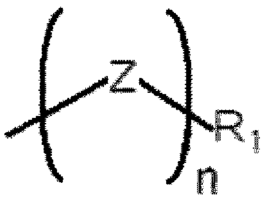
(IV)

When n = 1, the above General Formulas (III) and (IV) are the following General Formula (III-1) and (IV-1), respectively.

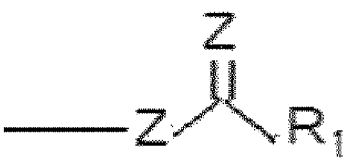
(III - 1)

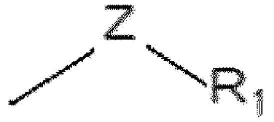

(IV-1)

When n = 2, the above General Formulas (III) and (IV) are the following General Formula (III-2) and (IV-2), respectively.

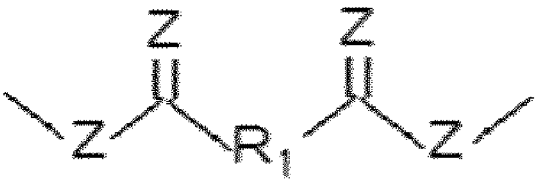

(III-2)

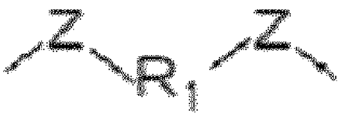

(IV-2)

In the case of General Formula (III-2) or (IV-2), one of the acyloxy groups may be bonded as X or Y and the other acyloxy group may be bonded as X' or Y' (not shown) in the same molecule or another molecule.

In the above General Formulas, Z denotes an oxygen atom or a sulfur atom, where when there are two or more Zs, the Zs may be identical or different; and $R_1$ represents a hydrogen atom [except for the cases of General Formulas (III-2) and (IV-2)], a monovalent organic group or a divalent organic group.

Preferably, $R_1$ is a hydrogen atom [except for the cases of General Formulas (III-2) and (IV-2)], a substituted or unsubstituted alkyl group, a substituted or unsubstituted alkenyl group, a substituted or unsubstituted alkynyl group, a substituted or unsubstituted alkoxyl group, a substituted or unsubstituted thioalkoxyl group, a substituted or unsubstituted aryl group, a substituted or unsubstituted heteroaryl group, or a cyano group. More preferably, $R_1$ is a hydrogen atom [except for the cases of General Formulas (III-2) and (IV-2)] or a substituted or unsubstituted alkyl group. Particularly preferably, $R_1$ is a substituted or unsubstituted alkyl group. The number of carbon atoms contained in the alkyl group is generally 1 to 38, preferably 2 to 22, still more preferably 3 to 18, considering various factors such as dissolvability and the boiling point of an eliminated component.

Examples of the eliminated component X-Y include alcohols (thiols), carboxylic acids (thiocarboxylic acids) and carbonate half esters (thiocarbonate half esters) that are obtained by cleaving the -O- or -S- bonding sites of the above substituted or unsubstituted ether groups or acyloxy groups and replacing the ends of the resultant products with hydrogen.

Examples of the alcohol include methanol, ethanol, propanol, isopropanol, butanol, isobutanol, tert-butyl alcohol, pentanol, hexanol, trifluoromethanol, 3,3,3-trifluoropropanol, pentafluoropropanol, cyclopropanol, cyclobutanol, cyclohexanol, trimethylsilanol, triethylsilanol, tert-butyldimethylsilanol and tert-butyldiphenylsilanol. Further examples include thiols obtained by replacing, with a sulfur atom, the oxygen atom in the ether bonds of the above alcohols.

Examples of the carboxylic acid include formic acid, acetic acid, propionic acid, butyric acid, valeric acid, isovaleric acid, pivalic acid, caproic acid, lauric acid, stearic acid, trifluoroacetic acid, 3,3,3-trifluoropropionic acid, pentafluoropropionic acid, cyclopropanecarboxyic acid, cyclobutanecarboxyic acid, cyclohexanecarboxyic acid, benzoic acid, p-methoxybenzoic acid and pentafluorobenzoic acid. Further examples include thiocarboxylic acids obtained by replacing, with a sulfur atom, the oxygen atom in the ether bonds of the above carboxylic acids.

After elimination, the above carbonate half esters (or thiocarbonate half esters) are further decomposed with heating to form the corresponding alcohols (or thiols) and carbon dioxide or carbonyl sulfide.

The above substituted or unsubstituted sulfonyloxy group is represented by the following General Formula (V), for example.

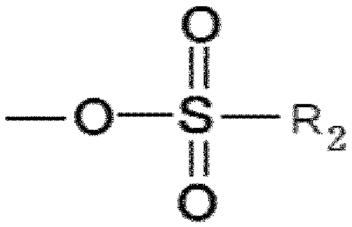 ( V )

In General Formula (V), example of substituents represented by $R_2$ include the same substituents as described above in relation to $Q_1$ to $Q_6$.

Specific examples include a substituted or unsubstituted alkyl group, a substituted or unsubstituted alkenyl group, a substituted or unsubstituted alkynyl group, a substituted or unsubstituted alkoxyl group, a substituted or unsubstituted thioalkyl group, a substituted or unsubstituted aryl group, a substituted or unsubstituted heteroaryl group and a cyano group.

Examples of the eliminated component X-Y include sulfonic acids and thiosulfonic acids that are obtained by cleaving the -O- or -S- bonding sites of the above sulfonyloxy groups and replacing the ends of the resultant products with hydrogen. Specific examples include methanesulfonic acid, ethanesulfonic acid, isopropylsulfonic acid, pivaloylsulfonic acid, pentanesulfonic acid, hexanoylsulfonic acid, toluenesulfonic acid, phenylsulfonic acid, trifluoromethanesulfonic acid and 3,3,3-trifluoropropionylsulfonic acid. Further examples include thiosulfonic acids obtained by replacing, with a sulfur atom, the oxygen atom in the ether bonds of the above sulfonic acids.

The substituents represented by $R_1$ and $R_2$ in General Formulas (III) and (V) are not particularly limited, so long as they are those described above. From the viewpoints of solvent solubility and film formability, it is advantageous that the substituents selected reduces intermolecular interaction to a certain extent and enhances affinity to a solvent. Meanwhile, when the volume before or after elimination of the substituents is considerably changed, there is a concern on problematic unevenness in coating of a thin film through elimination reaction. Therefore, the substituent used is preferably smaller in size to the greatest extent possible while maintaining appropriate solubility. Further, $R_1$ and $R_2$ each preferably represent an electron-attracting substituent (e.g., a halogen-containing alkyl group and a cyano group-containing group) with which the carbonyl oxygen is negatively charged to a larger extent, since elimination reaction can be efficiently performed (although the reason for this is still unclear).

As described above, the leaving substituent-containing compound disclosed herein contains leaving solvent-soluble substituents which impart solvent solubility to the leaving substituent-containing compound.

As disclosed herein, the term "solvent solubility" means that a compound shows a solubility of 0.05% by mass or more, preferably 0.1% by mass or more, more preferably 0.5% by mass or more, particularly preferably 1.0% by mass or more, when a solvent to which the compound is added is heated under reflux and then cooled down to room temperature.

Here, the term "solvent insolubilization" means that the solvent solubility of a compound is reduced by one digit figure or more. Specifically, when a solvent to which the compound is added is heated under reflux and then cooled down to room temperature, it is preferable to reduce the solvent solubility from 0.05% by mass to 0.005% by mass; more preferably from 0.1% by mass to 0.01% by mass; particularly preferably from 0.5% by mass or more but less than 0.05% by mass; most preferably from 1.0% by mass or more but less than 0.1% by mass. And, the term "solvent insolubility" means that a compound shows a solubility of less than 0.01% by mass, preferably 0.005% by mass or less, more preferably 0.001% by mass or less, when a solvent to which the compound is added is heated under reflux and then cooled down to room temperature.

The type of the solvent used for measuring the "solvent solubility" and "solvent insolubility" is not particularly limited. An actually used solvent may be used at an actually set temperature for the measurement of the solvent solubility. In addition, THF, toluene, chloroform, methanol, other solvents may be used at 25°C for the measurement of the solvent solubility.

Note that a solvent usable as disclosed herein should not be construed as being limited to these solvents.

The solubility greatly changes before or after conversion through elimination reaction of the substituents X and Y; i.e., before or after the leaving substituent-containing compound represented by General Formula (I) is converted to a compound represented by General Formula (Ia) (hereinafter may be referred to as a "specific compound" or "organic semiconductor compound"). As a result, even when another film is immediately formed on the underlying film made of the specific compound, the underlying film does not tend to be abraded by the solvent used for the formation of the another film. Thus, the compound disclosed herein is useful in production processes for organic electronic devices such as organic thin-film transistors, organic EL elements and organic solar cells.

The following compounds (Exemplary Compounds 1 to 42, which are reference compounds) will be given as specific examples of the leaving substituent-containing compound disclosed herein. The leaving substituent-containing compound should not be construed as being limited thereto. Also, it is easily supposed that there are several stereoisomers

of the leaving substituent-containing compound depending on the steric configuration of the leaving substituents, and that the following compounds may be mixtures of such stereoisomers having different steric configurations.

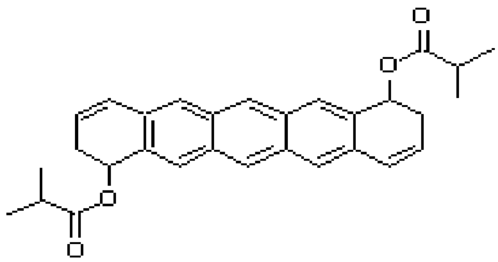

Exemplary Compound 1

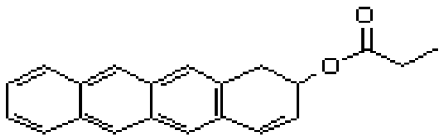

Exemplary Compound 2

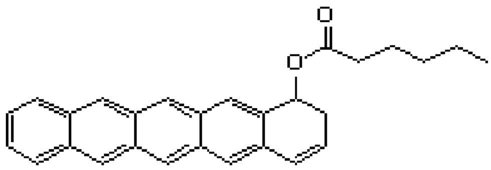

Exemplary Compound 3

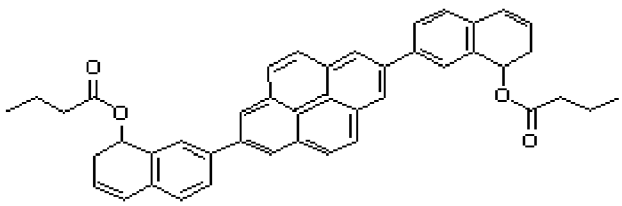

Exemplary Compound 4

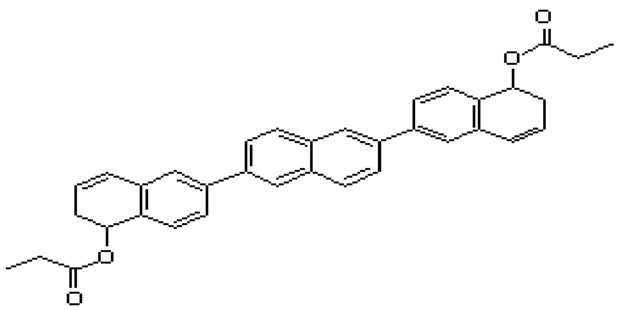

Exemplary Compound 5

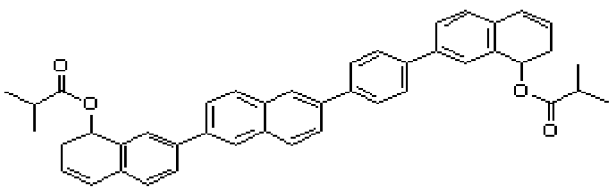

Exemplary Compound 6

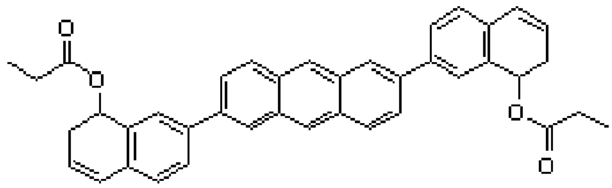

Exemplary Compound 7

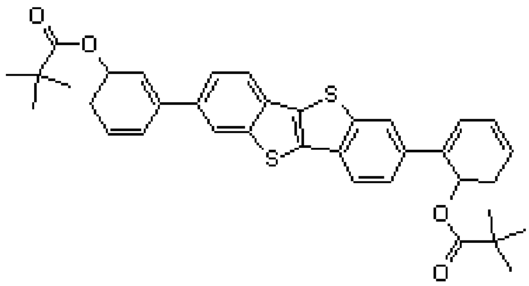

Exemplary Compound 8

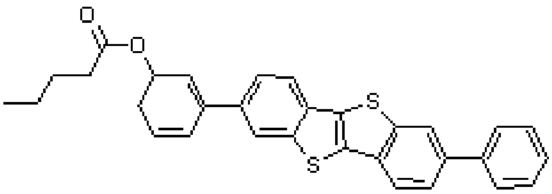

Exemplary Compound 9

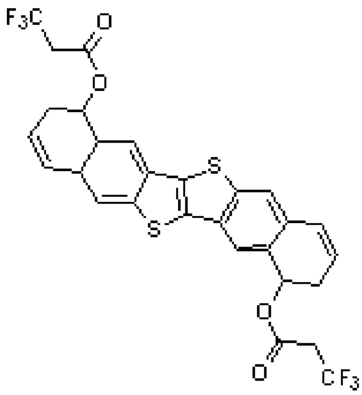

Exemplary Compound 10

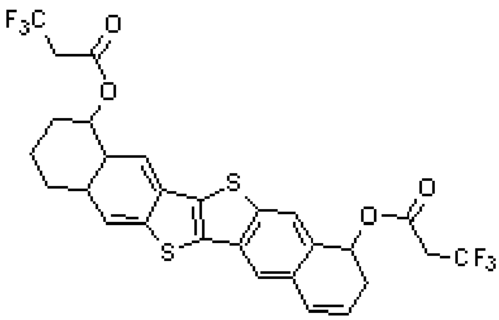

Exemplary Compound 11

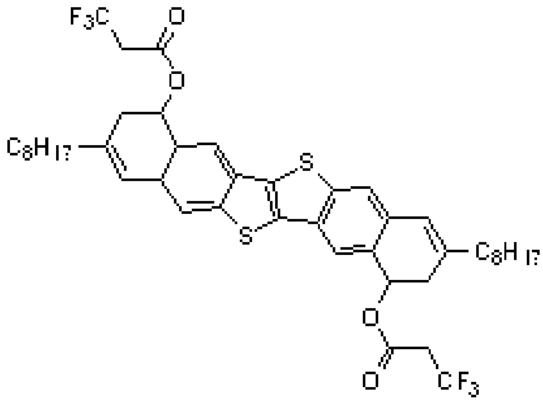

Exemplary Compound 12

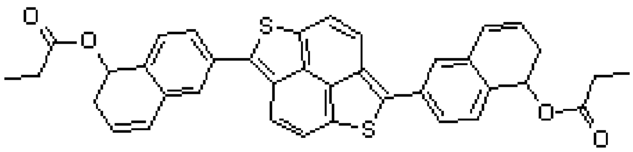

Exemplary Compound 13

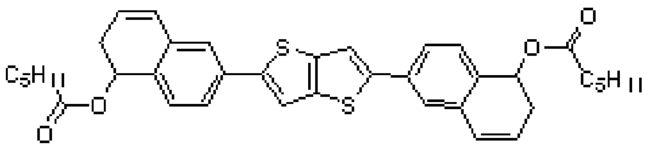

Exemplary Compound 14

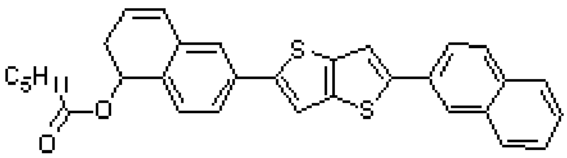

Exemplary Compound 15

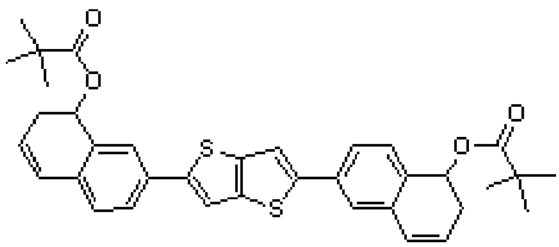

Exemplary Compound 16

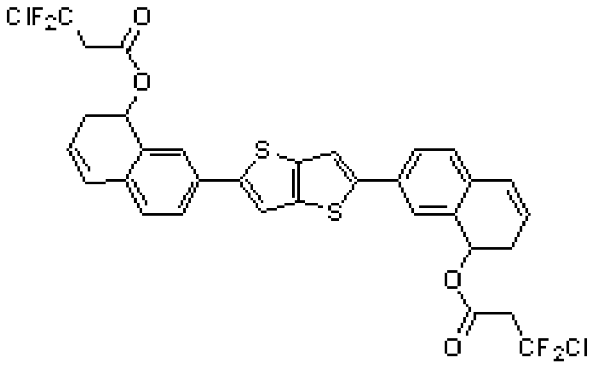

Exemplary Compound 17

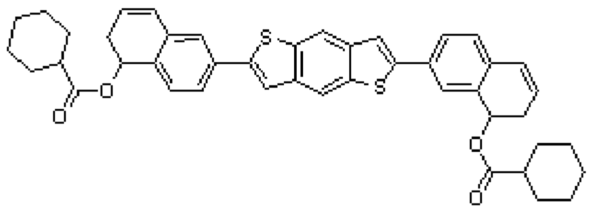

Exemplary Compound 18

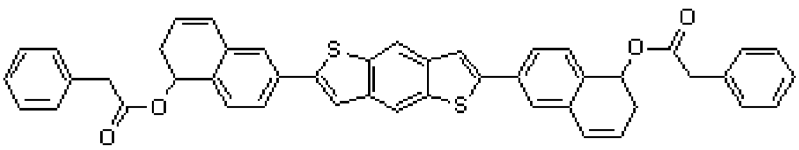

Exemplary Compound 19

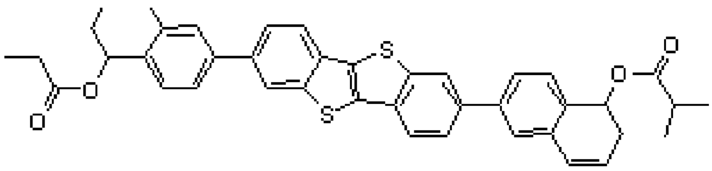

Exemplary Compound 20

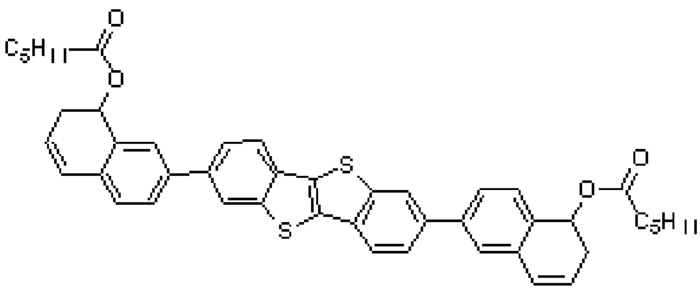

Exemplary Compound 21

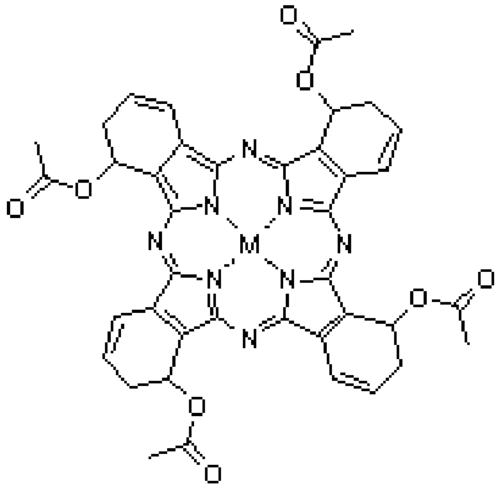

M = $H_2$: Exemplary Compound 22

M = Cu: Exemplary Compound 23

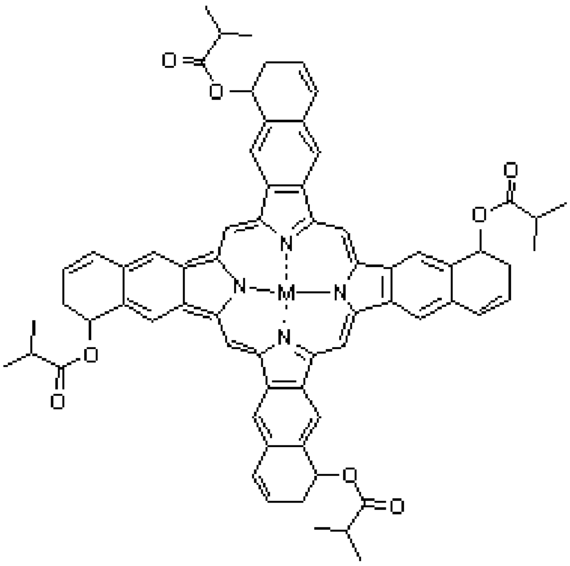

M = $H_2$: Exemplary Compound 24

M = Cu: Exemplary Compound 25

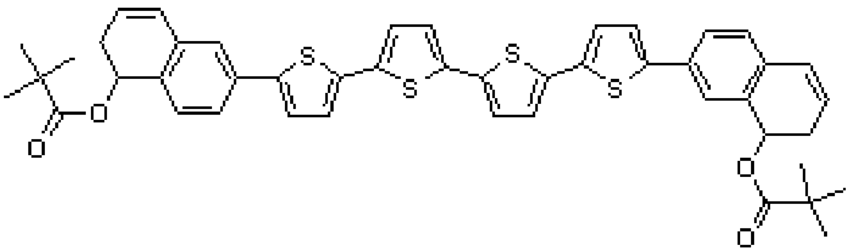

Exemplary Compound 26

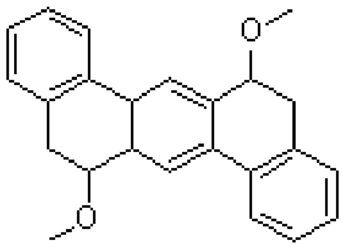

Exemplary Compound 27

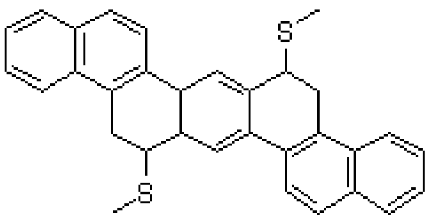

Exemplary Compound 28

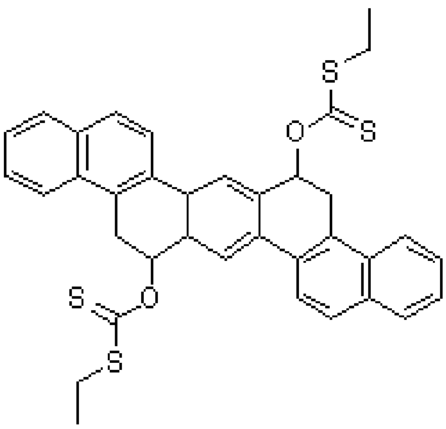

Exemplary Compound 29

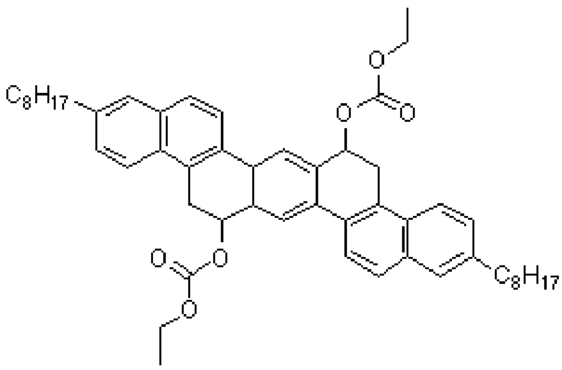

Exemplary Compound 30

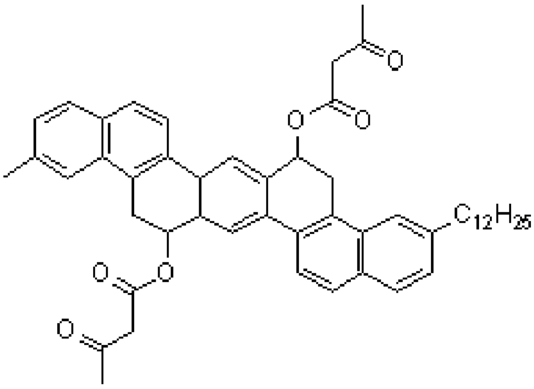

Exemplary Compound 31

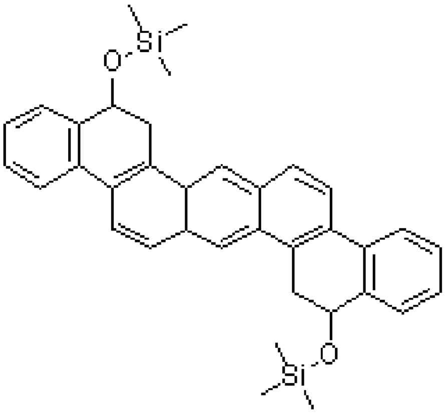

Exemplary Compound 32

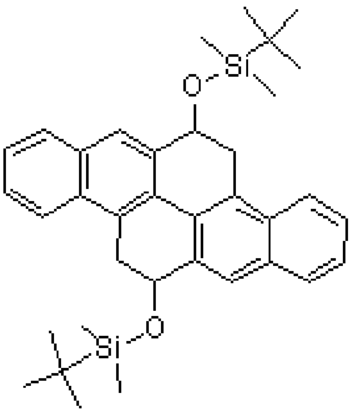

Exemplary Compound 33

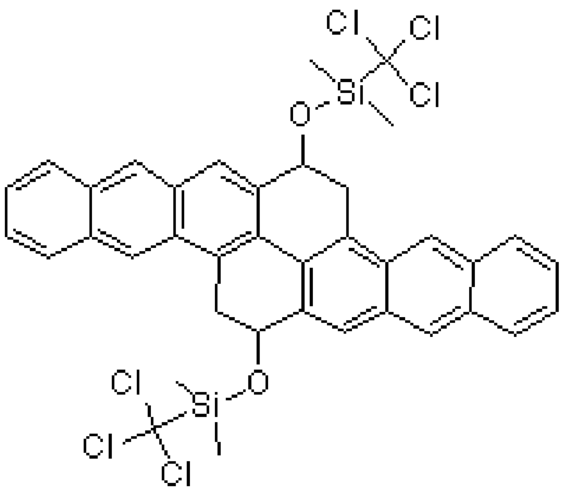

Exemplary Compound 34

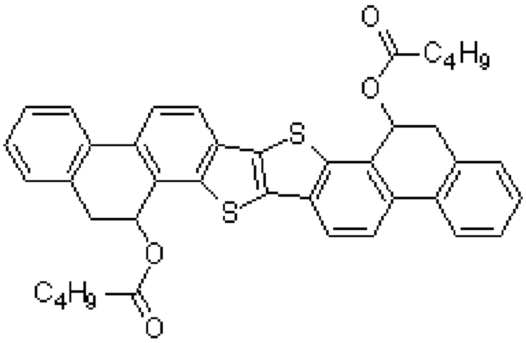

Exemplary Compound 35

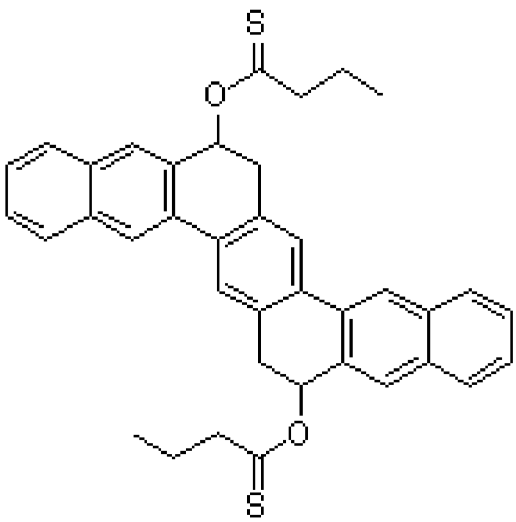

Exemplary Compound 36

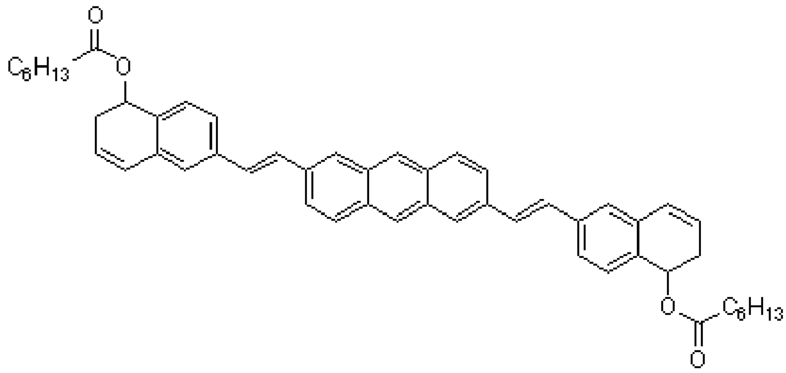

Exemplary Compound 37

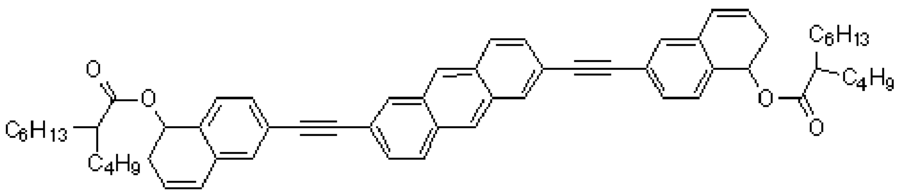

Exemplary Compound 38

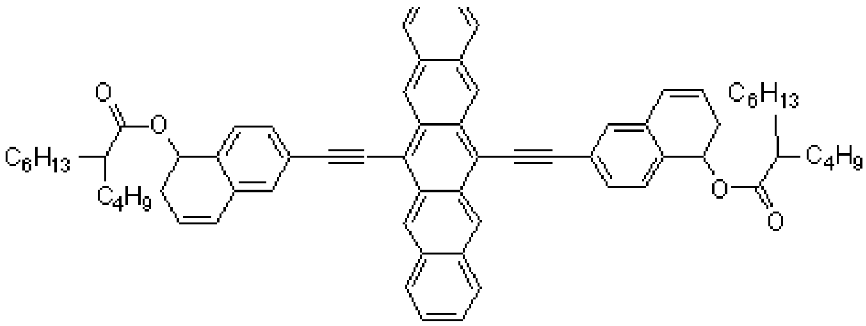

Exemplary Compound 39

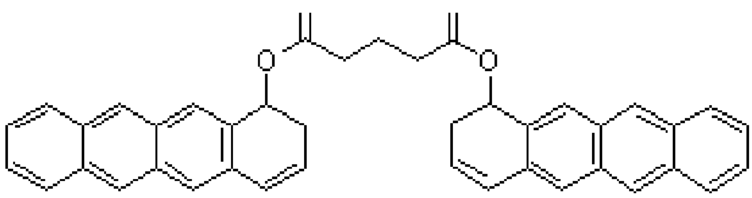

Exemplary Compound 40

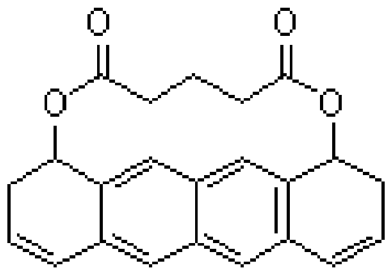

Exemplary Compound 41

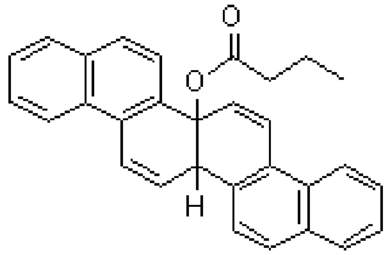

Exemplary Compound 42

When energy (e.g., heat) is applied to the above leaving substituent-containing compound (application of external stimulus), the below-described elimination reaction occurs, so that the substituents X and Y are removed to obtain a specific compound.

Next will be given exemplary specific compounds obtained from the above leaving substituent-containing compounds (Specific Compounds 1 to 29, which are reference compounds). However, the specific compound should not be construed as being limited thereto.

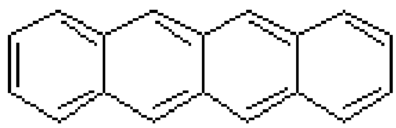

Specific Compound 1

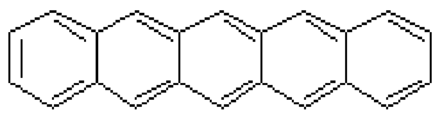

Specific Compound 2

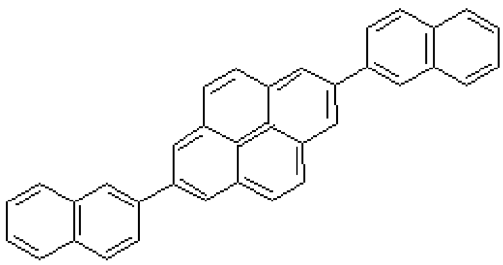

Specific Compound 3

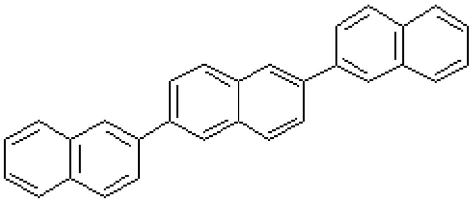

Specific Compound 4

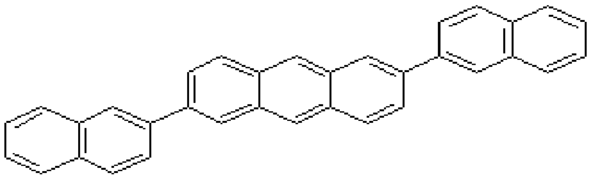

Specific Compound 5

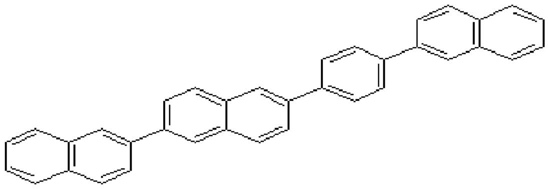

Specific Compound 6

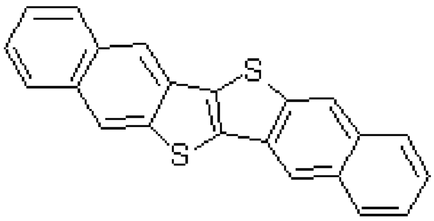

Specific Compound 7

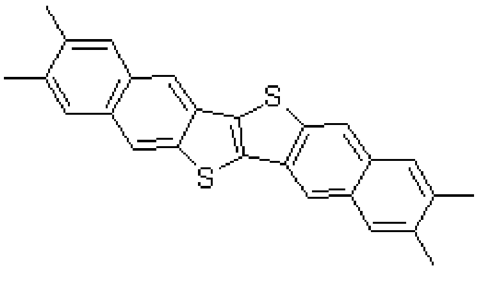

Specific Compound 8

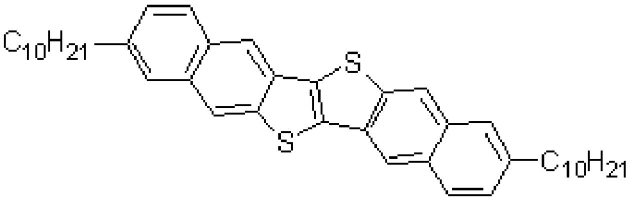

Specific Compound 9

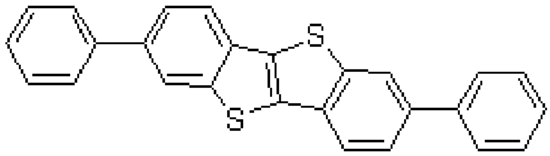

Specific Compound 10

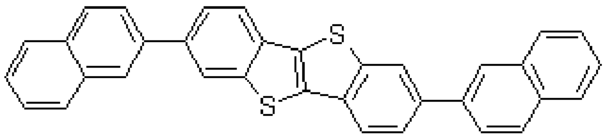

Specific Compound 11

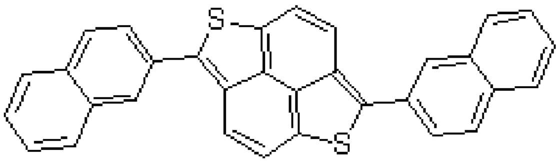

Specific Compound 12

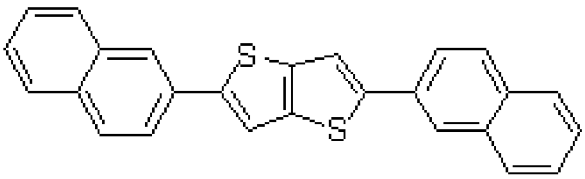

Specific Compound 13

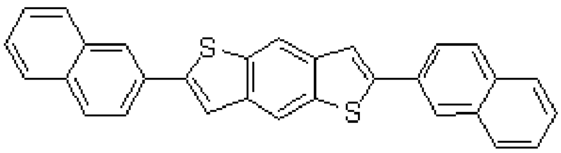

Specific Compound 14

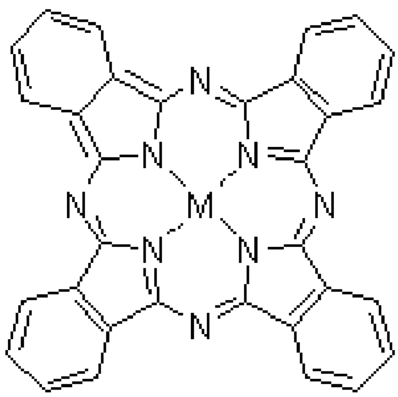

Specific Compound 15 (M = $H_2$)

Specific Compound 16 (M = Cu)

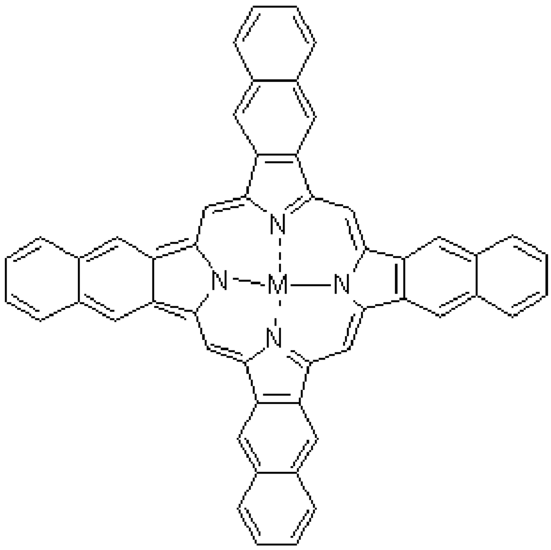

Specific Compound 17 (M = $H_2$)

Specific Compound 18 (M = Cu)

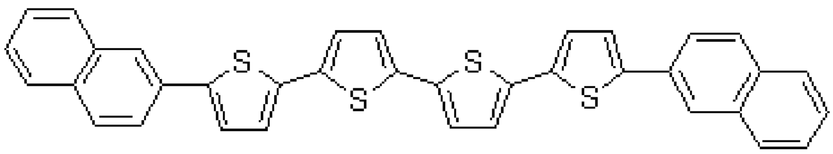

Specific Compound 19

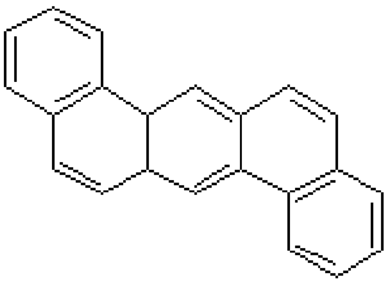

Specific Compound 20

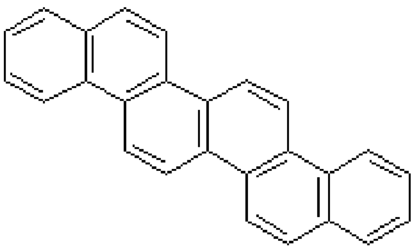

Specific Compound 21

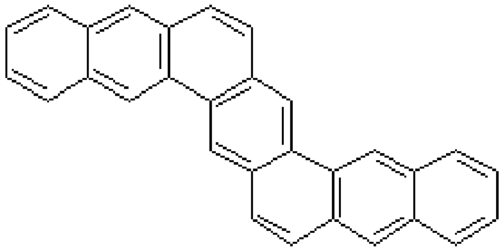

Specific Compound 22

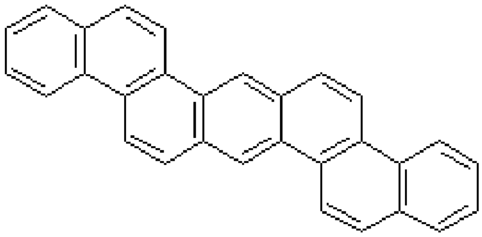

Specific Compound 23

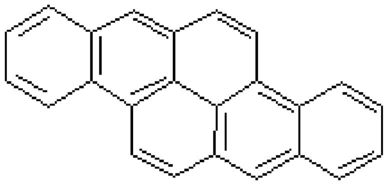

Specific Compound 24

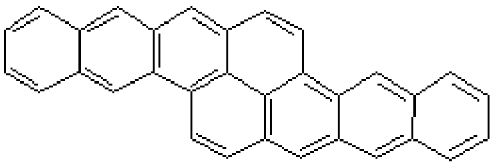

Specific Compound 25

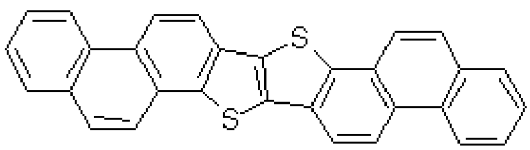

Specific Compound 26

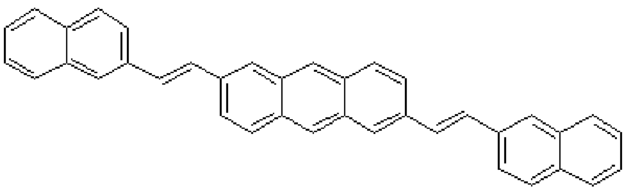

Specific Compound 27

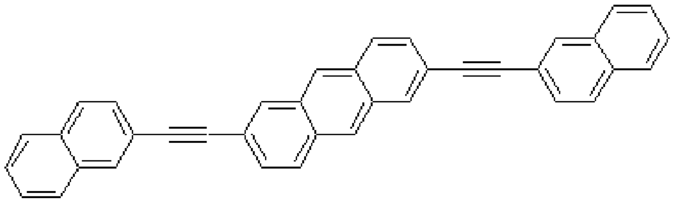

Specific Compound 28

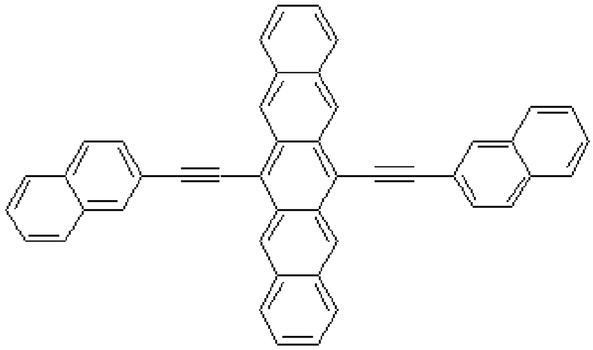

Specific Compound 29

[2. Production Method of Specific Compound by Elimination Reaction of Leaving Substituent-Containing Compound]

The elimination reaction will be specifically described.

As described above, the leaving substituent-containing compound represented by General Formula (I) is converted to the compound represented by General Formula (Ia) (specific compound) and the compound represented by General Formula (Ia) (eliminated component), by applying energy to the leaving substituent-containing compound.

There are several isomers of the compound represented by General Formula (I) depending on the steric configuration of the substituents. However, these isomers are all converted into the specific compound represented by General Formula (Ia) to produce the same eliminated component.

Groups X and Y, which are eliminated from the compound represented by General Formula (I), are defined as leaving substituents, and X-Y formed therefrom is defined as an eliminated component. The eliminated components may be solid, liquid, or gas. In view of removal of the eliminated component to the outside of a system, the eliminated components are preferably liquid or gas, particularly preferably gas at normal temperature, or solid or liquid formed into gas at a temperature for performing elimination reaction.

The boiling point of the eliminated component in an atmospheric pressure (1,013 hPa) is preferably 500°C or lower. From the viewpoint of easiness of removal of the eliminated component to the outside of the system, and the temperature of decomposition and sublimation of a $\pi$-electron conjugated compound to be generated, the boiling point is more preferably 400°C or lower, particularly preferably 300°C or lower.

As one example, next will be described conversion, through elimination reaction, of the compound represented by General Formula (I) where X is a substituted or unsubstituted acyloxy group and Y, $Q_1$ and $Q_6$ each are a hydrogen atom. Notably, conversion, through elimination reaction, of the leaving substituent-containing compoundshould not be construed as being limited to the following example.

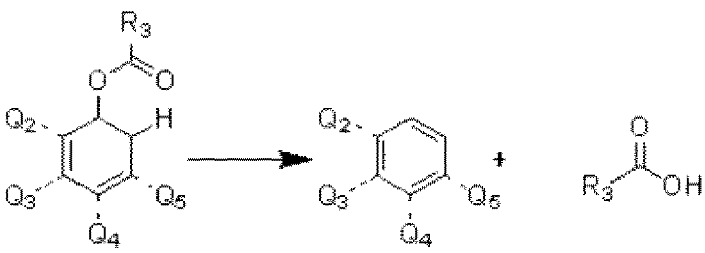

(VI) (VII) (VIII)

In the above formula, a cyclohexadiene structure represented by General Formula (VI) is converted by application of energy (heat) to a benzene ring-containing specific compound represented by General Formula (VII) as a result

of removal of an alkyl chain-containing carboxylic acid represented by General Formula (VIII) as the eliminated component. When the heating temperature exceeds the boiling point of the carboxylic acid, the carboxylic acid is rapidly vaporized.

The mechanism by which the eliminated component is removed from the compound represented by General Formula (VI) is outlined through the following reaction formula (scheme). Notably, in the following reaction scheme, the mechanism by which the eliminated component is removed from the cyclohexadiene structure corresponds to conversion from General Formula (VI-a) to General Formula (VII-a). For detail explanation, the mechanism by which the eliminated component is removed from the cyclohexene structure [General Formula (IX)] is also shown. In the following formula, $R_3$ and $R_6$ each represent a substituted or unsubstituted alkyl group.

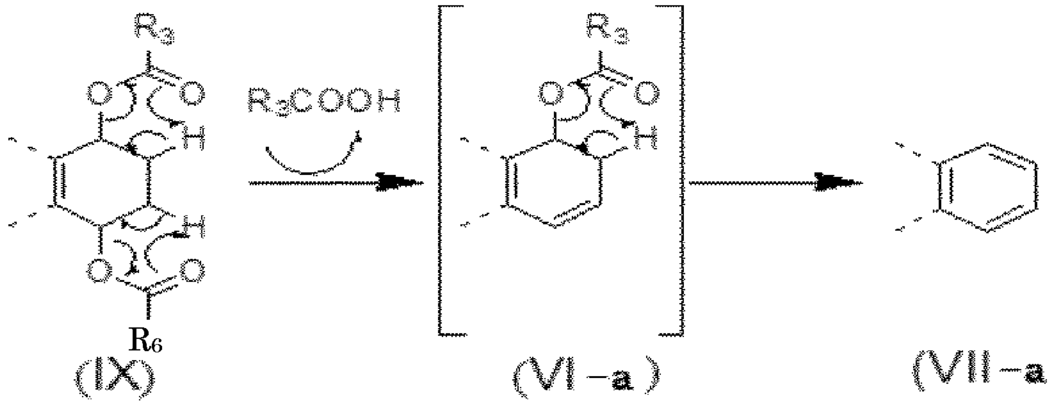


(IX) (VI-a) (VII-a)

As shown in the above reaction formula, the cyclohexadiene structure represented by General Formula (VI-a) is converted to the benzene structure represented by General Formula (VII-a) via a transition state of a six-membered ring structure. In this transition state, the hydrogen atom on the β-carbon and the oxygen atom of the carbonyl group are 1,5-transposed to cause concerted elimination reaction, so that a carboxylic acid compound is removed.

The elimination reaction of the compound [General Formula (IX)] having a cyclohexene structure with two acyloxy groups is thought to proceed through two steps. First, one carboxylic acid is removed to form the cyclohexadiene structure represented by General Formula (VI-a).

In this step, the activation energy necessary for removing one carboxylic acid from the disubstituted compound represented by General Formula (IX) is sufficiently larger than that necessary for removing one carboxylic acid from the monosubstituted compound represented by General Formula (VIa). Thus, the elimination reaction smoothly proceeds through two steps, to thereby form the compound represented by General

Formula (VII-a). In other words, the monosubstituted compound represented by General Formula (VI-a) cannot be isolated from the reaction system in the above reaction formula.

Even when there are several stereoisomers depending on the positions of the substituents (e.g., acyloxy group and hydrogen), the above reaction can proceed although the reaction rate is different.

As is inferred from the above reaction formula, synthesis of an active monosubstituted compound is advantageous since energy necessary for elimination reaction of the active monosubstituted compound is lower than that necessary for elimination reaction of the cyclohexene structure represented by General Formula (IX). That is, the leaving substituent can be removed from the cyclohexadiene structure by energy (external stimulus) lower than in the conventional compounds.

The effect that the leaving substituent can be removed from the above cyclohexadiene structure at lower temperatures can be obtained not only when the substituent is an acyloxy group but also when the substituent is an ether group, etc. The ether group, etc. requires high energy for elimination in the conventional cyclohexene skeleton, and thus is not suitably employed. However, in the skeleton as disclosed herein, the ether group requires low energy for elimination and can be employed similar to the acyloxy group.

In the above reaction formula, since removal and transition of the hydrogen atom on the β-carbon are the first step of the reaction, the stronger the force of the oxygen atom to attract the hydrogen atom, the easier the reaction occurs. The force of the oxygen atom to attract the hydrogen atom is changed, for example, according to the type of the alkyl chain at the side of the acyloxy group, or by replacing the oxygen atom with a chalcogen atom such as sulfur, selenium, tellurium, and polonium which belong to the same group 16 as the oxygen atom does.

Examples of the energies applied for performing elimination reaction include heat, light and electromagnetic wave. Heat or light is preferred in terms of reactivity, yield or post treatment. Particularly preferred is heat. Alternatively, in the presence of acid or base, the aforementioned energies may be applied.

Generally, the above elimination reaction depends on the structure of a functional group. However, most cases

of elimination reaction need heating from the standpoints of reaction speed and reaction ratio. Examples of heating methods for performing elimination reaction include, but not limited thereto, a method for heating on a support, a method for heating in an oven, a method for irradiation with microwave, a method for heating by converting light to heat using a laser beam, and a method using a photothermal conversion layer.

Heating temperature for performing elimination reaction may be a room temperature (approximately 25°C) to 500°C. In consideration of thermal stability of the materials and a boiling point of the eliminated components as to the lower limit of the temperature, and in consideration of energy efficiency, percentage of the presence of unconverted molecule, and the sublimation and decomposition of the compound after conversion as to the upper limit of the temperature, the temperature is preferably 40°C to 500°C. Moreover, in consideration of thermal stability of the leaving group-containing compound during synthesis, the temperature is more preferably 60°C to 500°C, and particularly preferably 80°C to 400°C.

As to the heating time, the higher the temperature is, the shorter the reaction time becomes. The lower the temperature is, the longer the time required for elimination reaction becomes. Heating time depends on the reactivity and amount of the leaving substituent-containing compound, and is generally 0.5 min to 120 min, preferably 1 min to 60 min, and particularly preferably 1 min to 30 min.

In the case where light is used as the external stimulus, for example, infrared lamp or irradiation of light of wavelength absorbed by a compound (for example, exposure to light of wavelength 405 nm or less) may be used. On this occasion, a semiconductor laser may be used. Examples of semiconductor laser beam include a near-infrared region laser beam (generally, a laser beam of wavelength around 780 nm), a visible laser beam (generally, a laser beam of wavelength in the range of 630 nm to 680 nm), and a laser beam of wavelength of 390 nm to 440 nm. Particularly preferable laser beam is a laser beam having a wavelength region of 390 nm to 440 nm, and a semiconductor laser beam having a laser emission wavelength of 440 nm or less is preferably used. Among these semiconductor laser beam, examples of preferable light sources include a bluish-violet semiconductor laser beam having an emission wavelength region of 390 nm to 440 nm (more preferably from 390 nm to 415 nm), and a bluish-violet SHG laser beam having a center emission wavelength of 425 nm that has been converted to a half wavelength of the infrared semiconductor laser beam having a center emission wavelength of 850 nm by using an optical waveguide element.

In the elimination reaction of the leaving substituents, the acid or base serves as a catalyst, and conversion can be performed at lower temperature. A method of using the acid or base is not particularly limited. Examples of the method include a method in which the acid or base may be directly added to the leaving substituent-containing compound, a method in which the acid or base is dissolved in any solvent to form a solution, and the solution is added to the leaving substituent-containing compound, a method in which the leaving substituent-containing compound is heated in the vaporized acid or base, and a method in which a photoacid generator and a photobase generator are used, and followed by light irradiation, to thereby obtain an acid and base in the reaction system.

Examples of the acids include, but not limited thereto, hydrochloric acid, nitric acid, sulfuric acid, acetic acid, trifluoroacetic acid, trifluoromethanesulfonic acid, 3,3,3-trifluoropropionic acid, formic acid, phosphoric acid and 2-butyl octanoic acid.

Examples of the photoacid generators include ionic photoacid generators such as sulfonium salt, and an iodonium salt; and nonionic photoacid generators such as imide sulfonate, oxime sulfonate, disulfonyl diazomethane, and nitrobenzyl sulfonate.

Examples of the bases include, but not limited thereto, hydroxides such as sodium hydrate, potassium hydrate, carbonates such as sodium hydrogen carbonate, sodium carbonate, potassium carbonate, amines such as triethylamine and pyridine, and amidines such as diazabicycloundecene, diazabicyclononene.

Examples of photobase generators include carbamates, acyloximes, and ammonium salts.

The elimination reaction is preferably performed in a volatile acid or base atmosphere from the standpoint of easiness of removal of the acid or base to the outside of the system after reaction.

The elimination reaction can be performed in an ambient atmosphere regardless of the absence or presence of the catalyst. Elimination reaction is preferably performed in an inert gas atmosphere (e.g., nitrogen or argon) or reduced pressure in order to reduce any influence of side reaction such as oxidation or influence of moisture, and to promote removal of an eliminated component to outside the system.

In addition to the method of obtaining carboxylate by reacting the alcohol described below with carboxylic acid chloride or carboxylic acid anhydride, or through exchange reaction between a halogen atom and silver carboxylate or carboxylic acid-quaternary ammonium salt, examples of methods for forming the leaving substituents include, but not limited thereto, a method in which phosgene is reacted with alcohol so as to obtain a carbonate ester, a method in which carbon disulfide is added in alcohol, and alkyl iodide is reacted therewith to obtain xanthate ester, a method in which tertiary amine is reacted with hydrogen peroxide or carboxylic acid so as to obtain amine oxide, and a method in which ortho selenocyano nitrobenzene is reacted with alcohol so as to obtain selenoxide.

[3. Method for producing Leaving Substituent-Containing Compound]

As described above, the leaving substituent-containing compound has a cyclohexadiene skeleton and a leaving substituent.

Since the structure of the cyclohexadiene skeleton and the leaving substituent is sterically bulky but not stiff, the crystallinity is poor. Thus, a molecule having such structure excels in solubility, and has properties of easily obtaining a film having low crystallinity (or an amorphous film), when a solution of the leaving substituent-containing compound is applied.

Next will be described in detail one exemplary method for forming a cyclohexadiene skeleton, an ether group and an acyloxy group.

The ether group or acyloxy group on the cyclohexadiene skeleton can be derived from a compound represented by the following General Formula (X) having a cyclohexen-1-one skeleton. The compound represented by General Formula (X) can be produced by a conventionally known method, and be used as a starting material. Considering the form of elimination reaction, the compound represented by General Formula (X) preferably has one or more hydrogen atoms at position 2 adjacent to the ketone group.

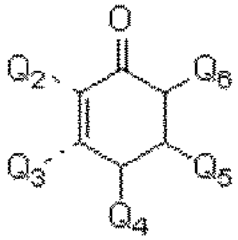

(X)

In formula (X), $Q_2$ to $Q_6$ have the same meanings as defined in General Formula (I).

Next, as shown in the following reaction formula, a reducing agent is used to reduce the ketone group at position 1 of the compound represented by General Formula (X) to an alcohol, to thereby form a compound represented by General Formula (XI).

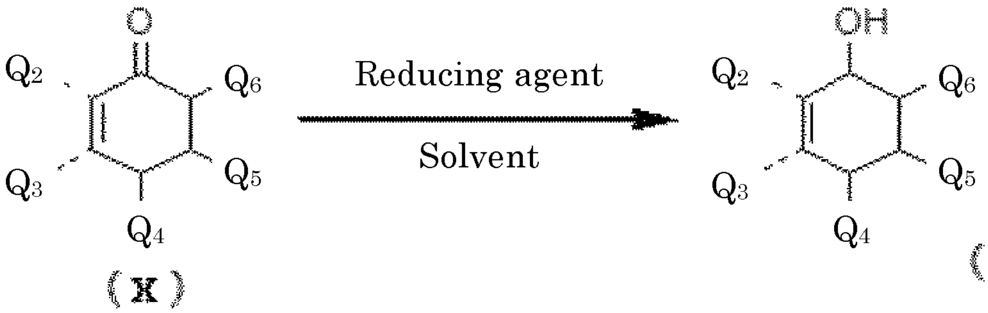


(X) (XI)

In formulas (X) and (XI), $Q_2$ to $Q_6$ have the same meanings as defined in General Formula (I).

The method for the reduction reaction is, for example, hydride reduction using a reducing agent such as sodium borohydride or lithium aluminum hydride; or catalytic reduction using hydrogen and a metal catalyst such as nickel, copper, ruthenium, platinum or palladium. Considering selectivity of a functional group or easiness of reaction, hydride reduction is more preferred.

The solvent used for the reduction reaction may be various organic solvents. In particular, alcohols such as methanol and ethanol are preferred from the viewpoint of reaction rate.

The reaction temperature is near 0°C, in general. Depending on the reactivity, the reaction temperature may be room temperature to the reflux temperature of the solvent used.

Subsequently, as shown in the following reaction formula, the compound represented by General Formula (XI) obtained through the above reaction is converted to a compound represented by General Formula (XII), so that the OH group of the alcohol compound is protected. The protective group employable is not particularly limited and examples thereof include an acetyl group, a methyl group, a trimethylsilyl group and a benzyl group. Among others, the protective group that can be removed under basic conditions is preferably used since the reaction conditions of the subsequent step (described below) are basic conditions. As a result, the number of steps can be reduced.

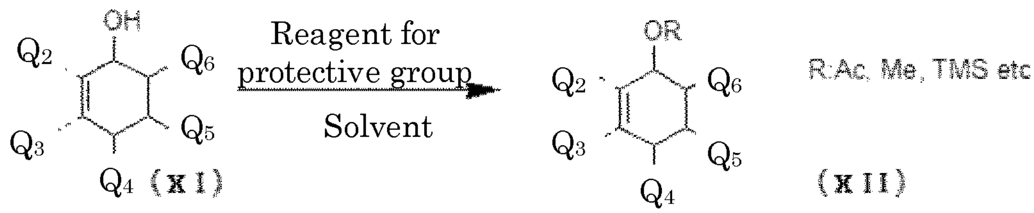


In formulas (XI) and (XII), $Q_2$ to $Q_6$ have the same meanings as defined in General Formula (I), and R represents a protective group for the OH group.

Examples of the protective group for the OH group include an acetyl group, a methyl group, a trimethylsilyl group and a benzyl group.

In the above reaction formula, one example of using the protective group that can be removed under basic conditions will be described.

The compound represented by formula (XII) is reacted with 1 eq. of carboxylic anhydride (e.g., acetic anhydride) in the presence of a base to form a carboxylic acid ester (XII-1). The base suitably usable is a tertially amine such as pyridine or triethylamine. An excessive amount of the base is used as a solvent.

In addition to the above, the solvent may be various inert organic solvents such as dichloromethane and tetrahydrofuran.

Subsequently, as shown in the following reaction formula, the compound represented by General Formula (XII-1) obtained through the above reaction is selectively halogenated at position 4 with 1 eq. of a halogenating agent to form a compound represented by General Formula (XIII).

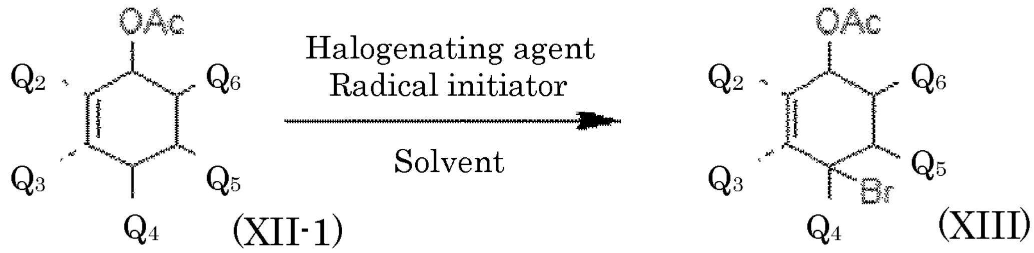


In formulas (XII-1) and (XIII), $Q_2$ to $Q_6$ have the same meanings as defined in General Formula (I), and Ac denotes an acetyl group.

In the above reaction formula, the halogen atom introduced into position 4 is preferably an iodine atom, a bromine atom or a chlorine atom, particularly preferably a bromine atom, considering the reactivity in the subsequent step. Examples of brominating agents include N-bromosuccinimide, N-iodosuccinimide and N-chlorosuccinimide. Bromination is preferably performed in the presence of a radical initiator (e.g., azobisisobutyronitrile or benzoyl peroxide) in combination with the brominating agent. The solvent is not necessarily used but various organic solvents may be used. In particular, benzene and carbon tetrachloride are suitably used. The reaction temperature may be room temperature to the reflux temperature of the solvent used.

In this step, there may be several stereoisomers depending on the reaction conditions. Specifically, a racemic mixture and a meso-form compound may be obtained at any ratio depending on the steric configuration of the carboxylic acid ester and the bromo group which are linked to the cyclohexene structure. Although it is not necessary to separate them, they can be separated through, for example, recrystallization or chromatography using an optically active stationary phase.

Subsequently, as shown in the following reaction formula, the compound represented by General Formula (XIII) obtained through the above reaction is allowed to undergone double bond formation through elimination of the bromo group using a base as well as conversion to a hydroxyl group through deprotection, to form a compound represented by General Formula (XIV).

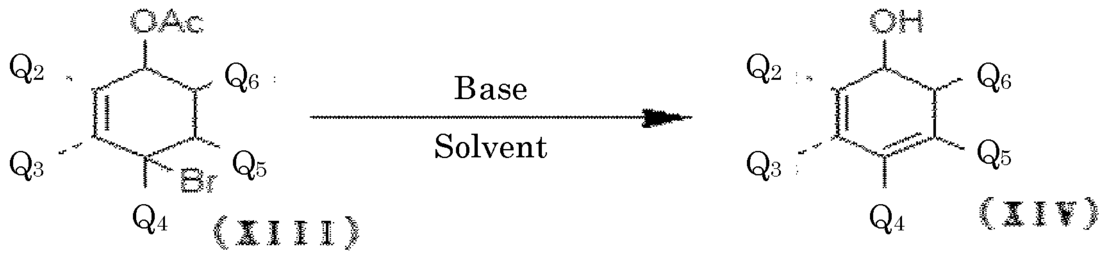


(XIII) (XIV)

In formulas (XIII) and (XIV), Ac denotes an acetyl group.

In the above reaction formula, the base may be, for example, sodium methoxide, sodium ethoxide, sodium hydroxide or potassium hydroxide. A strong base is particularly preferred since the protective group of the hydroxyl group can be removed simultaneously with double bond formation. The solvent used is not particularly limited but alcohol solvents such as methanol and ethanol are preferred from the viewpoint of reactivity.

Regarding the synthesis of the above alcohol compound, an alternative method may be employed for a polycyclic aromatic compound having an active K region (encircled in the below structural formulas). Specifically, a hydroxyl group may be introduced into the active region relatively easily so as to be substituted with a leaving group. Examples of such a polycyclic aromatic compound include phenanthrene, pyrene, chrysene, benzopyrene, picene and benzopicene.

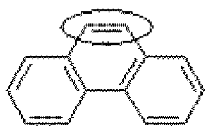

Phenanthrene

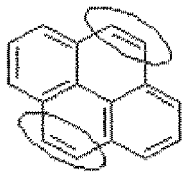

Pyrene

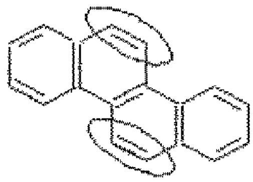

Chrysene

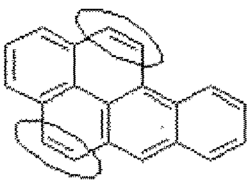

Benzopyrene

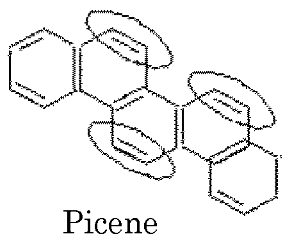

Picene

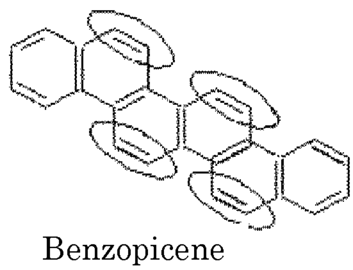

Benzopicene

Next will be described introduction of a hydroxyl group into the K region of phenanthrene having a structure represented by General Formula (XIV) where two benzene rings are fused with the cyclohexadiene structure at ($Q_2$, $Q_3$) and ($Q_4$, $Q_5$).

First, as shown in the following reaction formula, the K region of phenanthrene (positions 9 and 10) are epoxidated with an oxidizing agent to form an epoxy derivative represented by General Formula (XV).

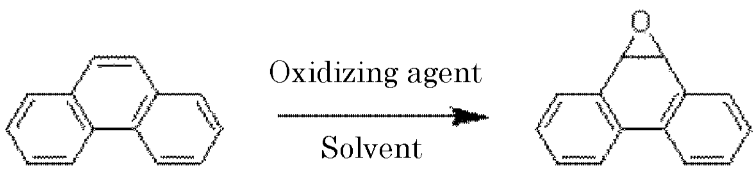


(XV)

The epoxidation of the K region may be performed with organic or inorganic peroxides such as m-peroxybenzoic acid, hydrogen peroxide, peracetic acid, oxone, dimethyldioxirane and aqueous sodium hypochlorite solution, which are conventionally known oxidizing agents used for epoxidation. Of these, m-peroxybenzoic acid, hydrogen peroxide and aqueous sodium hypochlorite solution are preferred from the viewpoint of easy handling.

The solvent used is not particularly limited, so long as it can sufficiently dissolve the compound and is not oxidized with the oxidizing agent. Examples thereof include dichloromethane, chloroform, carbon tetrachloride, benzene

and water, with dichloromethane, chloroform and water being particularly preferred.

Also, when such an oxidizing agent as aqueous sodium hypochlorite solution is used, preferably, the compound is dissolved in the organic phase, where the reaction is performed in the presence of a phase transfer catalyst (i.e., two-phase system). The phase transfer catalyst may be a surfactant. Examples thereof include surfactants mainly containing quarternary ammonium salts or sulfonium salts.

The reaction temperature is near 0°C, in general. Depending on the reactivity, the reaction temperature may be room temperature to the reflux temperature of the solvent used.

First, as shown in the following reaction formula, the epoxy derivative represented by General Formula (XV) is reduced with a reducing agent to form an alcohol compound of interest represented by General Formula (XVI).

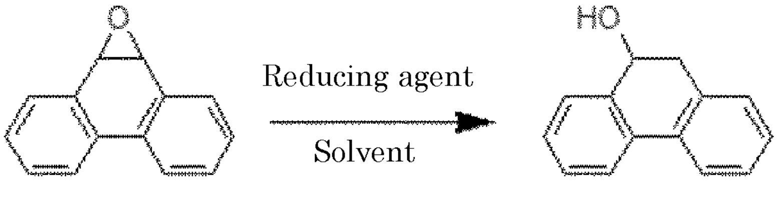


(XV) (XVI)

The method for the reduction reaction is, for example, hydride reduction using a reducing agent such as sodium borohydride, lithium aluminum hydride (LAH) or diisobutylaluminum hydride (DIBAL); or catalytic reduction using hydrogen and a metal catalyst such as nickel, copper, ruthenium, platinum or palladium. Considering selectivity of a functional group or easiness of reaction, hydride reduction is more preferred. Among the hydride reducing agents, relatively strong reducing agents are preferred for epoxy reduction. Examples thereof include lithium aluminum hydride (LAH) or diisobutylaluminum hydride (DIBAL).

The solvent used for the reduction reaction may be various organic solvents. However, the solvent used must not react with the reducing agent. In particular, ether solvents are preferred. Examples thereof include diethyl ether, tetrahydrofuran, dimethoxyethane and dioxane.

The reaction temperature is near 0°C, in general. Depending on the reactivity, the reaction temperature may be room temperature to the reflux temperature of the solvent used.

Subsequently, as shown in the following reaction formula, the compound (alcohol compound) represented by General Formula (XIV) obtained through the above reaction is treated with a base and a carboxylic anhydride, a carboxylic chloride or carbonate half esters (e.g., alkyl chloroformate) to form a compound represented by General Formula (XVII) (i.e., a target compound in which position 1 has been acyloxylated).

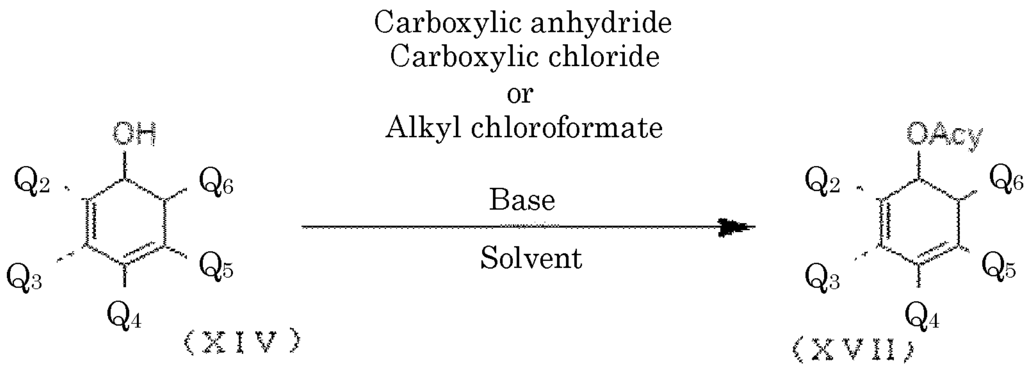


(XIV) (XVII)

In formulas (XIV) and (XVII), $Q_2$ to $Q_6$ have the same meanings as defined in General Formula (I), and Acy denotes an acyl group.

Examples of the carboxylic anhydride, carboxylic chloride or carbonate halfesters (e.g., alkyl chloroformate) include those derived from carboxylic acids (e.g., acetic acid, butyric acid, valeric acid, propionic acid, pivalic acid, caproic acid, stearic acid, trifluoroacetic acid and 3,3,3-trifluoropropionic acid). In the above reaction formula, the base may be, for example, pyridine, triethylamine, sodium hydroxide, potassium hydroxide, potassium carbonate or sodium hydride. In the acylation, it is not necessarily to use a strong base, if hydrochloric acid generated during reaction can be trapped. The solvent used may be various organic solvents such as pyridine, triethylamine (these serving also as the base), dichloromethane, tetrahydrofuran and toluene. The solvent is preferably dehydrated to the greatest extent possible before use from the viewpoints of reaction rate and prevention of side reactions.

The reaction temperature may be room temperature to the reflux temperature of the solvent used. To prevent side reactions such as elimination reaction, the reaction temperature is preferably 50°C or lower, particularly preferably room temperature (near 25°C) or lower.

Notably, when an alkyl halide, an alkylsilane halide or a sulfonic acid derivative is used at the above step instead of the carboxylic acid derivative, a skeleton having an alkyl ether group, a silyl ether group or a sulfonyloxy group can easily be formed with a known method.

Notably, when an alkyl halide or an alkylsilane halide is used, use of a strong base (e.g., sodium hydride, sodium hydroxide or potassium carbonate) is better than use of a weak base (e.g., triethylamine or pyridine).

The soluble substituent synthesized according to the above-described reaction formula can be used to produce a leaving substituent-containing compound (a ring-fused compound; e.g., the compound represented by General Formula (V)) through ring fusion using various conventionally known methods. Production of an organic semiconductor precursor compound, for example, heteroacenes, may be performed according to the method described in, for example, J. Am. Chem. Soc. 2007, 129, pp. 2224-2225.

The reaction formula (scheme) thereof will be given below in detail.

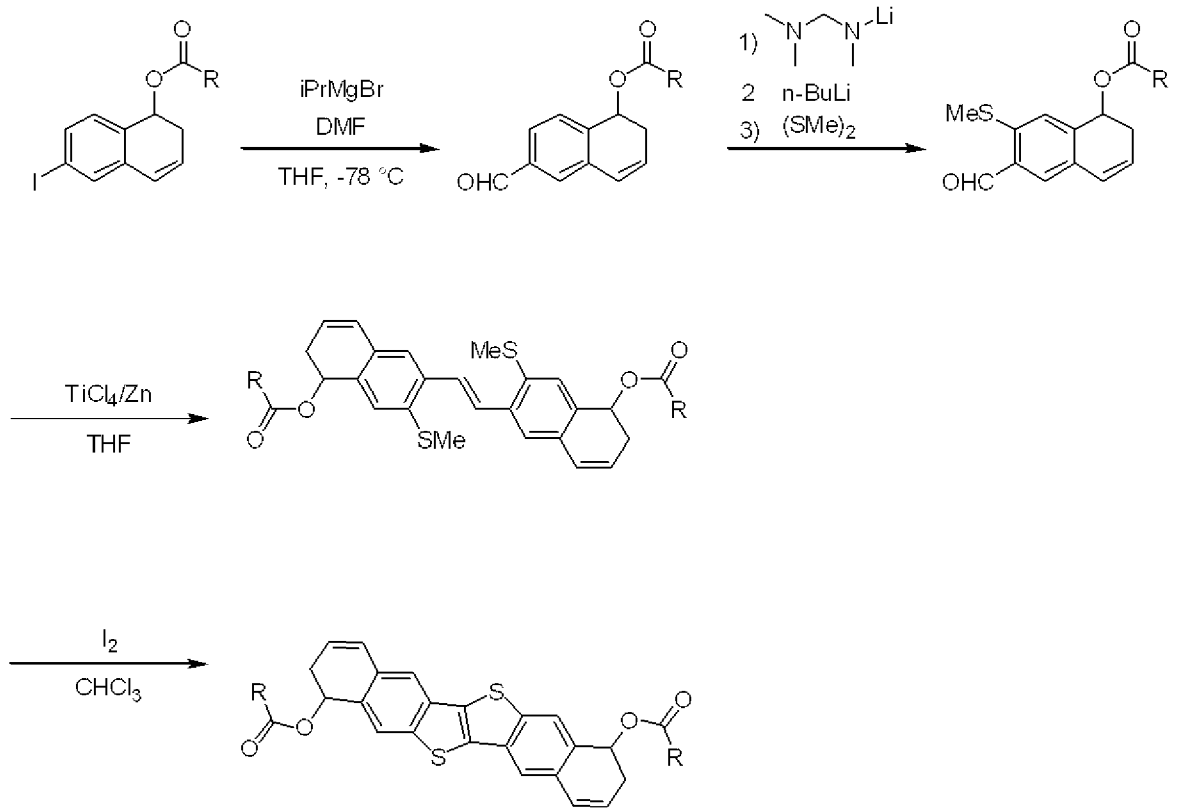


1-Acyloxy-6-iodo-1,2-dihydronaphthalene serving as a starting material used in the above reaction formula can be synthesized according to Synthesis Example.

As the first step, a Grignard exchange reaction is performed between an iodine atom and a Grignard reagent. Because of cryogenic reaction temperature and high reactivity of iodine, the Grignard exchange reaction selectively occurs so as to obtain a Grignard reagent. To the Grignard reagent, a formylation agent, such as dimethylformamide or morpholine, is added so as to perform formylation.

The second step is ortho-lithiation of a formyl group. Since amine and lithium added at the same time form a complex with the formyl group, the ortho position (position 7 of 1,2-dihydronaphthalene) is selectively lithiated without impairing other functional groups. To the lithiated formyl group, dimethyl sulfide is added, so as to form a SMe group.

Thereafter, in the third step, the formyl groups are subjected to McMurry Coupling reaction. The reaction is performed in the presence of zinc and titanium tetrachloride. Therefore, the formyl groups are coupled to form an olefin structure.

In the final step, ring-closing reaction with iodine is performed. Iodine is attached to a double bond portion, reacted with the SMe group, and eliminated in the form of MeI, to thereby form two thiophene rings. Thus, a desired ring-fused compound can be obtained.

Pentacene is ring-fused in accordance with the method described in J. Am. Chem. Soc., 129, 2007, pp. 15752.

In the case of phthalocyanines, ring formation reaction can be performed in accordance with "Phthalocyanine -chemical and function-", Hirofusa Shirai, Nagao Kobayashi, 1997, (IPC) pp. 1 to 62, and "Phthalocyanine as a functional dye" Ryo Hirohashi, Keiichi Sakamoto, Eiko Okumura, 2004 (IPC) pp. 29 to 77. Porphyrins are ring-fused in accordance with the method described in JP-A No. 2009-105336.

The leaving substituent-containing compound disclosed herein is bonded via a covalent bond to other skeletons of the solvent solubililty-imparting substituents (also referred to as "solvent-soluble substituent") (B in the following General Formula) by known methods for coupling reaction. Examples of the known coupling reactions include Suzuki coupling reaction, Stille coupling reaction, Kumada coupling reaction, Negishi coupling reaction, Hiyama coupling reaction, Sonogashira reaction, Heck reaction and Wittig reaction.

Of these, Suzuki coupling reaction and Stille coupling reaction are particularly preferred in terms of easy derivatization of an intermediate, reactivity and yield. For formation of carbon-carbon double bond, Heck reaction, and Wittig reaction are preferable in addition to the above-described reactions. For formation of carbon-carbon triple bond, Sonogashira reaction is preferable in addition to the above-described reactions.

Hereinafter, an embodiment in which a carbon-carbon bond is formed by Suzuki coupling reaction and Stille coupling reaction will be described.

Among compounds represented by General Formulas (XVIII) and (XIX), the reaction is performed using a combination of a halogenated product with a trifluoro triflate product, or a combination of a boronic acid derivative with an organotin derivative. However, in the case where the compounds represented by General Formulas (XVIII) and (XIX) are both a halogenated product and a triflate product, or a boronic acid derivative and an organotin derivative, coupling reaction does not occur. Such cases are excluded.

In the mixture described above, base is further added only in the case of Suzuki coupling reaction, and the mixture is reacted in the presence of a palladium catalyst.

$$Ar\text{-}(D)\ell \qquad (XVIII)$$

In General Formula (XVIII), Ar denotes an aryl group or a heteroaryl group, as described above; D denotes a halogen atom (a chlorine atom, a bromine atom, or an iodine atom), a triflate (trifluoromethanesulfonyl) group, boronic acid or ester thereof, or an organotin functional group; and 1 denotes an integer of 1 or more.

$$B\text{-}(C)k \qquad (XIX)$$

In General Formula (XIX), B denotes a solvent soluble substituent as described above; C denotes a halogen atom (a chlorine atom, a bromine atom, or an iodine atom), a triflate (trifluoromethanesulfonyl) group, boronic acid or ester thereof, or an organotin functional group; and k denotes an integer of 1 or more.

In the synthesis method by Suzuki coupling reaction, Stille coupling reaction, among halogenated products or triflate products in General Formulas (XVI) and (XVII), iodine products, bromine products, and triflate products are preferable, from the standpoint of reactivity.

As the organotin functional group in General Formulas (XVIII) and (XIX), a derivative having an alkyl tin group such as a $SnMe_3$ group or a $SnBu_3$ group can be used. The derivatives can be easily obtained in such a manner that hydrogen or a halogen atom in a desired position is replaced with lithium or a Grignard reagent using an organic metal reagent such as n-butyllithium, followed by quenching using trimethyltin chloride or tributyltin chloride.

As the boronic acid derivative, in addition to a boronic acid, a boronic ester derivative may be used. The boronic ester derivative is synthesized from a halogenated derivative using bis(pinacolato)diboron which has thermal stability and is easily handled in air, or synthesized by protecting boronic acid with diol such as pinacol.

As described above, either the substituent D or the substituent C may be halogen and a triflate product, or a boronic acid derivative and an organotin derivative. From the standpoints of easiness of derivatization and reduction of secondary reaction, it is preferred that the substituent D be a boronic acid derivative and an organotin derivative.

For the Stille coupling, reaction base is not necessary, while for the Suzuki coupling reaction base is necessary, and a relatively weak base, such as $Na_2CO_3$, or $NaHCO_3$ contributes to a good result. In the case where steric hindrance effects on the reaction, a strong base such as $Ba(OH)_2$, $K_3PO_4$ or NaOH is effective. Additionally, caustic potash and metal alkoxides, such as potassium t-butoxide, sodium t-butoxide, lithium t-butoxide, potassium 2-methy-2-butoxide, sodium 2-methy-2-butoxide, sodium methoxide, sodium ethoxide, potassium ethoxide and potassium methoxide may be also used as the bases. Moreover, organic bases such as triethylamine may be also used.

Examples of the palladium catalysts include palladium bromide, palladium chloride, palladium iodide, palladium cyanide, palladium acetate, palladium trifluoroacetate, palladium acetyl acetonato[$Pd(acac)_2$], diacetate bis(triphenylphosphine)palladium[$Pd(OAc)_2(PPh_3)_2$], tetrakis(triphenylphosphine)palladium[$Pd(PPh_3)_4$], dichloro bis(acetonitrile)palladium[$Pd(CH_3CN)_2Cl_2$], dichloro bis (benzonitrile)palladium[$Pd(PhCN)_2Cl_2$], dichloro[1,2-bis(diphenylphosphino)ethane]palladium[$Pd(dppe)Cl_2$], dichloro[1,1 - bis(diphenylphosphino)ferrocene]palladium[$Pd(dppf)Cl_2$], dichloro

bis (tricyclohexylphosphine)palladium[Pd[P($C_6H_{11}$)$_3$]$_2$Cl$_2$], dichloro bis(triphenylphosphine)palladium[Pd(PPh$_3$)$_2$Cl$_2$], tris (dibenzylideneacetone)dipalladium[Pd$_2$(dba)$_3$], and bis(dibenzylideneacetone)palladium[Pd(dba)$_2$]. Of these, phosphine catalysts such as tetrakis(triphenylphosphine)palladium[Pd(PPh$_3$)$_4$], dichloro[1,2-bis(diphenylphosphino)ethane]palladium[Pd(dppe)Cl$_2$], dichloro bis(triphenylphosphine)palladium[Pd(PPh$_3$)$_2$Cl$_2$] are preferred.

In addition to the above-described palladium catalysts, a palladium catalyst synthesized by reaction of a palladium complex and a ligand in a reaction system can be also used. Example of the ligands include triphenylphosphine, trimethylphosphine, triethylphosphine, tris(n-butyl)phosphine, tris(tert-butyl)phosphine, bis(tert-butyl)methylphosphine, tris(i-propyl)phosphine, tricyclohexylphosphine, tris(o-tolyl)phosphine, tris(2-furyl)phosphine, 2-dicyclohexylphosphinobiphenyl, 2-dicyclohexylphosphino-2'-methylbiphenyl, 2-dicyclohexylphosphino-2',4',6'-triisopropyl-1,1'-biphenyl, 2-dicyclohexylphosphino-2',6'-dimethoxy-1,1'-biphenyl, 2-dicyclohexylphosphino-2'-(N,N'-dimethylamino)biphenyl, 2-diphenylphosphino-2'-(N,N′-dimethylamino)biphenyl, 2-(di-tert-butyl)phosphine-2'-(N,N′-dimethylamino)biphenyl, 2-(di-tert-butyl)phosphinobiphenyl, 2-(di-tert-butyl)phosphino-2'-methylbiphenyl, diphenylphosphino ethane, diphenylphosphino propane, diphenylphosphino butane, diphenylphosphino ethylene, diphenylphosphino ferrocene, ethylenediamine, N,N′,N″,N‴-tetramethylethylenediamine, 2,2'-bipyridyl, 1,3-diphenyldihydro imidazolylidene, 1,3-dimethyl dihydroimidazolylidene, diethyl dihydroimidazolylidene, 1,3-bis(2,4,6-trimethylphenyl)dihydroimidazolylidene and 1,3-bis(2,6-diisopropylphenyl)dihydroimidazolylidene. A palladium catalyst in which any of these ligands coordinates can be used as a cross coupling catalyst.

A reaction solvent preferably has no functional group reactive with a starting material and can appropriately dissolve the starting material. Examples thereof include: water; alcohols and ethers such as methanol, ethanol, isopropanol, butanol, 2-methoxyethanol, 1,2-dimethoxyethane, bis(2-methoxyethyl)ether; cyclic ethers such as dioxane, tetrahydrofuran; benzene; toluene; xylene; chlorobenzene; dichlorobenzene; dimethyl sulfoxide (DMSO); N,N-dimethylformamide (DMF); N,N-dimethylacetamide; N-methylpyrrolidone; and 1,3-dimethyl-2-imidazolidinone. These solvents may be used alone or in combination. Moreover, it is preferred that these solvents be preliminarily dried and deaerated.

The temperature of the above-described reaction may be appropriately set depending on the reactivity of a starting material used or a reaction solvent. It is generally 0°C to 200°C. However, the reaction temperature is preferably a boiling point or lower of the solvent in any case. Additionally, the temperature is preferably set to a temperature at which elimination reaction occurs or lower in terms of yield. Specifically, the reaction temperature is preferably room temperature to 150°C, particularly preferably room temperature to 120°C, most preferably room temperature to 100°C.

The reaction time of the above reaction may be appropriately set depending on the reactivity of a starting material used. It is preferably 1 hour to 72 hours, more preferably 1 hour to 24 hours.

Through the above-described reaction, the leaving substituent-containing compound represented by the following General Formula (XX) can be obtained.

$$Ar\text{-}(B)_m \qquad (XX)$$

In General Formula (XX), Ar has the same meaning as defined in General Formula (XVIII), B has the same meaning as defined in General Formula (XIX), and m is an integer of 1 or more.

The obtained leaving substituent-containing compound is used after removal of impurities such as the catalyst used for reaction, unreacted starting materials, or by-products generated upon reaction such as boronic acid salts, organotin derivatives or the like. For the purification, conventionally known methods may be used, for example, reprecipitation, column chromatography, adsorption, extraction (including Soxhlet extraction), ultrafiltration, dialysis, use of scavenger for removing a catalyst, or the like.

Such materials having excellent solubility reduce limitation to these purification methods and as a result, favorably effect on device properties.

In order to form a thin film from the leaving substituent-containing compound obtained by the above-described production method, for example, a solution (e.g., an ink) containing the leaving substituent-containing compound and a solvent may be treated with a conventionally known film forming method such as spin coating, casting, dipping, inkjetting, doctor blade casting or screen printing. Alternatively, the organic semiconductor material itself obtained after conversion may be treated with a conventionally known film forming method such as vacuum vapor deposition or sputtering. Any of these film forming methods enables to form a good thin film having excellent strength, toughness, durability and the like without cracks.

Moreover, energy (external stimulus) is applied to the film of the leaving substituent-containing compound formed by the above film forming method, so as to eliminate the solubility-imparting leaving substituents as the compound (eliminated component) represented by General Formula (II), thereby forming an organic semiconductor film of the specific compound (organic semiconductor material) represented by General Formula (Ia). Therefore, the leaving substituent-containing compound may be used as various materials for functional elements such as photoelectric conversion elements, thin-film transistor elements, light-emitting elements and the like.

[4. Application of Leaving Substituent-Containing Compound to Device]

An organic semiconductor compound produced from the leaving substituent-containing compound can be used in an electronic device. Examples of the electronic devices include devices having two or more electrodes in which current and voltage between the electrodes are controlled by electricity, light, magnetism, chemical materials or the like; and apparatuses for generating light, electrical field, or magnetic field by application of voltage or current. Moreover, examples thereof include elements for controlling current or voltage by application of voltage or current, elements for controlling voltage or current by application of magnetic field, and elements for controlling voltage or current by action of a chemical material. For control, rectification, switching, amplification, oscillation or the like are used.

As a device currently realized using an inorganic semiconductor such as silicon or the like, resistors, rectifiers (diode), switching elements (transistor, thyristor), amplifying elements (transistor), memory elements, chemical sensors or the like, combinations of these elements, integrated devices, or the like are exemplified. Additionally, solar batteries in which electromotive force generated by light, photodiodes for generating photocurrent, photoelements such as phototransistors or the like are used.

As an electronic device, to which the leaving substituent-containing compound disclosed herein and the organic semiconductor compound produced by using the leaving substituent-containing compound are applied, an organic thin-film transistor, namely organic field effect transistor (OFET) is exemplified. Hereinafter, field effect transistor (FET) will be specifically described.

[Structure of Transistor]

FIGs. 1A to 1D are each a schematic view of an exemplary structure of an organic thin-film transistor. An organic semiconductor layer 1 of the organic thin-film transistor contains the organic semiconductor compound [compound represented by General Formula (Ia)] obtained through conversion performed by applying energy to the leaving substituent-containing compound. The organic thin-film transistor includes a first electrode (source electrode 2), a second electrode (drain electrode 3) and a third electrode (gate electrode 4), which are provided on a support (substrate) (not shown) with being separated each other. A insulating film 5 may be provided between the gate electrode 4 and the organic semiconductor layer 1.

The organic thin-film transistor is configured to control the current flowing through the organic semiconductor layer 1 between the source electrode 2 and the drain electrode 3 by applying voltage to the gate electrode 4. It is important for a switching element to largely modulate the amount of the current flowing between the source electrode 2 and the drain electrode 3 by the conditions of applying voltage from the gate electrode 4. This means that large current flows depending on the drive state of the transistor, and no current flows in non drive state of the transistor.

The organic thin-film transistor may be formed on the substrate. As the substrate, a typical substrate formed of, for example, glass, silicon, plastic or the like may be used. A conductive substrate can be used to serve as the gate electrode. The gate electrode and the conductive substrate may be layered. However, a plastic sheet is preferably used as the substrate in case where a device, to which the organic thin-film transistor is applied, is expected to have properties such as flexibility, lightweight, lower production cost and shock resistance.

Examples of the plastic sheets include films of polyethylene terephthalate, polyethylene naphthalate, polyether sulfone, polyetherimide, polyether ether ketone, polyphenylene sulfide, polyarylate, polyimide, polycarbonate, cellulose triacetate, and cellulose acetate propionate.

[Film Deposition Method: Organic Semiconductor Layer]

As described above, the leaving substituent-containing compound is used as an organic semiconductor precursor, and the organic semiconductor precursor is dissolved in a solvent such as dichloromethane, tetrahydrofuran, chloroform, toluene, chlorobenzene, dichlorobenzene and/or xylene, and the resultant solution is applied on a substrate so as to form a thin film of the organic semiconductor precursor. Then, an energy may be applied to the thin film of the organic semiconductor precursor so as to be converted into an organic semiconductor film.

As described above, the leaving substituent-containing compound has a cyclohexadiene structure and an acyloxy group, which are sterically bulky, and poor crystallinity. A molecule having such structure excels in solubility and has properties of easily obtaining a film having low crystallinity (amorphous), when a solution containing the molecule is applied.

Examples of methods for depositing the thin films include spray coating, spin coating, blade coating, dipping, casting, roll coating, bar coating, dye coating, inkjetting and dispensing; printing methods such as screen printing, offset printing, relief printing, flexographic printing; soft lithography such as a micro contact printing. Moreover, these methods may be used in combination.

From the above-described deposition methods and solvents, a deposition method and solvent may be appropriately selected according to materials.

The organic semiconductor material itself which has been thermally converted can deposit a film through vapor phase, such as vacuum deposition.

In the organic thin-film transistor, the thickness of the organic semiconductor layer is not particularly limited, and the thickness of the organic semiconductor layer is so selected as to deposit a uniform thin film, namely, a thin film having no gaps and holes that adversely affect the carrier transportation characteristics of the organic semiconductor layer. The thickness of the organic semiconductor layer is generally 1 μm or less, and particularly preferably 5 nm to 100 nm. In the organic thin-film transistor, the organic semiconductor layer deposited from the above mentioned compounds is formed in contact with the source electrode, the drain electrode, and the insulating film.

[Film Deposition Method: Post Treatment of Organic Semiconductor Film]

The properties of the organic semiconductor film converted from the precursor-containing thin film can be improved by post treatment. For example, deformation of the film caused during film deposition can be reduced by heat treatment. This heat treatment enhances the crystallinity, and the properties are improved and stabilized. Moreover, the organic semiconductor film is placed in an organic solvent (such as toluene and chloroform), so that the deformation of the film can be reduced in the same manner as in heat treatment, and the crystallinity can be further enhanced.

Moreover, by exposing the organic semiconductor film to oxidizing or reducing air and/or liquid, such as oxygen, hydrogen, etc. properties can be changed by oxidization or reduction. The oxidization or reduction is used for the purpose of increasing or decreasing the density of carrier in the film.

[Electrode]

The materials of the gate electrode and the source electrode used in the organic thin-film transistor are not particularly limited, as long as conductive materials are used. Examples thereof include platinum, gold, silver, nickel, chromium, copper, iron, tin, antimony, lead, tantalum, indium, aluminum, zinc, magnesium, and alloys thereof; conductive metal oxides such as indium/tin oxides; organic and inorganic semiconductors in which conductivity is improved by doping, etc., such as a silicon single crystal, polysilicon, amorphous silicon, germanium, graphite, polyacetylene, polyparaphenylene, polythiophene, polypyrrol, polyaniline, polythienylene vinylene, polyparaphenylene vinylene, complexes consisting of polyethylene dioxythiophene and polystyrene sulfonic acid.

Of the conductive materials described above, materials having a low electric resistance at the surface in contact with the semiconductor layer are preferred for the source electrode and drain electrode.

Examples of methods for forming an electrode include a method in which a conductive thin film, which has been deposited using the material mentioned above by deposition or sputtering, is formed into an electrode by a known method such as a photolithographic method or liftoff technology; and a method in which an electrode is formed by etching a resist on a metal foil of, for example, aluminum and copper, by thermal transfer, inkjet or the like. In addition, an electrode may be formed by directly patterning by inkjet printing using a solution or dispersion liquid of a conductive polymer or a dispersion liquid of conductive particles, or may be formed from a coated layer by lithography or laser ablation. It is also possible to use a method in which an ink, conductive paste, etc. containing conductive polymers or conductive particles are patterned by a printing method such as relief printing, intaglio printing, planographic printing or screen printing.

The organic thin-film transistor can have an extraction electrode from each electrode if necessary.

[Insulating Film]

The insulating film used in the organic thin-film transistor is formed of various materials for insulating film. Examples thereof include the inorganic insulating materials such as silicon oxide, silicon nitride, aluminum oxide, aluminum nitride, titanium oxide, tantalum oxide, tin oxide, vanadium oxide, barium-strontium-titanium oxide, barium-titanium-zirconium oxide, lead-zirconium-titanium oxide, lead lanthanum titanate, strontium titanate, barium titanate, barium magnesium fluoride, bismuth-niobium-tantalum oxide and yttrium trioxide.

Additionally, examples thereof include polymer compounds such as polyimides, polyvinyl alcohols, polyvinyl phenols, polyesters, polyethylene, polyphenylenesulfides, unsubstituted or halogen atom substituted polyparaxylylene, polyacrylonitrile and cyanoethylpullulan.

These insulating materials may be used in combination. The insulating material is not particularly limited, and it is preferred to select an insulating material having a high dielectric constant and a low conductivity.

Examples of the methods of depositing the insulating film using the insulating materials include dry deposition processes such as a chemical vacuum deposition (CVD), a plasma CVD, a plasma polymerization and vapor deposition; and wet coating processes such as spray coating, spin coating, dip coating, inkjetting, casting, blade coating and bar

coating.

[Modification of Interface between Organic Semiconductor and Insulating Film]

In the organic thin-film transistor, the organic thin film may be provided between the insulating film, electrode and the organic semiconductor layer to improve adhesiveness thereof, decrease gate voltage and reduce leak current. The organic thin film is not particularly limited as long as the organic thin film does not have a chemical effect on an organic semiconductor layer. For example, an organic molecular film and a polymer thin film can be used.

In the case of the organic molecular film, coupling agents such as octyltrichlorosilane, octadecyl trichlorosilane, hexamethylene disilazane and phenyltrichlorosilane, benzenethiol, trifluorobenzenethiol, perfluorobenzenethiol, perfluorodecanethiol, etc. may be used. In addition, as the polymer thin film, the aforementioned polymer insulating materials can be used, and these may function as a sort of the insulating film.

This organic thin film may be subject to an anisotropic treatment by rubbing or the like.

"Protective Layer"

The organic thin-film transistor can be stably driven in the atmosphere. If necessary, a protective layer can be provided in terms of protection from mechanical destruction and moisture and/or gas, and protection required for integration of a device.

"Applied Device"

The leaving substituent-containing compound and the organic semiconductor are useful since they can form various organic electronic devices such as photoelectric conversion elements, thin-film transistor elements and light-emitting elements.

The organic thin-film transistors can be utilized as an element for driving various known image display elements such as liquid crystal, electroluminescence, electrochromic, and electrophoretic migration. When such elements are integrated, it is possible to produce a display referred to as "electronic paper."

The display device includes liquid crystal display elements in the case of a liquid display device, organic or inorganic electroluminescence display elements in the case of an EL display device, and electrophoresis display elements in the case of an electrophoresis display device, and a plurality of such display elements are aligned in the form of matrix in X direction and Y direction to construct the display device using the aforementioned display element as one display picture element (i.e. one pixel). As illustrated in Fig. 2, the display element is equipped with the organic thin-film transistor as a switching element for applying voltage or supplying a current to the display element. The display device includes the same number of the switching elements to the number of the display element, i.e. the number of the display picture elements (i.e., the pixels). Notably, in Fig. 2, reference numeral 6 denotes a source electrode, 7: an organic semiconductor, 8: a drain electrode, 9: an interlayer insulating layer, 10: a pixel electrode, 11: a substrate, 12: a gate electrode, 13: a gate insulating film and 14: a through hole.

The display element contains, other than the switching elements, members such as a substrate, an electrode (e.g., a transparent electrode), a polarizer, and a color filter. These members are suitably selected from those known in the art depending on the intended purpose without any restriction.

When the display device forms a certain image, only certain switching elements selected from all the switching elements provided in the matrix form, as illustrated in Fig. 3, turn on or off for applying voltage or a current to the corresponding display elements. In Fig. 3, reference numeral 15 denotes a scanning line/gate electrode, 16: an organic semiconductor, 17: a source electrode and 18: a drain electrode. When voltage or a current is not applied to the display elements, all the switching elements remain the state of OFF or ON. The display device can display the image at high speed and high contrast by having such configuration. Note that, the display device displays an image by the conventional display operation known in the art.

For example, in the case of the liquid display element, the molecule alignments of the liquid crystals are controlled by applying voltage to the liquid crystals, to thereby display an image or the like. In the case of the organic or inorganic electroluminescence display element, a current is supplied to a light-emitting diode formed of an organic material or inorganic material to emit the organic or inorganic film, to thereby display an image or the like. In the case of the electrophoresis display element, voltage is applied to white coloring particles and black coloring particles each charged with the opposite polarity to each other to make the coloring particles electrically migrate in a certain direction. As a result, an image or the like is displayed.

The display device can be produced by a simple process, such as a process of coating or printing the switching element, can use as a substrate, and a plastic substrate or paper that does not have sufficient resistance to a high temperature processing. Moreover, the display device having a large area can be produced at low energy and cost, as

the switching elements can be formed at low energy and cost.

Moreover, it is also possible to use an IC in which the organic thin-film transistors are integrated as a device such as an IC tag.

[π-Electron conjugated compound and film-like product obtained from π-electron conjugated compound precursor by a production method of the present invention]

In methods of the present invention for producing a film-like product and a π-electron conjugated compound, an external stimulus is applied to a "π-electron conjugated compound precursor" having a specific solvent-soluble substituent, so that the specific solvent-soluble substituent is removed, thereby producing a film-like product and a π-electron conjugated compound of interest. The above "π-electron conjugated compound precursor" is represented by A-(B)m.

In A-(B)m, A represents a π-electron conjugated substituent, B represents a solvent-soluble substituent containing a structure represented by General Formula (I) as at least a partial structure, and m is a natural number. Here, the solvent-soluble substituent represented by B is linked via a covalent bond with the π-electron conjugated substituent represented by A where the covalent bond is formed at $Q_1$ to $Q_6$ with an atom present on the π-electron conjugated substituent represented by A or the solvent-soluble substituent represented by B is ring-fused with the π-electron conjugated substituent represented by A via atoms present on the π-electron conjugated substituent represented by A. By applying an external stimulus to the π-electron conjugated compound precursor, specific leaving substituents X and Y are eliminated in the form of X-Y (a molecule in which X is bonded to Y), and the solvent-soluble substituent B is converted to substituent C represented by General Formula (Ia) having a benzene ring structure, to thereby obtain a π-electron conjugated compound represented by A-(C)m. In addition, a film-like product containing this compound is obtained.

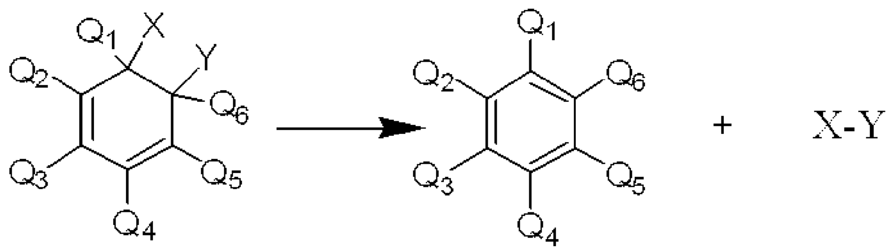

(I) (Ia) (II)

in General Formulas (I), (Ia) and (II), X and Y each represent a hydrogen atom, a substituted or unsubstituted ether group having 1 to 38 carbon atoms or a substituted or unsubstituted acyloxy group having 1 to 38 carbon atoms, where one of X and Y is the ether or acyloxy group and the other is the hydrogen atom; $Q_2$ to $Q_5$ each represent a hydrogen atom or a monovalent organic group; $Q_1$ and $Q_6$ each represent a hydrogen atom, a halogen atom, an alkyl group, an alkenyl group, an alkynyl group, an aryl group or a heteroaryl group; and among the monovalent organic groups represented by $Q_1$ to $Q_6$, adjacent monovalent organic groups may be linked together to form a ring, with the proviso that when adjacent $Q_2$ to $Q_5$ are monovalent organic groups linked together to form a ring structure,; and

wherein the monovalent organic group is any one of an alkyl group, an alkenyl group, an alkynyl group, an aryl group and a heteroaryl group, wherein the heteroaryl group is selected from a thiophene ring, a pyridine ring, a pyrazine ring, a pyrimidine ring, a triazine ring, a pyrrol ring, a pyrazole ring, an imidaziole ring, a triazole ring, an oxazole ring, a thiazole ring and a furan ring.

In General Formulas (I), (Ia) and (II), X, Y and $Q_1$ to $Q_6$ are otherwise the same as in the above-described leaving substituent-containing compound and the compound obtained therefrom through elimination reaction.

Next, the π-electron conjugated substituent A will be described. The π-electron conjugated substituent A is not particularly limited, so long as it has a π-electron conjugated plane. Preferred examples thereof include a benzene ring, a thiophene ring, a pyridine ring, a pyrazine ring, a pyrimidine ring, a triazine ring, a pyrrole ring, a pyrazole ring, an imidazole ring, a triazole ring, an oxazole ring, a thiazole ring, a furan ring, a selenophene ring and a silole ring. More preferably, Ar is at least one π-electron conjugated compound selected from the group consisting of (i) compounds in which one or more aromatic hydrocarbon rings are ring-fused with one or more aromatic heterocyclic rings, compounds in which two or more aromatic hydrocarbon rings are ring-fused together, and compounds in which two or more aromatic heterocyclic rings are ring-fused together; and (ii) compounds in which one or more aromatic hydrocarbon rings are linked via covalent bond with one or more aromatic heterocyclic rings, compounds in which two or more aromatic hydrocarbon rings are linked together via a covalent bond, and compounds in which two or more aromatic heterocyclic rings are linked together via a covalent bond. Further, π electrons contained in the aromatic hydrocarbon rings or aromatic

heterocyclic rings are preferably delocalized throughout the ring-fused or linked structure by the interaction as a result of ring-fused linkage or covalently bonding.

The number of the aromatic hydrocarbon rings or aromatic heterocyclic rings which are ring-fused or linked together via a covalent bond is preferably two or more. Specific examples thereof include naphthalene, anthracene, tetracene, chrycene and pyrene (the following General Formula Ar3), pentacene and thienothiophene (the following General Formula Ar1), thienodithiophene triphenylene, hexabenzocoronene and benzothiophene (the following General Formula Ar2), benzodithiophene and [1]benzothieno[3,2-b][1]benzothiophene (BTBT) (the following General Formula Ar4), dinaphto[2,3-b:2',3'-f] [3,2-b]thienothiophene (DNTT) and benzodithienothiophene (TTPTT) (the following General Formula Ar5), fused polycyclic compounds such as naphthodithienothiophene (TTNTT) (the following General Formulas Ar6 and Ar7), and oligomers of aromatic hydrocarbon rings and aromatic heterocyclic rings such as biphenyl, terphenyl, quaterphenyl, bithiophene, terthiophene and quaterthiophene; phthalocyanines; and porphyrins.

Here, the "covalent bond" may be, for example, a carbon-carbon single bond, a carbon-carbon double bond, a carbon-carbon triple bond, an oxyether bond, a thioether bond, an amide bond and an ester bond, with a carbon-carbon single bond, a carbon-carbon double bond and a carbon-carbon triple bond being preferred.

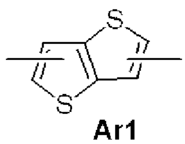
Ar1

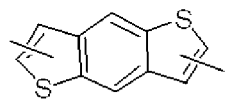
Ar2

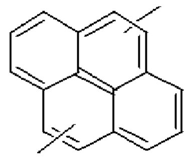
Ar3

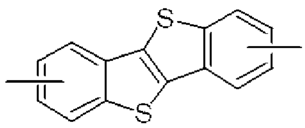
Ar4

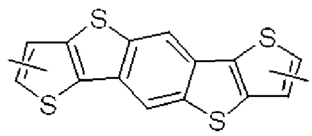
Ar5

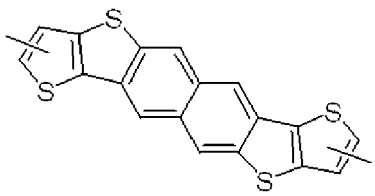
Ar6

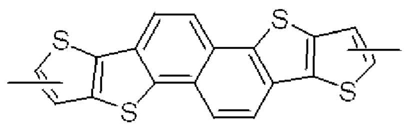
Ar7

Next will be described the binding form between the π-electron conjugated substituent A and the solvent-soluble substituent B. The solvent-soluble substituent B is linked via a covalent bond with the π-electron conjugated substituent A where the covalent bond is formed with an atom present on the π-electron conjugated substituent A, or the solvent-soluble substituent B is ring-fused with the π-electron conjugated substituent A via atoms present on the π-electron conjugated substituent A. The π-electron conjugated substituent A can be located at any positions other than the binding positions of the leaving substituents of the solvent-soluble substituent B. Here, the "covalent bond" may be, for example, a carbon-carbon single bond, a carbon-carbon double bond, a carbon-carbon triple bond, an oxyether bond, a thioether bond, an amide bond and an ester bond, with a carbon-carbon single bond, a carbon-carbon double bond and a carbon-carbon triple bond being preferred.

Also, needless to say, the number of the solvent-soluble substituent B, which is linked via a covalent bond or ring-fused with the π-electron conjugated substituent A depends on the number of atoms on the A that can provide sites for substitution or ring fusion. For example, an unsubstituted benzene ring can provide up to six sites for substitution via a covalent bond or for ring fusion. However, considering the size of the A itself, the number of substituents depending on dissolvability, symmetry of the molecule and easiness of synthesis, the number of soluble substituents in the present invention contained in one molecule is preferably 2 or more. Meanwhile, when the number of soluble substituents is too large, the soluble substituents sterically interact with each other, which is not preferred. Thus, considering symmetry of the molecule, the number of substituents depending on dissolvability and easiness of synthesis, the number of soluble substituents contained in one molecule is preferably 4 or less.

As described above, the π-electron conjugated compound precursor A-(B)m used in the production methods

of the present invention is formed of the π-electron conjugated substituent A and the solvent-soluble substituent B. Here, B represents a solvent-soluble substituent containing a structure represented by General Formula (I) as at least a partial structure, with the proviso that in General Formula (I), the solvent-soluble substituent B is linked via a covalent bond with the π-electron conjugated substituent A where the covalent bond is formed between an atom present on $Q_1$ to $Q_6$ and an atom present on the π-electron conjugated substituent A or the solvent-soluble substituent B is ring-fused with the π-electron conjugated substituent A via atoms present on the π-electron conjugated substituent A. C represents a substituent containing a structure represented by General Formula (Ia) as at least a partial structure. In the methods of the present invention for producing a film-like product containing a π-electron conjugated compound and the π-electron conjugated compound, a specific compound X-Y is eliminated from the solvent-soluble substituent B of the precursor through elimination reaction, whereby the solvent-soluble substituent B is converted to substituent C having a benzene ring. As a result, the π-electron conjugated compound precursor A-(B)m is converted to a π-electron conjugated compound A-(C)m.

As described above, the π-electron conjugated compound precursor has solvent-soluble, leaving substituents which impart solvent solubility to the precursor.

Specific examples of the precursor A-(B)m, which is formed by combining the π-electron conjugated substituent A with the solvent-soluble substituent B, include the above-listed compounds (Exemplary Compounds 1 to 42). However, the π-electron conjugated compound precursor should not be construed as being limited thereto. Also, it is easily supposed that there are several stereoisomers of the solvent-soluble substituent depending on the steric configuration of the leaving substituents, and that the above compounds may be mixtures of such stereoisomers having different steric configurations.

The storage stability of the π-electron conjugated compound precursor means that the leaving, soluble substituents are not eliminated unintentionally before conversion. The form of the π-electron conjugated compound precursor may be a solid. Alternatively, the π-electron conjugated compound precursor may be dissolved in a solvent to prepare an ink or waste. Furthermore, the π-electron conjugated compound precursor may be a film formed from the ink.

The degree of the storage stability can be determined by analyzing the state of the solid or ink which has been left to stand for a certain period (e.g., for one month) at a certain temperature (in general, about 20°C) under certain conditions (humidity: 50%, in darkness). Here, the amount of the molecules that have unintentionally been converted through elimination reaction is determined.

Since the elimination reaction is allowed to proceed through application of energy, the storage stability of the π-electron conjugated compound precursor can be increased when the π-electron conjugated compound precursor is stored at low temperatures (e.g., -100°C to 0°C), in darkness and/or in an inert atmosphere. Preferably, the unintentional elimination of the leaving group does not occur at -40°C in darkness (for example, after storage of the precursor with a LC purity of 99.5%, new impurities exceeding 0.5% are not detected). More preferably, the unintentional elimination does not occur at 0°C to 5°C. Particularly preferably, the unintentional elimination does not occur at 5°C to 40°C.

By applying energy such as heat to the precursor A-(B)m (applying external stimulus thereto) to eliminate substituents X and Y through the below-described elimination reaction, the π-electron conjugated compound A-(C)m and the film-like product containing the compound can be obtained.

Specific examples of the compound A-(C)m (hereinafter referred to as "specific compound") produced from the above-listed precursor A-(B)m include the above specific compounds 1 to 29. However, the π-electron conjugated compound should not be construed as being limited thereto.

[Method for producing π-electron conjugated compound through elimination reaction of π-electron conjugated compound precursor]

The method of the present invention for producing the film-like product containing the π-electron conjugated compound has a core step of producing the π-electron conjugated compound through elimination reaction. Thus, the elimination reaction will be described in detail.

In the production method of the present invention, the precursor represented by General Formula (I) is contained in a precursor-containing film formed by, for example, coating on a substrate (support) such as a plastic substrate, a metal substrate, a silicon wafer and a glass substrate. The precursor is converted by energy to the compound represented by General Formula (Ia) (specific compound) and the compound represented by General Formula (II) (eliminated component).

There are several isomers of the compound represented by General Formula (I) depending on the steric configuration of the substituents. However, these isomers are all converted into the specific compound represented by General Formula (Ia) to produce the same eliminated component.

Groups X and Y, which are eliminated from the compound represented by General Formula (I), are defined as leaving substituents, and X-Y formed therefrom is defined as an eliminated component. The eliminated components may be solid, liquid, or gas. In view of removal of the eliminated component to the outside of a system, the eliminated components are preferably liquid or gas, particularly preferably gas at normal temperature, or solid or liquid formed into

gas at a temperature for performing elimination reaction.

The boiling point of the eliminated component in an atmospheric pressure (1,013 hPa) is preferably 500°C or lower. From the viewpoint of easiness of removal of the eliminated component to the outside of the system, and the temperature of decomposition and sublimation of a $\pi$-electron conjugated compound to be generated, the boiling point is more preferably 400°C or lower, particularly preferably 300°C or lower.

As one example, next will be described conversion, through elimination reaction, of the compound represented by General Formula (I) where X is a substituted or unsubstituted acyloxy group and Y, $Q_1$ and $Q_6$ each are a hydrogen atom. Notably, conversion, through elimination reaction, of the $\pi$-electron conjugated compound precursor should not be construed as being limited to the following example.

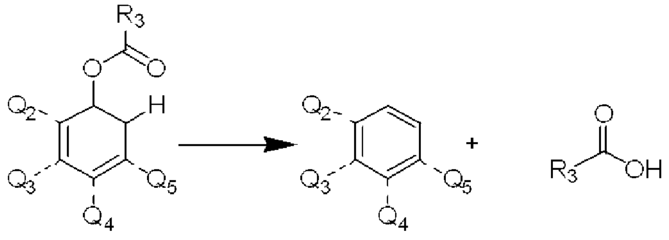

(VI) (VII) (VIII)

In the above formula, a cyclohexadiene structure represented by General Formula (VI) is converted by application of energy (heat) to a benzene ring-containing specific compound represented by General Formula (VII) as a result of removal of an alkyl chain-containing carboxylic acid represented by General Formula (VIII) as the eliminated component. When the heating temperature exceeds the boiling point of the carboxylic acid, the carboxylic acid is rapidly vaporized.

The mechanism by which the eliminated component is removed from the compound represented by General Formula (VI) is outlined through the following reaction formula (scheme). Notably, in the following reaction scheme, the mechanism by which the eliminated component is removed from the cyclohexadiene structure in the present invention corresponds to conversion from General Formula (VI-a) to General Formula (VII-a). For detail explanation, the mechanism by which the eliminated component is removed from the cyclohexene structure [General Formula (IX)] is also shown. In the following formula, $R_3$ represents a substituted or unsubstituted alkyl group.

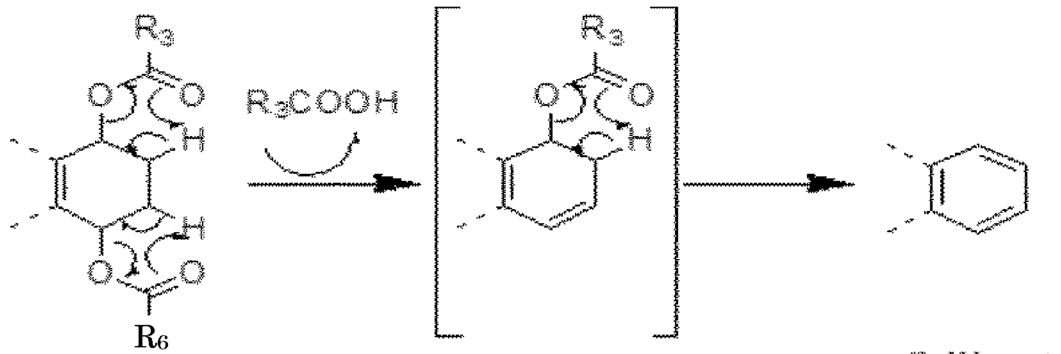


(IX) (VI -a) (VII-a)

As shown in the above reaction formula, the cyclohexadiene structure represented by General Formula (VI-a) is converted to the benzene structure represented by General Formula (VII-a) via a transition state of a six-membered ring structure. In this transition state, the hydrogen atom on the $\beta$-carbon and the oxygen atom of the carbonyl group are 1,5-transposed to cause concerted elimination reaction, so that a carboxylic acid compound is removed.

The elimination reaction of the compound [General Formula (IX)] having a cyclohexene structure with two acyloxy groups is thought to proceed through two steps. First, one carboxylic acid is removed to form the cyclohexadiene structure represented by General Formula (VI-a).

In this step, the activation energy necessary for removing one carboxylic acid from the disubstituted compound represented by General Formula (IX) is sufficiently larger than that necessary for removing one carboxylic acid from the monosubstituted compound represented by General Formula (VIa). Thus, the elimination reaction smoothly proceeds through two steps, to thereby form the compound represented by General Formula (VII-a). In other words, the monosubstituted compound represented by General Formula (VI-a) cannot be isolated from the reaction system in the above reaction formula.

Even when there are several stereoisomers depending on the positions of the substituents (e.g., acyloxy group and hydrogen), the above reaction can proceed although the reaction rate is different.

As is inferred from the above reaction formula, synthesis of an active monosubstituted compound is advantageous since energy necessary for elimination reaction of the active monosubstituted compound is lower than that necessary for elimination reaction of the cyclohexene structure represented by General Formula (IX). That is, the leaving substituent can be removed from the cyclohexadiene structure by energy (external stimulus) lower than in the conventional compounds.

The effect that the leaving substituent can be removed from the above cyclohexadiene structure at lower temperatures can be obtained not only when the substituent is an acyloxy group but also when the substituent is an ether group, etc. The ether group, etc. requires high energy for elimination in the conventional cyclohexene skeleton, and thus is not suitably employed. However, in the skeleton disclosed herein, the ether group requires low energy for elimination and can be employed similar to the acyloxy group.

In the above reaction formula, since removal and transition of the hydrogen atom on the β-carbon are the first step of the reaction, the stronger the force of the oxygen atom to attract the hydrogen atom, the easier the reaction occurs. The force of the oxygen atom to attract the hydrogen atom is changed, for example, according to the type of the alkyl chain at the side of the acyloxy group, or by replacing the oxygen atom with a chalcogen atom such as sulfur, selenium, tellurium, and polonium which belong to the same group 16 as the oxygen atom does.

Examples of the energies applied for performing elimination reaction include heat, light and electromagnetic wave. Heat or light is preferred in terms of reactivity, yield or post treatment. Particularly preferred is heat. Alternatively, in the presence of acid or base, the aforementioned energies may be applied.

Generally, the above elimination reaction depends on the structure of a functional group. However, most cases of elimination reaction need heating from the standpoints of reaction speed and reaction ratio. Examples of heating methods for performing elimination reaction include, but not limited thereto, a method for heating on a support, a method for heating in an oven, a method for irradiation with microwave, a method for heating by converting light to heat using a laser beam, and a method using a photothermal conversion layer.

Heating temperature for performing elimination reaction may be a room temperature (approximately 25°C) to 500°C. In consideration of thermal stability of the materials and a boiling point of the eliminated components as to the lower limit of the temperature, and in consideration of energy efficiency, percentage of the presence of unconverted molecule, and the sublimation and decomposition of the compound after conversion as to the upper limit of the temperature, the temperature is preferably 40°C to 500°C. Moreover, in consideration of thermal stability of the π-electron conjugated compound precursor during synthesis, the temperature is more preferably 60°C to 500°C, and particularly preferably 80°C to 400°C.

As to the heating time, the higher the temperature is, the shorter the reaction time becomes. The lower the temperature is, the longer the time required for elimination reaction becomes. Heating time depends on the reactivity and amount of the π-electron conjugated compound precursor, and is generally 0.5 min to 120 min, preferably 1 min to 60 min, and particularly preferably 1 min to 30 min.

In the case where light is used as the external stimulus, for example, infrared lamp or irradiation of light of wavelength absorbed by a compound (for example, exposure to light of wavelength 405 nm or less) may be used. On this occasion, a semiconductor laser may be used. Examples of semiconductor laser beam include a near-infrared region laser beam (generally, a laser beam of wavelength around 780 nm), a visible laser beam (generally, a laser beam of wavelength in the range of 630 nm to 680 nm), and a laser beam of wavelength of 390 nm to 440 nm. Particularly preferable laser beam is a laser beam having a wavelength region of 390 nm to 440 nm, and a semiconductor laser beam having a laser emission wavelength of 440 nm or less is preferably used. Among these semiconductor laser beam, examples of preferable light sources include a bluish-violet semiconductor laser beam having an emission wavelength region of 390 nm to 440 nm (more preferably from 390 nm to 415 nm), and a bluish-violet SHG laser beam having a center emission wavelength of 425 nm that has been converted to a half wavelength of the infrared semiconductor laser beam having a center emission wavelength of 850 nm by using an optical waveguide element.

In the elimination reaction of the leaving substituents, the acid or base serves as a catalyst, and conversion can be performed at lower temperature. A method of using the acid or base is not particularly limited. Examples of the method include a method in which the acid or base may be directly added to the π-electron conjugated compound precursor, a method in which the acid or base is dissolved in any solvent to form a solution, and the solution is added to the π-electron conjugated compound precursor, a method in which the π-electron conjugated compound precursor is heated in the vaporized acid or base, and a method in which a photoacid generator and a photobase generator are used, and followed by light irradiation, to thereby obtain an acid and base in the reaction system.

Examples of the acids include, but not limited thereto, hydrochloric acid, nitric acid, sulfuric acid, acetic acid, trifluoroacetic acid, trifluoromethanesulfonic acid, 3,3,3-trifluoropropionic acid, formic acid, phosphoric acid and 2-butyl

octanoic acid.

Examples of the photoacid generators include ionic photoacid generators such as sulfonium salt, and an iodonium salt; and nonionic photoacid generators such as imide sulfonate, oxime sulfonate, disulfonyl diazomethane, and nitrobenzyl sulfonate.

Examples of the bases include, but not limited thereto, hydroxides such as sodium hydrate, potassium hydrate, carbonates such as sodium hydrogen carbonate, sodium carbonate, potassium carbonate, amines such as triethylamine and pyridine, and amidines such as diazabicycloundecene, diazabicyclononene.

Examples of photobase generators include carbamates, acyloximes, and ammonium salts.

The elimination reaction is preferably performed in a volatile acid or base atmosphere from the standpoint of easiness of removal of the acid or base to the outside of the system after reaction.

The elimination reaction can be performed in an ambient atmosphere regardless of the absence or presence of the catalyst. Elimination reaction is preferably performed in an inert gas atmosphere or reduced pressure in order to reduce any influence of side reaction such as oxidation or influence of moisture, and to promote removal of an eliminated component to outside the system.

In addition to the method of obtaining carboxylate by reacting the alcohol described below with carboxylic acid chloride or carboxylic acid anhydride, or through exchange reaction between a halogen atom and silver carboxylate or carboxylic acid-quaternary ammonium salt, examples of methods for forming the acyloxy group serving as the leaving substituent include, but not limited thereto, a method in which phosgene is reacted with alcohol so as to obtain a carbonate ester, a method in which carbon disulfide is added in alcohol, and alkyl iodide is reacted therewith to obtain xanthate ester, a method in which tertiary amine is reacted with hydrogen peroxide or carboxylic acid so as to obtain amine oxide, and a method in which ortho selenocyano nitrobenzene is reacted with alcohol so as to obtain selenoxide.

Also, the ether group can be formed as follows. Specifically, an alcohol is treated with a base and the resultant compound is treated with, for example, an alkyl halide or an alkylsilane halide (Williamson ether synthesis). However, the method for forming the ether group should not be construed as being limited thereto.

(Method for producing $\pi$-electron conjugated compound precursor)

As described above, the $\pi$-electron conjugated compound precursor has a cyclohexadiene skeleton and a leaving substituent (the structure of the cyclohexadiene skeleton and the leaving substituent is entirely defined as solvent-soluble substituent B).

Since the structure of the cyclohexadiene skeleton and the leaving substituent, so-called solvent-soluble substituent B, is sterically bulky but not stiff, the crystallinity is poor. Thus, a molecule having such structure excels in solubility, and has properties of easily obtaining a film having low crystallinity (or an amorphous film), when a solution of the $\pi$-electron conjugated compound precursor is applied.

The method for forming the halogen group or acyloxy group in the cyclohexene skeleton of solvent-soluble substituent B is the same as described above in the section "Method for producing Leaving Substituent-Containing Compound."

Also, in the precursor A-(B)m used in the production method of the present invention, the method for linking the $\pi$-electron conjugated substituent A with the solvent-soluble substituent B via a covalent bond is the same as the above method for binding the solvent-soluble substituent with other skeletons via a covalent bond.

In order to form a thin film from the $\pi$-electron conjugated compound precursor obtained by the above-described production method, for example, a solution (e.g., an ink) containing the $\pi$-electron conjugated compound precursor in a solvent may be treated with a conventionally known film forming method such as spin coating, casting, dipping, inkjetting, doctor blade casting or screen printing. Alternatively, the $\pi$-electron conjugated compound itself obtained after conversion may be treated with a conventionally known film forming method such as vacuum vapor deposition or sputtering. Any of these film forming methods enables to form a good thin film having excellent strength, toughness, durability and the like without cracks.

Moreover, by applying energy (external stimulus) to the film of the $\pi$-electron conjugated compound precursor A-(B)m of the present invention formed by the above film forming method, the compound (eliminated component) represented by General Formula (II) is eliminated from the soluble leaving substituent B represented by General Formula (I) to form substituent C represented by General Formula (Ia). As a result, an organic semiconductor film formed of the $\pi$-electron conjugated compound A-(C)m (which is applied, for example, to an organic semiconductor material) can be obtained. Therefore, the $\pi$-electron conjugated compound precursor A-(B)m may be used as various materials for functional elements such as photoelectric conversion elements, thin-film transistor elements, light-emitting elements and the like.

(Electronic device)

The specific compound disclosed hereinmay be used in, for example, the electronic devices described above in the section "Application of Leaving Substituent-Containing Compound to Device."

[Film Forming Method: Organic Semiconductor Layer]

Additionally, the π-electron conjugated compound precursor disclosed herein is used as an organic semiconductor precursor. The organic semiconductor precursor is dissolved in a solvent (e.g., dichloromethane, tetrahydrofuran, chloroform, toluene, chlorobenzene, dichlorobenzene or xylene) to prepare a solution (ink composition). The resultant solution is applied on a substrate so as to form a thin film. Energy may be applied to the thin film formed of the organic semiconductor precursor to attain conversion to an organic semiconductor film.

[Ink composition and solvent]

The solvent used in the ink composition can be determined as follows.

The π-electron conjugated compound precursor is dissolved in a solvent such as dichloromethane, tetrahydrofuran, chloroform, toluene, chlorobenzene, dichlorobenzene or xylene). The resultant solution can be applied onto a substrate to form a thin film. That is, the solvent used for preparing a coating liquid containing the precursor is not particularly limited and may be appropriately selected depending on the intended purpose. The solvent preferably has a boiling point of 500°C or lower since such solvent can be easily removed. However, highly volatile solvents are not necessarily preferred, and the solvent used preferably has a boiling point of 50°C or higher. Although thorough examination has not yet been conducted, for obtaining a sufficient conductivity, it is thought to be important that the precursors variously change in position for intermolecular contact as well as that the leaving groups in the precursor are simply eliminated. In other words, after the leaving groups (substituents) have been eliminated from the precursor present in the coating film, it is possibly required in the solvent that the resultant compounds be at least partially change in direction or position from a random state for intermolecular contact, rearrangement, aggregation, crystallization, etc.

The solvent used may be, for example, those having affinity to a polar carboester group or ether group serving as an leaving group contained in the precursor A-(B)m. Specific examples thereof include polar (water-miscible) solvents such as alcohols (e.g., methanol, ethanol and isopropanol), glycols (e.g., ethylene glycol, diethylene glycol and propylene glycol), ethers (e.g., tetrahydrofuran (THF) and dioxane), ketones (e.g., methyl ethyl ketone and methyl isobutyl ketone), phenols (e.g., phenol and cresol), nitrogen-containing organic solvents (e.g., dimethyl formamide (DMF), pyridine, dimethylamine and triethylamine) and CELLOSOLVE (registered trademark) (e.g., methyl cellosolve and ethyl cellosolve). The solvent may also be those having relatively high affinity to the other structure than the leaving groups. Specific examples thereof include hydrocarbons (e.g., toluene, xylene and benzene), halogenated hydrocarbon solvents (e.g., carbon tetrachloride, methylene chloride, 1,2-dichloroethane, 1,1,2-trichloroethane, trichloroethylene, chloroform, monochlorobenzene and dichloroethylidene), esters (e.g., methyl acetate and ethyl acetate) and nitrogen-containing organic solvents (e.g., nitromethane and nitroethane). These solvents may be used alone or in combination.

In use, polar (water-miscible) solvents such as tetrahydrofuran (THF) are particularly preferably combined with water-immiscible solvents such as halogenated hydrocarbons (e.g., toluene, xylene, benzene, methylene chloride, 1,2-dichloroethane, chloroform and carbon tetrachloride) and esters (e.g., ethyl acetate).

Also, the coating liquid may further contain a volatile or self-decomposable acid or base for promoting decomposition of the carboester group in such an amount that the effects of the present invention cannot be impaired. Further, strongly-acid solvents such as trichloroacetic acid (which decomposes into chloroform and carbon dioxide with heating) and trifluoroacetic acid (volatile) are preferably used since the carboester group (weak Lewis acid) is readily removed in the presence of them.

As described above, the π-electron conjugated compound precursor disclosed herein has a cyclohexadiene structure and a substituted or unsubstituted ether or acyloxy group, which are sterically bulky, and poor crystallinity. A molecule having such a structure excels in solubility and has properties of easily obtaining a film having low crystallinity (amorphous), when a solution containing the molecule is applied.

Examples of methods for depositing the thin films include spray coating, spin coating, blade coating, dipping, casting, roll coating, bar coating, dye coating, inkjetting and dispensing; printing methods such as screen printing, offset printing, relief printing, flexographic printing; soft lithography such as a micro contact printing. Moreover, these methods may be used in combination.

From the above-described deposition methods and solvents, a deposition method and solvent may be appropriately selected according to materials.

The organic semiconductor material itself which has been thermally converted can deposit a film through vapor phase, such as vacuum deposition.

Among the above printing or coating methods, the liquid droplet-coating methods such as inkjetting can effectively utilize materials with no waste since the liquid droplets are dropped only on a predetermined position of the substrate. Thus, unlike the other methods, the liquid droplet-coating methods do not require a process for removing the material at unnecessary portions, simplifying the step.

The conditions for stably jetting the liquid droplets should be considered from the viewpoints of at least two points as follows: the drying speed of a solvent and the solute concentration of an ink (the solubility of the solute).

Regarding the drying speed, when using a solvent having excessively high vapor pressure; i.e., having a relatively low boiling point, the solvent is rapidly dried near nozzles of an inkjet device to form precipitates of the solute, problematically causing nozzle clogging. Thus, use of such a solvent is not suitable to production on the industrial scale. In general, the solvent used for inkjetting preferably has a high boiling point. In the present invention, the solvent preferably includes a solvent having a boiling point of 150°C or higher. More preferably, the solvent includes a solvent having a boiling point of 200°C or higher.

Regarding the solubility of the solute to the ink solvent, preferred are solvents capable of dissolving the organic semiconductor material used in an amount of 0.1% by mass or more. More preferred are solvents capable of dissolving it in an amount of 0.5% by mass or more. Examples of such solvents include cumene, cymene, mesitylene, 2,4-trimethylbenzene, propylbenzene, butylbenzene, amylbenzene, 1,3-dimethoxybenzene, nitrobenzene, benzonitrile, N,N-dimethylaniline, N,N-diethylaniline, tetralin, 1,5-dimethyltetralin, cyclohexanone, methyl benzoate, ethyl benzoate and propyl benzoate.

In the organic thin-film transistor, the thickness of the organic semiconductor layer is not particularly limited. The organic semiconductor layer is formed so as to have such a thickness that a uniform thin film can be formed, which is specifically a film having no gaps or holes that adversely affect carrier transporting property of the organic semiconductor layer. In general, the thickness of the organic semiconductor thin film is preferably 1 μm or smaller, particularly preferably 5 nm to 100 nm. In the organic thin-film transistor, the organic semiconductor layer formed from the above compound is formed such that it is in contact with a source electrode, a drain electrode and an insulating film.

## Examples

Hereinafter, the present invention will be further described with the following Examples, which should not be construed as limiting the scope of the present invention thereto.

First, intermediates of specific compounds and other compounds relating to compounds used in Examples and Comparative Examples were synthesized.

The identification of the compounds used in the following Synthesis Examples and Examples was performed using a NMR spectrometer [JNM-ECX (product name), 500 MHz, product of JEOL Ltd.], a mass spectrometer [GC-MS, GCMS-QP2010 Plus (product name), product of SHIMADZU CORPORATION], a precise mass spectrometer [LC-TofMS, Alliance-LCT Premire (product name), product of Waters Co.)], an elemental analyzer [(CHN) (CHN recoder MT-2, product of Yanagimoto Mfg. Co., Ltd.), and an elemental analyzer (sulfur) (ion chromatography; anion analysis system: DX320 (product name), product of Dionex Corporation)].

[Synthesis Example 1: Synthesis 1 of intermediate of specific compound]

<Synthesis of Compound (2)>

According to the following reaction formula (scheme), Compound (2) was synthesized.

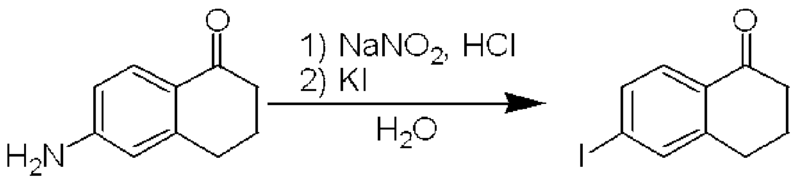


1 2

The amine compound having the above formula 1 was purchased from SIGMA Aldrich Co. and subjected to no treatments before use.

A 500 mL beaker was charged with the compound having the above formula 1 (20 g, 119.0 mmol) and 15% HCl (96 mL). While the resultant mixture was being maintained at 5°C or lower with ice cooling, aqueous sodium nitrite solution (9.9 g, 143.0 mmol + water (42 mL)) was added dropwise thereto. After completion of dropwise addition, the

mixture was stirred at the same temperature for 30 min. Then, aqueous potassium iodide solution (23.7 g, 143.0 mmol + water (77 mL)) was added to the mixture at one time. The beaker was taken out from the ice bath and the mixture was stirred for 2.5 hours. Thereafter, the mixture was heated at 60°C for 0.5 hours until generation of nitrogen was terminated. After cooled to room temperature, the reaction solution was extracted three times with diethyl ether. The organic layer was washed with 5% aqueous sodium thiosulfate solution (100 mL × 3) and further washed with saturated brine (100 mL × 2). Moreover, the organic layer was dried with sodium sulfate, followed by filtration. The filtrate was concentrated to obtain red oil.

The obtained red oil was purified through silica gel chromatography (solvent: ethyl acetate/hexane = 9/1) to obtain a pale orange solid. Further, the obtained solid was recrystallized from 2-propanol to obtain Compound (2) as pale orange crystals (yield amount: 11.4 g, yield rate: 35.2%).

The analysis results of Compound (2) are shown below.

$^1$H NMR (500 MHz, $CDCl_3$, TMS, δ): 2.13 (quint, 2H, J = 5.7 Hz), 2.64 (t, 2H, J = 6.3 Hz), 2.92 (t, 2H, J = 6.0 Hz), 7.66 (d, 1H, J = 8.0 Hz), 7.67 (s, 1H), 7.72 (d, 1H, J = 8.0 Hz)
Melting point: 74.0°C-75.0°C
Mass spectrometry: GC-MS m/z = 272(M+)

From the above analysis results, it was confirmed that a structure of the synthesized product did not contradict that of Compound (2).

<Synthesis of Compound (3)>

According to the following reaction formula (scheme), Compound (3) was synthesized.

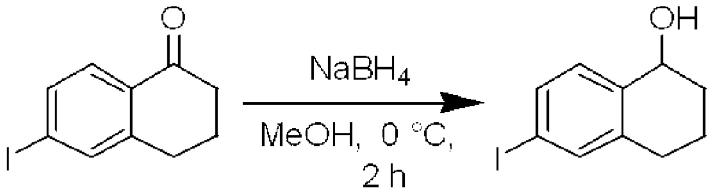


2 3

A 200 mL round-bottom flask was charged with Compound (2) (4.1 g, 15 mmol) and methanol (100 mL). Sodium borohydride (850 mg, 22.5 mmol) was gradually added to the resultant mixture at 0°C with ice cooling, followed by stirring for 3 hours at 0°C. Subsequently, excessive sodium borohydride was neutralized with dilute hydrochloric acid, and saturated brine was added to the mixture, which was then extracted with ethyl acetate (50 mL) 5 times. The extraction liquid was washed with ammonium chloride (100 mL) once and with brine (100 mL) twice. Thereafter, sodium sulfate was added thereto, followed by filtration. The filtrate was concentrated to obtain Compound (3) as a pale red solid (yield amount: 3.93 g, yield rate: 95.5%), which was directly used in the next step without any further purification.

The analysis results of Compound (3) are shown below.

$^1$H NMR (500 MHz, $CDCl_3$, TMS, δ): 1.71 (d, 1H, J = 5.8 Hz), 1.84-2.02 (m, 4H), 2.65-2.71 (m, 1H), 2.75-2.81 (m, 1H), 4.72 (d, 1H, J =4.6 Hz), 7.17 (d, 1H, J = 8.0 Hz), 7.47 (s, 1H), 7.52 (d,t 1H, $J_1$ = 8.0 Hz, $J_2$ = 1.2 Hz)
Mass spectrometry: GC-MS m/z = 274 (M+)
Melting point: 82.0°C-84.0°C

From the above analysis results, it was confirmed that a structure of the synthesized product did not contradict that of Compound (3).

<Synthesis of Compound (4)>

According to the following reaction formula (scheme), Compound (4) was synthesized.

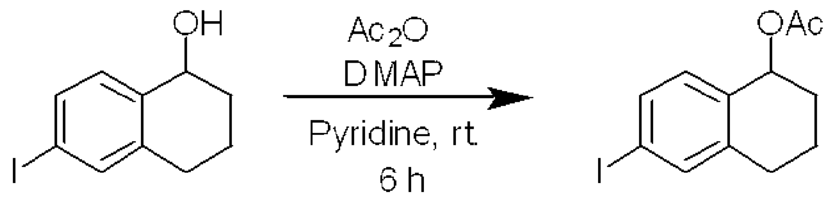


3 4

A 50 mL round-bottom flask was charged with Compound (3) (3.70 g, 13.5 mmol) and N,N-dimethylaminopyridine (hereinafter referred to as "DMAP," 10 mg). After the flask had been purged with argon gas, anhydrous pyridine (8.1 mL) and acetic anhydride (6.2 mL) were added thereto, followed by stirring at room temperature for 6 hours. Water (50 mL) was added to the reaction solution, which was then extracted with ethyl acetate (20 mL) five times. The combined organic layer was washed with dilute hydrochloric acid (100 mL) three times, then with sodium hydrogen carbonate solution (100 mL) twice and finally with saturated brine (100 mL) twice. The mixture was dried with magnesium sulfate, followed by filtration. The filtrate was concentrated to obtain Compound (4) as a brown liquid (yield amount: 4.28 g, yield rate: 100%), which was directly used in the next step without any further purification.
The analysis results of Compound (4) are shown below.

$^1$H NMR (500 MHz, $CDCl_3$, TMS, δ): 1.76-1.83 (m, 1H), 1.89-2.10 (m, 1H), 2.07 (s, 3H) , 2.67-2.73 (m, 1H), 2.79-2.84 (m, 1H), 5.93 (t, 1H, J =5.2 Hz), 7.01 (d, 1H, J = 8.6 Hz), 7.49 (d, 1H, J = 2.3 Hz), 7.52 (s, 1H)
Mass spectrometry: GC-MS m/z = 316(M+)

From the above analysis results, it was confirmed that a structure of the synthesized product did not contradict that of Compound (4).

<Synthesis of Compound (5)>

According to the following reaction formula (scheme), Compound (5) was synthesized.

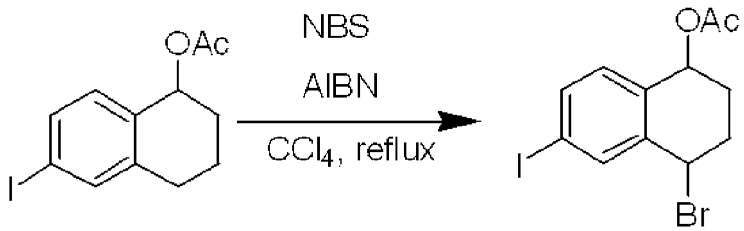


4 5

A 100 mL round-bottom flask was charged with Compound (4) (4.27 g, 13.5 mmol), azobisisobutylonitrile (hereinafter referred to as "AIBN," 25 mg), carbon tetrachloride (100 mL) and N-bromosuccinimide (hereinafter referred to as "NBS," 2.64 g, 14.8 mmol). After the flask had been purged with argon gas, the mixture was gently heated to 80°C, stirred for 1 hour at the same temperature and then cooled to room temperature.
The precipitates were removed through filtration. The filtrate was concentrated under reduce pressure to obtain a pale yellow solid, which was purified through silica gel chromatography (solvent: ethyl acetate/hexane = 8/2) to obtain Compound (5) as pale red oil (yield amount: 4.9 g, yield rate: 92.0%). Compound (5) was obtained as a 10 : 7 mixture of cis form and trans form.
The analysis results of Compound (5) are shown below.
Precise mass spectrometry: LC-MS m/z = 393.907 (100.0%), 395.904 (97.3%)
From the above analysis results, it was confirmed that a structure of the synthesized product did not contradict that of Compound (5).

<Synthesis of Compound (6)>

According to the following reaction formula (scheme), Compound (6) was synthesized.

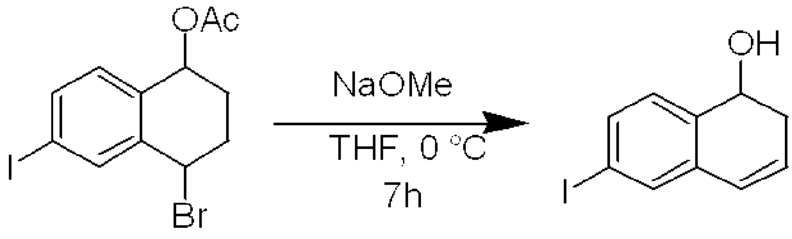


5 6

A 500 mL round-bottom flask was charged with Compound (5) (4.2 g, 10.6 mmol) and then purged with argon gas, followed by addition of THF (300 mL). Subsequently, sodium methoxide-methanol solution (25% by mass, 24 mL) was added to the resultant mixture at 0°C with ice cooling, followed by stirring at the same temperature for 6 hours. Water (300 mL) was added to the mixture, which was extracted with ethyl acetate (100 mL) four times. The extraction liquid was washed with saturated brine (100 mL) twice and dried with sodium sulfate, followed by filtration. The filtrate was concentrated to obtain a brown liquid. The obtained brown liquid was purified using a column to obtain Compound (6) as colorless crystals (yield amount: 1.2 g, yield rate: 41.0%).

The analysis results of Compound (6) are shown below.

$^1H$ NMR (500 MHz, $CDCl_3$, TMS, δ): 1.70 (d, 1H, J =3.4 Hz), 2.58-2.61 (m, 2H), 4.76 (q , 1H, J =6.3 Hz), 6.04 (q, 1H, J =5.2 Hz), 6.47 (d, 1H, J =9.8 Hz), 7.13 (d, 1H, J =8.1 Hz), 7.47 (d, 1H, J =1.7 Hz), 7.57 ($J_1$=8.1 Hz $J_2$ =1.7 Hz)
Mass spectrometry: GC-MS m/z = 272(M+)

From the above analysis results, it was confirmed that a structure of the synthesized product did not contradict that of Compound (6).

<Synthesis of Compound (7-1)>

According to the following reaction formula (scheme), Compound (7-1) was synthesized.

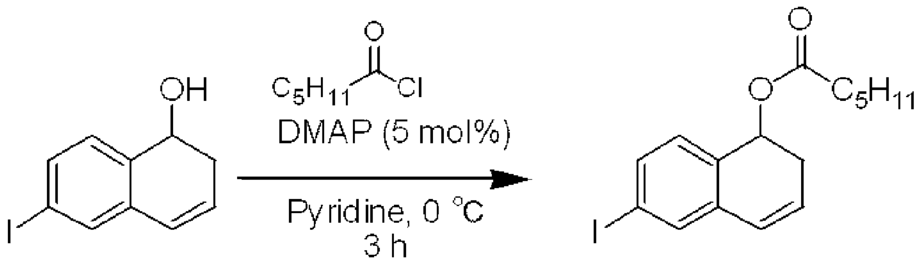


6 7-1

A 50 mL round-bottom flask was charged with Compound (6) (680 mg, 2.5 mmol) and DMAP (15.3 mg, 0.125 mmol), and then was purged with argon gas, followed by addition of pyridine (15 mL). Subsequently, hexanoylchloride (370 mg, 2.75 mmol) was added dropwise to the resultant mixture at 0°C with ice cooling, and stirred at the same temperature for 3 hours.

Water was added to the reaction solution, which was extracted with ethyl acetate (50 mL) three times. The organic layer was washed sequentially with saturated sodium hydrogen carbonate solution and saturated brine and dried with magnesium sulfate, followed by filtration. The filtrate was concentrated to obtain a brown liquid. The obtained liquid was dissolved in ethyl acetate/hexane (95/5), and the resultant solution was caused to pass through a silica gel pad having a thickness of 3 cm. The filtrate was concentrated to obtain Compound (7-1) as a colorless liquid (yield amount: 560 g, yield rate: 60.5%).

The analysis results of Compound (7-1) are shown below.

$^1H$ NMR (500 MHz, $CDCl_3$, TMS, δ): 0.86 (t, 3H, J =7.2 Hz), 1.21-1.30 (m, 4H) , 1.54-1.60 (m, 2H), 2.23 (td, 2H, $J_1$=7.5 Hz J $_2$= 2.3 Hz ), 2.58-2.62 (m, 2H), 5.95 (t, 1H, J =5.2 Hz), 6.03 (quint, 1H, J=4.6 Hz), 6.48 (d, 1H, J=9.8 Hz), 7.10 (d, 1H, J=8.0 Hz), 7.48 (d, 1H, J=1.7 Hz), 7.54 (dd, 1H, J1=8.0 Hz, J2=1.8 Hz)
Mass spectrometry: GC-MS m/z = 370(M+), 254 (thermally decomposed product)

From the above analysis results, it was confirmed that a structure of the synthesized product did not contradict that of Compound (7-1).

<Synthesis of Compound (7-2)>

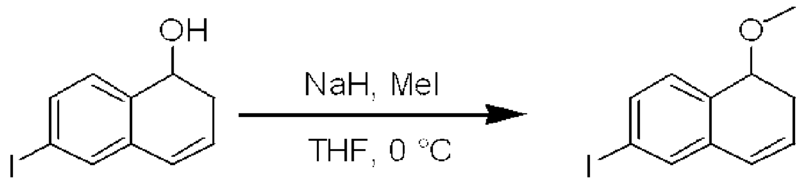


6 7-2

A 50 mL round-bottom flask was charged with Compound (6) (680 mg, 2.5 mmol) and THF (15 mL), and then purged with argon gas. Subsequently, sodium hydride (3.0 mmol, 72.0 mg) was added to the resultant mixture at 0°C with ice cooling, followed by stirring at the same temperature for 30 min. Thereafter, methyl iodide (3.0 mmol, 426 mg) was added dropwise to the mixture, followed by stirring at the same temperature for 1 hours. Water was added to the reaction solution, and the aqueous layer was extracted with ethyl acetate (50 mL) three times. The combined organic layer was washed with saturated brine and dried with magnesium sulfate, followed by filtration. The filtrate was concentrated to obtain a brown liquid. The obtained liquid was dissolved in ethyl acetate/hexane (90/10), and the resultant solution was caused to pass through silica gel pad having a thickness of 3 cm. The filtrate was concentrated to obtain Compound (7-2) as a colorless oily solid (yield amount: 607 mg, yield rate: 85.0%).

The analysis results of Compound (7-2) are shown below.

$^1$H NMR (500 MHz, $CDCl_3$, TMS, $\delta$): 2.57-2.61 (m, 2H),3.38 (s, 3H), 5.90 (t, 1H, J =5.2 Hz), 6.03 (quint, 1H, J=4.6 Hz), 6.48 (d, 1H, J=9.8 Hz), 7.10 (d, 1H, J=8.0 Hz), 7.48 (d, 1H, J=1.7 Hz), 7.54 (dd, 1H, J=1.8 Hz)
Mass spectrometry: GC-MS m/z = 286(M+), 254 (thermally decomposed product)

From the above analysis results, it was confirmed that a structure of the synthesized product did not contradict that of Compound (7-2).

<Synthesis of Compound (7-3)>

According to the following reaction formula (scheme), Compound (7-3) was synthesized.

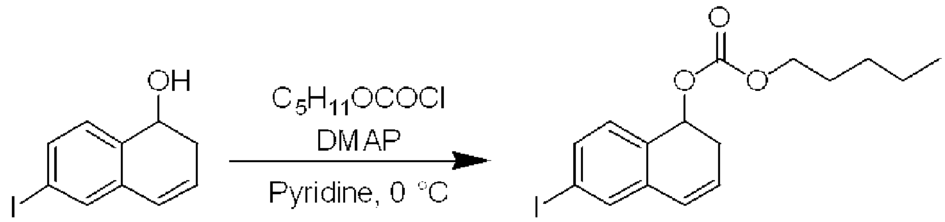


6 7-3

A 50 mL round-bottom flask was charged with Compound (6) (680 mg, 2.5 mmol) and DMAP (15.3 mg, 0.125 mmol) and purged with argon gas, followed by addition of pyridine (15 mL). Subsequently, amyl chloroformate (414 mg, 2.75 mmol) was added dropwise to the resultant mixture at 0°C with ice cooling, followed by stirring at the same temperature for 5 hours. Water was added to the reaction solution, which was then extracted with ethyl acetate (50 mL) three times. The organic layer was washed sequentially with saturated sodium hydrogen carbonate solution and saturated brine, and dried with magnesium sulfate. The filtrate was concentrated to obtain a brown liquid. The obtained liquid was dissolved in ethyl acetate/hexane (95/5), and the resultant solution was caused to pass through a silica gel pad having a thickness of 3 cm. The filtrate was concentrated to obtain Compound (7-3) as a colorless liquid (yield amount: 535 mg, yield rate: 55.5%).

The analysis results of Compound (7-3) are shown below.

> $^1H$ NMR (500 MHz, $CDCl_3$, TMS, δ): 0.86 (t, 3H, J =7.2 Hz), 1.21-1.30 (m, 4H) , 1.60-1.65 (m, 2H), 2.58-2.62 (m, 2H), 4.15-4.17 (m, 2H),5.94 (t, 1H, J =5.2 Hz), 6.02 (quint, 1H, J=4.6 Hz), 6.50 (d, 1H, J=9.8 Hz), 7.08 (d, 1H, J=8.0 Hz), 7.47 (d, 1H, J=1.7 Hz), 7.53 (dd, 1H, J=1.8 Hz)
> Mass spectrometry: GC-MS m/z = 386(M+), 254 (thermally decomposed product)

From the above analysis results, it was confirmed that a structure of the synthesized product did not contradict that of Compound (7-3).

<Synthesis of Compound (7-4)>

According to the following reaction formula (scheme), Compound (7-4) was synthesized.

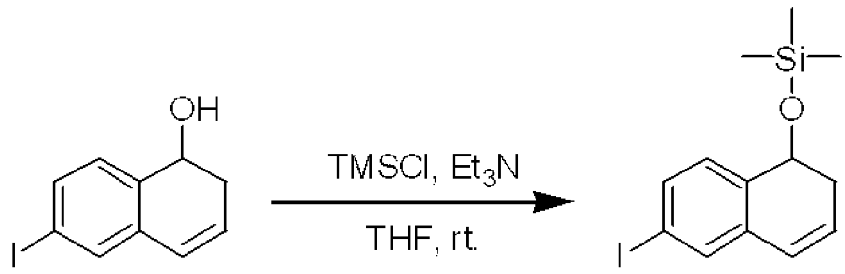


**6** **7-4**

A 50 mL round-bottom flask was charged with Compound (6) (680 mg, 2.5 mmol) and THF (20 mL) and purged with argon gas, followed by addition of triethylamine (3 mL).
Subsequently, trimethylsilyl chloride (300 mg, 2.75 mmol) was added dropwise to the resultant mixture at 0°C with ice cooling, followed by stirring at the same temperature for 1 hour. The flask was taken out from the ice bath. The mixture was returned to room temperature and then further stirred for 7 hours.
Water was added to the reaction solution, which was then extracted with ethyl acetate (50 mL) three times. The organic layer was washed with saturated brine and dried with magnesium sulfate. The filtrate was concentrated to obtain a brown liquid. The obtained liquid was dissolved in ethyl acetate/hexane (95/5), and the resultant solution was caused to pass through a silica gel pad having a thickness of 3 cm. The filtrate was concentrated to obtain Compound (7-3) as a colorless oily solid (yield amount: 688 mg, yield rate: 80.0%).
The analysis results of Compound (7-4) are shown below.

> $^1H$ NMR (500 MHz, $CDCl_3$, TMS, δ): 0.04 (s, 9H), 2.50-2.55 (m, 2H), 5.15 (t, 1H, J =5.2 Hz), 6.02 (quint, 1H, J=4.6 Hz), 6.53 (d, 1H, J=9.8 Hz), 7.07 (d, 1H, J=8.0 Hz), 7.44 (d, 1H, J=1.7 Hz), 7.54 (dd, 1H, J=1.8 Hz)
> Mass spectrometry: GC-MS m/z = 344(M+), 254 (thermally decomposed product)

From the above analysis results, it was confirmed that a structure of the synthesized product did not contradict that of Compound (7-4).

<Synthesis of Compound (7-5)>

According to the following reaction formula (scheme), Compound (7-5) was synthesized.

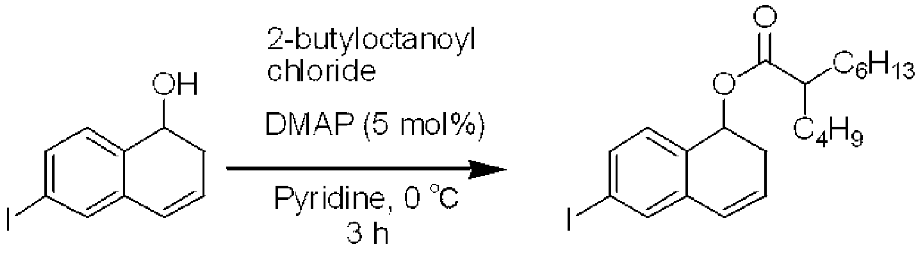


6 7-5

The synthesis procedure of Compound (7-1) was repeated, except that hexanoylchloride was changed to 2-butyloctanoyl chloride (2.75 mmol), to thereby obtain Compound (7-5) as a pale yellow liquid (yield amount: 1.33 g, yield rate: 97.8%).

The analysis results of Compound (7-5) are shown below.

$^1$H NMR (500 MHz, $CDCl_3$, TMS, δ): 0.74-0.83 (m, 6H), 1.10-1.32 (m, 12H) , 1.36-1.43 (m, 2H), 1.50-1.60(m, 2H), 2.27-2.32 (m, 1H), 2.58-2.62 (m, 2H), 5.95 (t, 1H, J =5.2 Hz), 6.03 (quint, 1H, J=4.6 Hz), 6.48 (d, 1H, J=9.8 Hz), 7.10 (d, 1H, J=8.0 Hz), 7.48 (d, 1H, J=1.8 Hz), 7.54 (dd, 1H, J1=8.0 Hz, J2=1.8 Hz)
Mass spectrometry: GC-MS m/z = 454(M+), 254 (thermally decomposed product)

From the above analysis results, it was confirmed that a structure of the synthesized product did not contradict that of Compound (7-5).

[Synthesis Example 2: Synthesis 2 of compound intermediate]

<Synthesis of Compound (8)>

According to the following reaction formula (scheme), Compound (8) was synthesized.

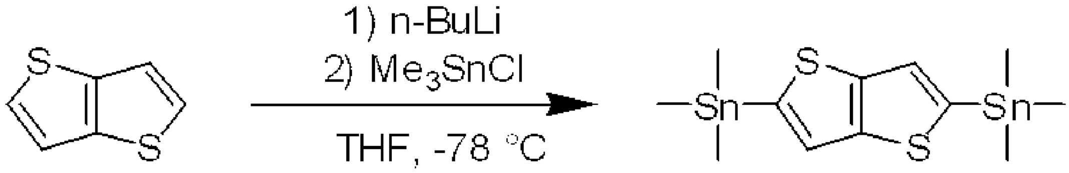


8

A 200 mL round-bottom flask was thoroughly dried and charged with thieno[3,2-b]thiophene (2.81 g, 20.0 mmol). The flask was purged with argon, followed by addition of anhydrous tetrahydrofuran (hereinafter abbreviated as "THF") (50 mL). The mixture was cooled to -78°C in an acetone-dry ice bath. Then, n-butyllithium (2.2 eq., 28.1 mL (1.6M hexane solution), 44 mmol) was added dropwise to the mixture for 15 min. The reaction system was increased to room temperature, followed by stirring at the same temperature for 16 hours. Then, the mixture was cooled again to -78°C, and trimethyltin chloride (2.5 eq., 50 mL (1.0M hexane solution), 50 mmol) was added thereto at one time. The reaction system was increased to room temperature, followed by stirring for 24 hours. Water (80 mL) was added to quench the mixture, and ethyl acetate was added thereto to separate an organic layer. The organic layer was washed sequentially with saturated aqueous potassium fluoride solution and saturated brine and dried with sodium sulfate, followed by filtration. The filtrate was concentrated to obtain a brown solid, which was then recrystallized from acetonitrile (repeatedly three times) to obtain Compound (8) as colorless crystals (yield amount: 5.0 g, yield rate: 54.1%).

The analysis results of Compound (8) are shown below.

$^1$H NMR (500 MHz, $CDCl_3$, TMS, δ): 0.38 (s, 18H), 7.23 (s, 2H)
Mass spectrometry: GC-MS m/z = 466(M+)

From the above analysis results, it was confirmed that a structure of the synthesized product did not contradict that of Compound (8).

<Synthesis of Compound (9)>

According to the following reaction formula (scheme), Compound (9) was synthesized.

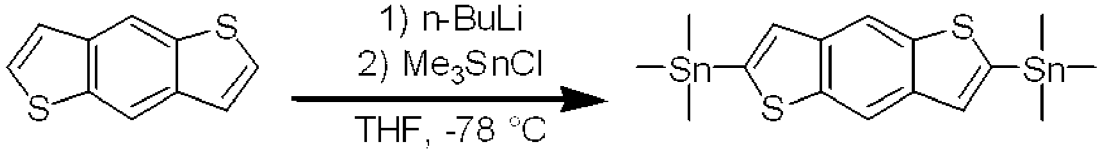


9

A 200 mL round-bottom flask was thoroughly dried and charged with benzo[1,2-b:4,5-b']dithiophene (3.81 g, 20.0 mmol). The flask was purged with argon, followed by addition of anhydrous THF (50 mL). The mixture was cooled to -78°C in an acetone-dry ice bath. Then, n-butyllithium (2.2 eq., 28.1 mL (1.6M hexane solution), 44 mmol) was added dropwise to the mixture for 15 min. The reaction system was increased to room temperature, followed by stirring for 16 hours. Then, the mixture was cooled again to -78°C, and trimethyltin chloride (2.5 eq., 50 mL (1.0M hexane solution), 50 mmol) was added thereto at one time. The reaction system was increased to room temperature, followed by stirring for 24 hours.

Water (80 mL) was added to quench the mixture, and ethyl acetate was added thereto to separate an organic layer. The organic layer was washed sequentially with saturated aqueous potassium fluoride solution and saturated brine and dried with sodium sulfate, followed by filtration. The filtrate was concentrated to obtain a brown solid, which was then recrystallized from acetonitrile (repeatedly three times) to obtain Compound (9) as pale yellow crystals (yield amount: 7.48 g, yield rate: 72.5%).

The analysis results of Compound (9) are shown below.

$^1$H NMR (500MHz, $CDCl_3$, TMS, $\delta$): 0.44 (s, 18H), 7.41 (s, 2H), 8.27 (s, 2H)
Mass spectrometry: GC-MS m/z = 518(M+)

From the above analysis results, it was confirmed that a structure of the synthesized product did not contradict that of Compound (9).

<Synthesis of Compound (10)>

According to the following reaction formula (scheme), Compound (10) was synthesized.

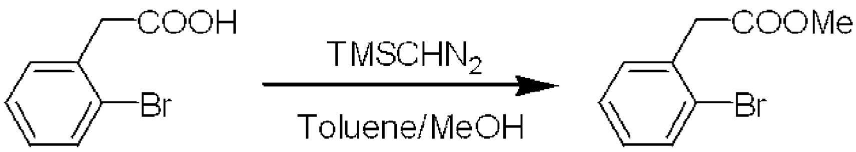


10

A 100 mL round-bottom flask was charged with 2-bromophenyl acetate (5.12 g, 23.8 mmol) (serving as a starting material), toluene (20 mL) and methanol (10 mL). Then, 2M hexane solution (12.5 mL) of trimethylsilyldiazomethane was gradually added dropwise to the resultant mixture, followed by stirring for 15 min. Excessive trimethylsilyldiazomethane was quenched with acetic acid, and the reaction solution was concentrated under reduced pressure with an evaporator. Toluene was added to the residue, and the resultant solution was caused to pass through a silica gel pad having a thickness of 3 cm, followed by concentrating again, to thereby obtain Compound (10) as a pale yellow liquid (yield amount: 5.1 g, yield rate: 94%).

The analysis results of Compound 10 are shown below.
Precise mass spectrometry: LC-TofMS m/z = 227.966 (found), 227.979 (calculated)

From the above analysis results, it was confirmed that a structure of the synthesized product did not contradict that of Compound 10.

<Synthesis of Compound (11)>

According to the following reaction formula (scheme), Compound (11) was synthesized.

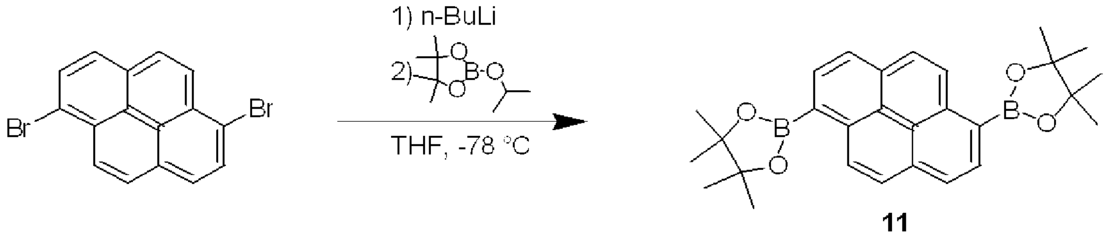


11

A 200 mL round-bottom flask was thoroughly dried and charged with 1,6-dibromopyrene (2.5 g, 6.9 mmol). The flask was purged with argon, followed by addition of anhydrous THF (120 mL). The mixture was cooled to -78°C in an acetone-dry ice bath. Then, n-butyllithium (2.15 eq., 9.1 mL (1.6M hexane solution), 14.9 mmol) was added dropwise to the mixture for 5 min, followed by stirring for 2 hours. At -78°C, 2-isopropoxy-4,4,5,5-tetramethyl-1,3,2-dioxaborolane (2.2 eq., 15.3 mmol, 3.1 mL) was added to the mixture at one time. The resultant mixture was stirred at the same temperature for 1 hour. The reaction system was increased to room temperature, followed by stirring for 2 hours.

Aqueous ammonium chloride solution (50 mL) and water (100 mL) were added to quench the mixture. Further, toluene was added thereto to separate an organic layer. The aqueous layer was extracted with toluene twice. The combined organic layer was washed with saturated brine and dried with sodium sulfate. The filtrate was concentrated to obtain a yellow solid. The obtained solid was dissolved in a minimum required amount of toluene, and the resultant solution was caused to pass through a silica gel column (3 cm). The filtrate was concentrated to obtain a pale yellow solid, which was then recrystallized from toluene/acetonitrile to obtain Compound (11) as colorless crystals (yield amount: 2.2 g, yield rate: 70%).

The analysis results of Compound 11 are shown below.

Precise mass spectrometry: LC-TofMS m/z = 454.244 (found), 454.249 (calculated)

From the above analysis results, it was confirmed that a structure of the synthesized product did not contradict that of Compound 11.

<Synthesis of Compound (12)>

According to the following reaction formula (scheme), Compound (12) was synthesized.

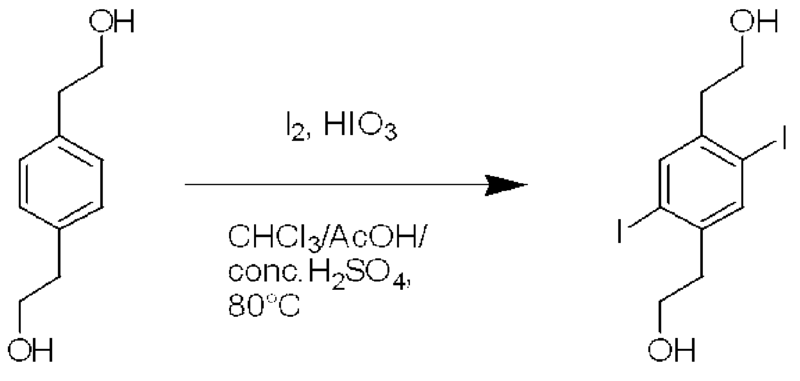


12

A 300 mL three-neck flask was charged with 1,4-benzenediethanol (15.7 g, 94.6 mmol), iodine (19.2 g, 75.7 mmol), iodic acid (8.3 g, 47.3 mmol), chloroform (50 mL), acetic acid (50 mL) concentrated sulfuric acid (10 mL). The flask was purged with argon gas, and the mixture was stirred at 80°C for 3 hours. Thereafter, iodine and iodic acid (each 1/4 mol of the amount of 1,4-benzenediethanol) were further added to the mixture, followed by stirring for 1 hour. The mixture was cooled to room temperature, and the precipitated target product was obtained through filtration using a PTFE filter. Then, aqueous sodium hydrogen sulfite solution was added to the residue, and the resultant mixture was extracted with chloroform. After washing with brine, the solvent was removed for concentration to obtain a brownish-red

solid. The obtained solid was combined with the filtered product to obtain Compound (12) as a brownish-red solid (yield amount: 28.0 g, yield rate: 71%).

The analysis results of Compound (12) are shown below.

Mass spectrometry: GC-MS m/z = 458(M+)

From the above analysis results, it was confirmed that a structure of the synthesized product did not contradict that of Compound (12).

<Synthesis of Compound (13)>

According to the following reaction formula (scheme), Compound (13) was synthesized.

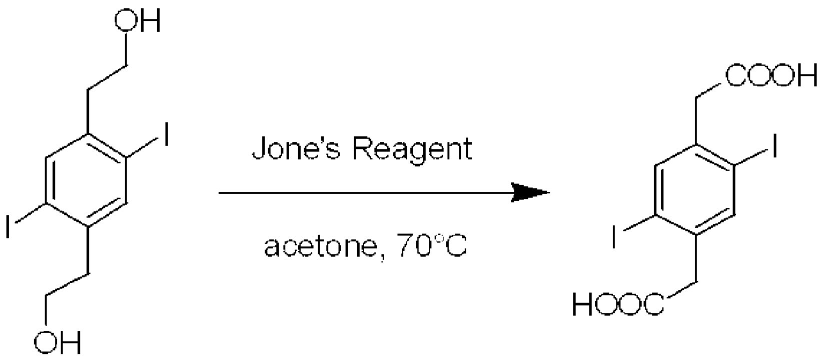


**12** **13**

A 500 mL egg-plant flask was charged with Compound 2 (4.18 g, 10 mmol) and acetone (30 mL), followed by stirring at room temperature. Jone's reagent (1.94 M) was added to the resultant mixture in an amount of 6.18 mL, and the mixture was heated under reflux conditions. While confirming that the color of the mixture became green, additional Jone's reagent was added to the mixture in a total amount of 30.9 mL. After confirming that the reaction sufficiently proceeded through TLC, the mixture was returned to room temperature and 2-propanol (20 mL) was added thereto, followed by stirring for 30 min. The precipitated matter was filtrated and thoroughly washed with water to obtain white solid 3 (yield amount: 3.16 g, yield rate: 71%).

Through FTIR, the CO stretch of carboxylic acid was observed at near 1,750 $cm^{-1}$.

The analysis results of Compound (13) are shown below.

Mass spectrometry: GC-MS m/z = 446(M+)

From the above analysis results, it was confirmed that a structure of the synthesized product did not contradict that of Compound (13).

<Synthesis of Compound (14)>

According to the following reaction formula (scheme), Compound (14) was synthesized.

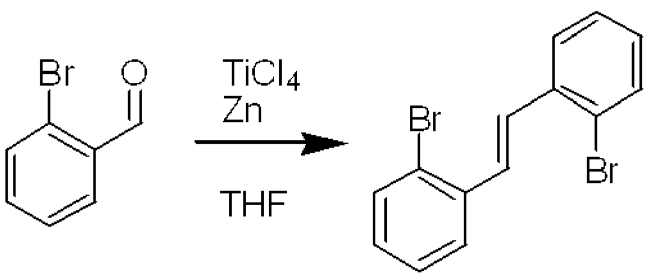


**14**

A 500 mL four-neck flask was charged with THF (300 mL) and zinc powder (17.23 mL, 0.263 mol) and cooled to 0°C in an ice bath. Titanium tetrachloride (50.0 g, 0.263 mol) was added dropwise to the mixture, followed by refluxing

for 1.5 hours. After the mixture had been cooled to room temperature, 2-bromobenzaldehyde (10.16 mL, 0.0879 mol) was added thereto, followed by refluxing for 5 hours.

After cooled to room temperature, the reaction solution was added to aqueous saturated sodium hydrogen carbonate solution (500 mL), followed by stirring. Further, ethyl acetate (500 mL) was added thereto and the resultant mixture was stirred overnight. Insoluble matter was removed through filtration using Celite, and the obtained solution was extracted with ethyl acetate. The organic layer was washed with saturated brine, and the obtained organic layer was dried with magnesium sulfate. The magnesium sulfate was removed through filtration, followed by recrystallization with ethyl acetate, to thereby obtain Compound (14) (yield amount: 7.0 g, yield rate: 54%).

The analysis results of Compound (14) are shown below.

$^1$H NMR (500MHz,$CDCl_3$,TMS,δ): 7.15(t,2H,J=5.5Hz), 7.34(t,2H,J=5.5Hz), 7.40(s,2H,), 7.60(dd,2H,J1=7.9Hz,J2=1.5Hz), 7.73(dd,2H,J1=7.9Hz,J2=1.5Hz)

From the above analysis results, it was confirmed that a structure of the synthesized product did not contradict that of Compound (14).

<Synthesis of Compound (15)>

According to the following reaction formula (scheme), Compound (15) was synthesized.

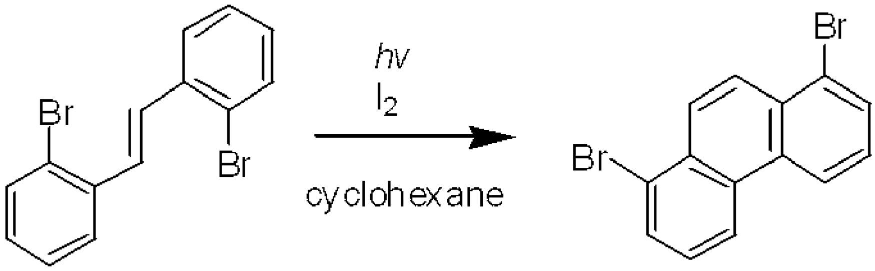


14 15

A 500 mL four-neck flask was charged with cyclohexane (400 mL), Compound (14) and iodine (0.22 g, 0.0017 mol). The flask was irradiated with light for 24 hours using a low-pressure mercury lamp (product of USHIO Co.). The precipitated solid was removed through filtration, followed by washing with cyclohexane and recrystallization from toluene, to thereby obtain Compound (15) (yield amount: 1.7 g, yield rate: 57%).

The analysis results of Compound (15) are shown below.

$^1$H-NMR($CDCl_3$,TMS)σ: 7.53(dd,2H,$J_1$=8.1Hz,$J_2$=7.7Hz), 7.94(d,2H,J=8.1Hz), 8.31(s,2H,), 8.67(d,2H,J=7.7Hz)

From the above analysis results, it was confirmed that a structure of the synthesized product did not contradict that of Compound (15).

<Synthesis of Compound (16)>

According to the following reaction formula (scheme), Compound (16) was synthesized.

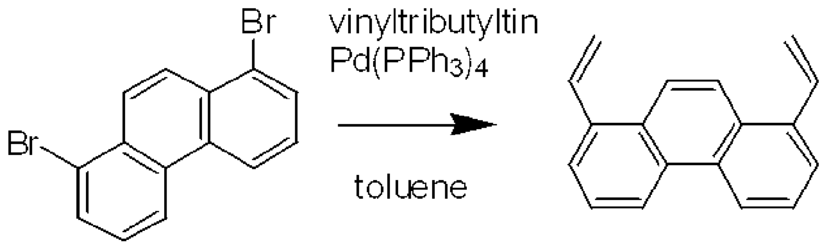


15 16

A 200 mL four-neck flask was charged with toluene (60 mL), vinyltributyltin (1.47 mL, 0.0050 mol) and Compound (15) (0.77 g, 0.0023 mol). The resultant mixture was stirred for 45 min with argon gas bubbling. Tetrakis(triphenylphosphine) palladium (0.23 g, 0.00020 mol) was added to the mixture, followed by refluxing for 4 hours. The mixture was cooled to room temperature, and poured into aqueous saturated potassium fluoride solution (100 mL), followed by

stirring. Further, ethyl acetate (100 mL) was added thereto, followed by stirring. Insoluble matter was removed through filtration using Celite, and the obtained solution was extracted with ethyl acetate.

The organic layer was washed with saturated brine, and the obtained organic layer was dried with magnesium sulfate. The magnesium sulfate was removed through filtration, followed by recrystallization from ethyl acetate, to thereby obtain Compound (16) (yield amount: 0.43 g, yield rate: 82%).

The analysis results of Compound (16) are shown below.

$^1$H-NMR($CDCl_3$,TMS)σ: 5.52(dd,2H,Ji=17.4Hz,J2=1.4Hz), 5.81(dd,2H,$J_1$=10.9Hz,$J_2$=1.4Hz),

7.55(dd,2H,$J_1$=17.4Hz,$J_2$=10.9Hz),
7.64(dd,2H,$J_1$=8.0Hz,$J_2$=7.2Hz), 7.74(d,2H,J=7.2Hz), 8.10(s,2H,), 8.68(d,2H,J=8.0Hz)

From the above analysis results, it was confirmed that a structure of the synthesized product did not contradict that of Compound (16).

<Synthesis of Compound (18)>

According to the following reaction formula (scheme), Compound (18) was synthesized.

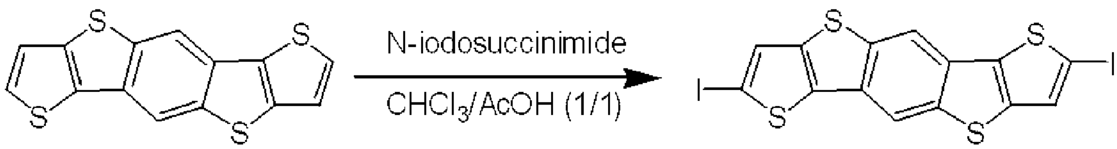


17 18

A 2 L round-bottom flask was charged with Compound (17) dithieno[2,3-d;2',3'-d']benzo[1,2-b;4,5-b']dithiophene (12.7 g, 42 mmol), which had been synthesized according to the method described in Advanced Materials, 2009, 21, 213-216. After the flask had been purged with argon gas, anhydrous chloroform (600 mL) and acetic acid (600 mL) were added thereto. The internal temperature of the flask was maintained at 0°C to 3°C using an ice bath. Next, N-iodosuccinimide (20.8 g, 92.4 mmol) was gradually added to the mixture in the dark. After stirred for 1 hour, the flask was taken out from the ice bath and returned to room temperature, followed by stirring overnight.

The precipitate was obtained through filtration and washed sequentially with aqueous saturated sodium hydrogen sulfite solution, ethanol, toluene and ethanol. The precipitate was dried under vacuum to obtain a pale yellow solid. The obtained solid contained not only disubstituted product (Compound (18)) but also a trace amount of monosubstituted product, but was directly used in the next reaction without any purification (separation) at this step (yield amount: 21.5 g, yield rate: 92.3%).

The analysis results of Compound (18) are shown below.
Mass spectrometry: GC-MS m/z = 554 (M+), 428 (M+ -I)

From the above analysis results, it was confirmed that the synthesized product mainly contained Compound 18.

<Synthesis of Compound (19)>

According to the following reaction formula (scheme), Compound (19) was synthesized.

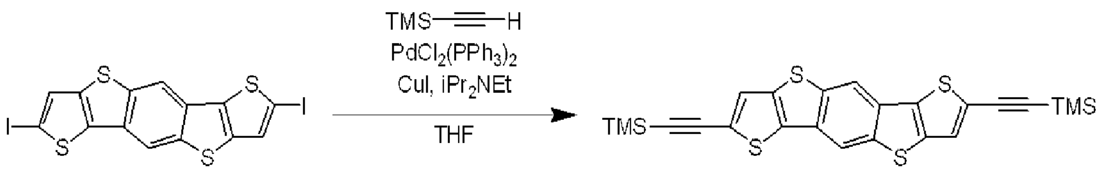


18 19

A 300 mL round-bottom flask was charged with Compound (22) (2.55 g, 4.60 mmol) and copper iodide (43.7 mg, 0.23 mmol). After the flask had been purged with argon gas, tetrahydrofuran (hereinafter referred to as "THF," 100

mL), diisopropylethylamine (6.5 mL) and dichlorobis(triphenylphosphine)palladium(II) (hereinafter referred to as "$PdCl_2(PPh_3)_2$," 97.2 mg, 0.138 mmol) were added to the flask. While the mixture was being stirred thoroughly, trimethylsilylacetylene (1.4 mL, 10.12 mmol) was gradually added to the mixture. The resultant mixture was stirred at room temperature overnight to obtain red homogeneous solution. Water (200 mL) and toluene (100 mL) were added to the mixture to separate an organic layer. The aqueous layer was extracted with toluene (50 mL) three times. The combined organic layer was washed with saturated brine (100 mL) and dried with sodium sulfate, followed by filtration. The filtrate was concentrated and subjected to purification using a column (stationary phase: silica gel, mobile phase: toluene), to thereby obtain a red solid.

The obtained solid was recrystallized from toluene/acetonitrile to obtain Compound (19) as yellow needles (yield amount: 1.35 g, yield rate: 59.1%).

The analysis results of Compound (19) are shown below.

Mass spectrometry: GC-MS m/z = 494.0(M+)

From the above analysis results, it was confirmed that a structure of the synthesized product did not contradict that of Compound (19).

<Synthesis of Compound (20)>

According to the following reaction formula (scheme), Compound (20) was synthesized.

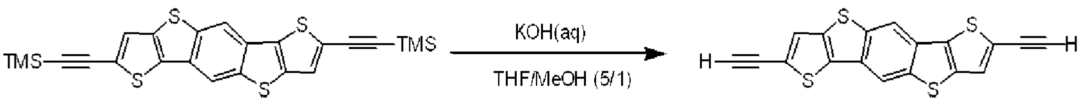


19 20

A 300 mL round-bottom flask was charged with Compound (19) (2.3 g, 4.65 mmol), THF (100 mL) and methanol (30 mL). Then, potassium hydroxide solution (prepared by dissolving 1.2 g of potassium hydroxide in 15 mL of water) was added to the resultant mixture, followed by stirring for 3 hours. Water (100 mL) and methanol (100 mL) were added to the mixture. The precipitates were recovered through filtration and washed sequentially with water and methanol, followed by drying under vacuum, to thereby obtain Compound (20) as a brown solid (yield amount 1.62 g, yield rate: 99.5%).

The analysis results of Compound (20) are shown below.

Mass spectrometry: GC-MS m/z = 349.9(M+)

From the above analysis results, it was confirmed that a structure of the synthesized product did not contradict that of Compound (20).

<Synthesis of Compound (21)>

According to the following reaction formula (scheme), Compound (21) was synthesized. 2,7-Diiodo-9,10-dihydrophenanthrene serving as a starting material used was synthesized according to the method described in Chem. Mater., 2008, 20 (20), pp. 6289 to 6291.

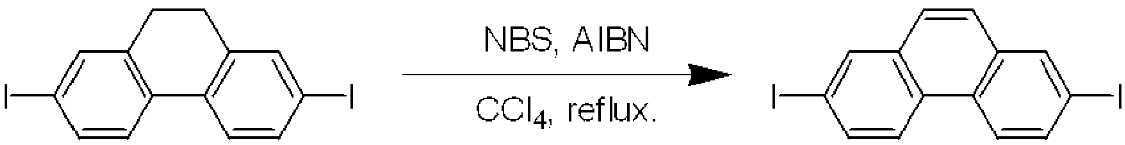


21

A 300 mL round-bottom flask was charged with 2,7-diiodo-9,10-dihydrophenanthrene (8.21 g, 19 mmol), AIBN (0.38 mmol, 62.4 mg), NBS (22.8 mmol, 4.06 g) and carbon tetrachloride (150 mL). In an argon atmosphere, the resultant mixture was stirred at a reflux temperature for 2 hours. The mixture was cooled to room temperature. The precipitates were recovered through filtration and washed sequentially with water, hot water and methanol, followed by drying under reduce pressure, to thereby obtain Compound (21) as a pale yellow solid (yield amount: 7.41 g, yield rate: 91%).

The analysis results of Compound (21) are shown below.

$^1H$ NMR (500 MHz, $CDCl_3$, TMS, $\delta$): 7.63 (s, 2H), 7.91 (dd, 2H, $J_1$=8.5 Hz, J2=1.7 Hz), 8.26 (d, 2H, J=1.7 Hz), 8.35

(d, 2H, J=8.5 Hz)
Mass spectrometry: GC-fMS m/z = 430(M+)

From the above analysis results, it was confirmed that a structure of the synthesized product did not contradict that of Compound (21).

<Synthesis of Compound (22)>

According to the following reaction formula (scheme), Compound (22) was synthesized.

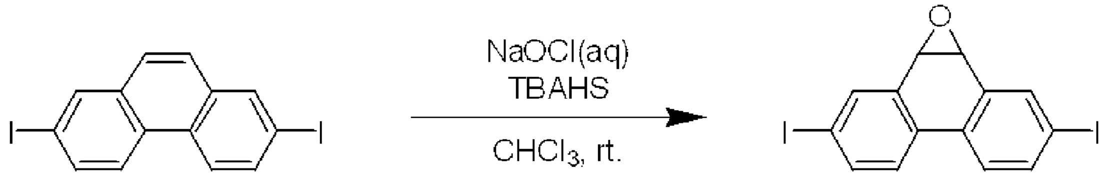


**21** **22**

A 2 L round-bottom flask was charged with Compound (21) (14 mmol, 6.02 g) and chloroform (300 mL). While the flask was being cooled in a water bath (20°C), aqueous sodium hypochlorite solution (product of KANTO KAGAKU, chlorine concentration; 5%, pH 8 to pH 10, 750 mL) and tetrabutylammonium sodium hydrogen sulfate (7 mmol, 2.38 g) were added to the mixture, followed by stirring at 20°C for 5 hours. Ice water (400 mL) was added to the mixture to separate an organic layer. The aqueous layer was extracted with chloroform twice. The combined organic layer was washed sequentially with waster and saturated brine, followed by drying with potassium carbonate. The filtrate was concentrated and purified using a column (stationary phase: silica gel, mobile phase: toluene) to obtain Compound (22) as a pale yellow solid (yield amount: 1.25 g, yield rate: 20.0%).
The analysis results of Compound (22) are shown below.

$^1$H NMR (500 MHz, $CDCl_3$, TMS, δ):4.50 (s, 2H), 7.45 (dd, 2H, J1=8.6 Hz, J2=2.3 Hz), 7.65 (d, 2H, J=2.3 Hz), 7.97 (d, 2H, J=8.6 Hz)
Mass spectrometry: GC-fMS m/z = 446(M+)

From the above analysis results, it was confirmed that a structure of the synthesized product did not contradict that of Compound (22).

<Synthesis of Compound (23)>

According to the following reaction formula (scheme), Compound (23) was synthesized.

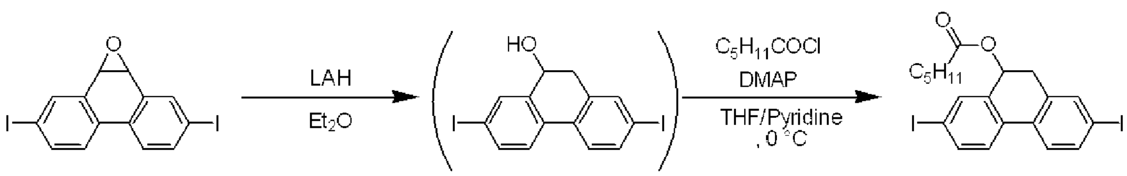


22 23

A 300 mL round-bottom flask was charged with Compound (22) (4.46 g, 10 mmol) and diethyl ether (200 mL). After the flask had been purged with argon gas, lithium aluminum hydride (12 mmol, 455 mg) was added to the mixture, followed by stirring under reflux for 4 hours. Water (0.5 mL), 1.0N aqueous sodium hydroxide solution (0.5 mL) and water (1.5 mL) were added sequentially to the mixture, followed by stirring at room temperature for 1 hour. The reaction solution was filtrated through Celite, and the filtrate was concentrated under reduced pressure to obtain an oily solid, which was then used in the next reaction without any further purification.
A 200 mL round-bottom flask was charged with the above-obtained oily solid (4.48 g), THF (30 mL), pyridine (5 mL) and DMAP (0.5 mmol, 61 mg). With ice cooling, caproic acid chloride (1.1 eq., 11 mmol, 1.48 g) was gradually added dropwise to the resultant mixture, followed by stirring at 0°C for 1 hour. The flask was taken out from the ice bath, and the mixture was stirred at room temperature for 6 hours. Subsequently, 10% aqueous sodium hydrogen carbonate

solution (100 mL) was added to the mixture, followed by stirring for 30 min. Chloroform (50 mL) was added to the mixture to separate an organic layer. The aqueous layer was extracted with chloroform twice. The combined organic layer was washed sequentially with 10% aqueous sodium hydrogen carbonate solution, water and saturated brine and dried with sodium sulfate. The filtrate was concentrated to obtain Compound (23) as pale yellow oil (yield amount: 3.55 g, yield rate: 65%).

The analysis results of Compound (23) are shown below.

> $^1$H NMR (500 MHz, $CDCl_3$, TMS, δ):0.86 (t, 3H, J =7.2 Hz), 1.21-1.30 (m, 4H), 1.54-1.60 (m, 2H), 2.20-2.23 (m, 2H), 3.05 (d, 2H), 6.05(t, 1H), 7.45-7.95 (m, 6H)
> Mass spectrometry: GC-fMS m/z =546(M+), 430(thermally decomposed product)

From the above analysis results, it was confirmed that a structure of the synthesized product did not contradict that of Compound (23).

[Synthesis Example 3: Synthesis of Compound of Comparative Example 1 (Comp. Ex. 1 Compound)]

According to the following reaction formula (scheme), Comp. Ex. 1 compound was synthesized.

Iodotetralin derivative (7') (intermediate) and Comp. Ex. 1 Compound were synthesized according to the method described in International Publication No. WO/2011-030918.

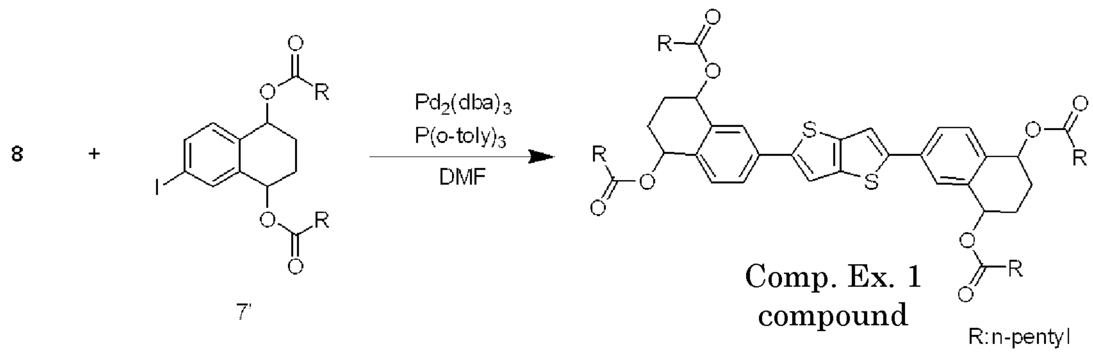


A 100 mL round-bottom flask was charged with Compound (7') (973 mg, 2.0 mmol), Compound 8 (466 mg, 1 mmol) and DMF (10 mL). The resultant mixture was bubbled with argon gas for 30 min. Tris(dibenzylideneacetone)dipalladium(0) (18.3 mg, 0.02 mmol) and tri(orthotolyl)phosphine (24.4 mg, 0.08 mmol) were added to the mixture, followed by stirring at room temperature for 20 hours in an argon atmosphere. The reaction solution was diluted with chloroform, and insoluble matter was removed through filtration using Celite. Water was added to the filtrate to separate an organic layer. The aqueous layer was extracted with chloroform three times. The combined organic layer was washed sequentially with aqueous potassium fluoride solution and saturated brine and dried with magnesium sulfate. The filtrate was concentrated to obtain a red liquid. The obtained liquid was purified through column chromatography (stationary phase: neutral silica gel (product of KANTO KAGAKU) + 10% by mass potassium fluoride, solvent: hexane/ethyl acetate, 9/1→8/2, v/v) to obtain a yellow solid. The obtained solid was recrystallized from hexane/ethanol to obtain Comp. Ex. 1 compound as a yellow solid (yield amount: 680 mg, yield rate: 79.3%).

The analysis results of Comp. Ex. 1 compound are shown below.

> $^1$H NMR (500 MHz, $CDCl_3$, TMS, δ): 0.87-0.89(m,12H), 1.28-1.33(m,16H), 1.61-1.69(m,8H), 1.96-2.01(m,4H), 2.28-2.36(m,12H), 6.08(d,4H,J=12.1Hz), 7.37(d,2H,J=8.6Hz), 7.48(s,2H), 7.57-7.59(m,4H)
> Elemental analysis ($C_{50}H_{64}O_8S_2$): C,69.92; H,7.67; 0,14.85; S,7.44 (found), C,70.06; H,7.53; 0,14.93; S,7.48 (calculated)
> Melting point: 113.7°C-114.7°C

From the above analysis results, it was confirmed that a structure of the synthesized product did not contradict that of Comp. Ex. 1 compound.

Next, the leaving substituent-containing compound was synthesized using the specific compound intermediates synthesized in the above Synthesis Examples.

Also, the specific compound intermediates synthesized in the above Synthesis Examples were used to synthesize π-electron conjugated compound precursors. Notably, the below description on the "leaving substituent-containing compound" is also applied to the "π-electron conjugated compound precursor" by replacing the "leaving substituent-containing compound" with the "π-electron conjugated compound precursor" in the below description.

(Examples 1 and 2)

<Synthesis of Compound of Example 1 (Ex. 1 compound) and Compound of Example 2 (Ex. 2 compound)>

According to the following reaction formula (scheme), Ex. 1 compound and Ex. 2 compound were synthesized.

**7-1** + **8**

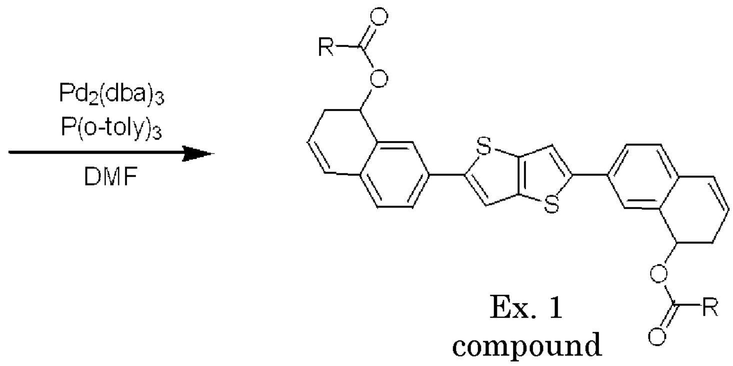


Ex. 1 compound

R:n-pentyl

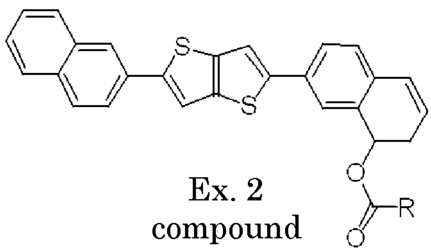

R:n-pentyl

Ex. 2 compound

A 100 mL round-bottom flask was charged with Compound (7-1) (550 mg, 1.49 mmol), Compound (8) (346 mg, 0.74 mmol) and N,N-dimethylformamide (hereinafter abbreviated as "DMF," 10 mL). The resultant mixture was bubbled with argon gas for 30 min. Tris(dibenzylideneacetone)dipalladium(0) (18.3 mg, 0.02 mmol) and tri(orthotolyl)phosphine (24.4 mg, 0.08 mmol) were added to the mixture, followed by stirring at room temperature for 24 hours in an argon atmosphere. The reaction solution was diluted with dichloromethane, and water was added to the mixture to separate an organic layer. The aqueous layer was extracted with dichloromethane three times. The combined organic layer was washed sequentially with aqueous saturated potassium fluoride solution and saturated brine and dried with magnesium sulfate, followed by filtration. The filtrate was caused pass through a silica gel pad (thickness: 3 cm), followed by concentration, to thereby obtain a red solid. The obtained solid was washed with methanol and hexane to obtain a yellow-green solid (yield amount: 235 mg).

The obtained solid was separated and purified with a recycle preparative HPLC (product of Japan Analytical Industry Co., Ltd., LC-9104) to obtain Ex. 1 compound (yield amount: 85 mg) and Ex. 2 compound (yield amount: 110 mg) as yellow crystals.

The analysis results of Ex. 1 compound are shown below. [Ex. 1 compound];

$^1H$ NMR (500 MHz, $CDCl_3$, TMS, δ) : 0.86 (t, 6H, J = 6.9 Hz), 1.21-1.31 (m, 8H) , 1.57-1.63 (m, 4H), 2.27 (td, 2H, $J_1$=7.6 Hz $J_2$= 1.7 Hz ), 2.60-2.70 (m, 4H), 5.95 (t, 1H, J = 5.2 Hz), 6.03-6.09 (m, 4H), 6.63 (d, 2H, J = 9.7 Hz), 7.40 (d, 4H, J = 8.1 Hz), 7.49 (s, 2H), 7.491 (dd, 2H, $J_1$ = 7.7Hz, $J_2$ = 2.3 Hz)
Precise mass (LC-TofMS) (m/z): 624.232 (found), 624.237 (calculated)

From the above analysis results, it was confirmed that a structure of the synthesized product did not contradict that of Ex. 1 compound.

The analysis results of Ex. 2 compound are shown below. [Ex. 2 compound];

$^1H$ NMR (500 MHz, $CDCl_3$, TMS, δ): 0.86 (t, 3H, J =7.5 Hz), 1.22-1.32 (m, 4H) , 1.57-1.64 (m, 2H), 2.28 (td, 2H, $J_1$=7.7 Hz $J_2$= 1.2 Hz ), 2.62-2.72 (m, 2H), 6.03-6.10 (m, 2H), 6.63 (d, 1H, J = 9.8 Hz), 7.40-7.42 (m, 2H,), 7.46-7.52 (m, 3H), 7.53 (s, 1H), 7.61 (s, 1H), 7.79 (dd, 2H, $J_1$ = 8.6 Hz, $J_2$= 1.7 Hz), 7.84 (d, 1H, J = 8.1 Hz), 7.88 (d, 2H, J

= 8.1 Hz), 8.07 (d, 1H, J = 8.1 Hz),
Precise mass (LC-TofMS) (m/z): 508.149 (found), 508.153 (calculated)

From the above analysis results, it was confirmed that a structure of the synthesized product did not contradict that of Ex. 2 compound.

(Example 3)

<Synthesis of Compound of Example 3 (Ex. 3 compound)>

According to the following reaction formula (scheme), Ex. 3 compound was synthesized.

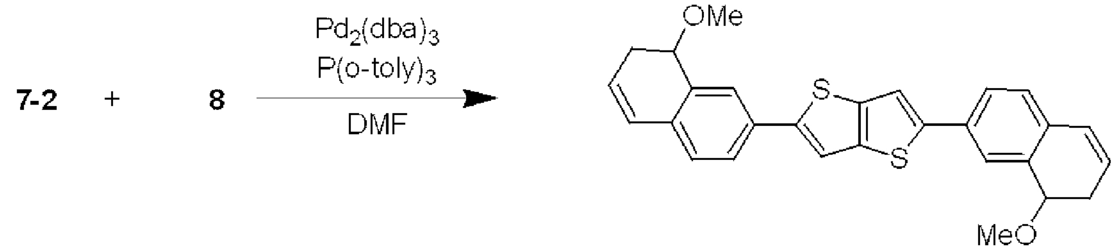


Ex. 3 compound

The procedure of Example 1 was repeated, except that Compound (7-1) was changed to Compound (7-2), to thereby obtain Ex. 3 compound as yellow crystals (yield amount: 253 mg, yield rate: 75%).
The analysis results of Ex. 3 compound are shown below. [Ex. 3 compound];

$^1$H NMR (500 MHz, $CDCl_3$, TMS, $\delta$): 2.60-2.70 (m, 4H), 3.38 (s, 6H), 5.90 (t, 2H, J = 5.2 Hz), 6.03-6.09 (m, 4H), 6.63 (d, 2H, J = 9.7 Hz), 7.40 (d, 4H, J = 8.1 Hz), 7.49 (s, 2H), 7.50 (dd, 2H, $J_1$ = 7.7Hz, $J_2$ = 2.3 Hz)
Precise mass (LC-TofMS) (m/z): 456.127 (found), 456.122 (calculated)

From the above analysis results, it was confirmed that a structure of the synthesized product did not contradict that of Ex. 3 compound.

(Example 4)

<Synthesis of Compound of Example 4 (Ex. 4 compound)>

According to the following reaction formula (scheme), Ex. 4 compound was synthesized.

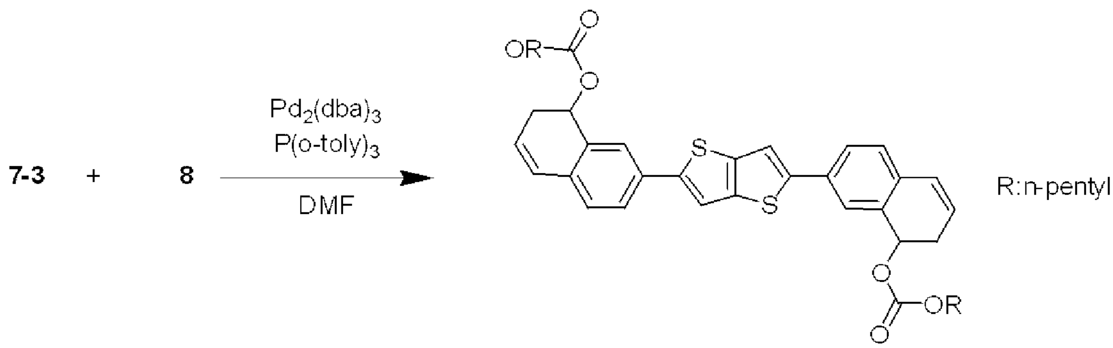


Ex. 4 compound

The procedure of Example 1 was repeated, except that Compound (7-1) was changed to Compound (7-3), to thereby obtain Ex. 4 compound as yellow crystals (yield amount: 291 mg, yield rate: 60%).

The analysis results of Ex. 4 compound are shown below. [Ex. 4 compound];

$^1$H NMR (500 MHz, $CDCl_3$, TMS, δ): 0.86 (t, 6H, J = 6.9 Hz), 1.21-1.31 (m, 8H) , 1.57-1.63 (m, 4H), 2.60-2.70 (m, 4H), 4.15-4.17 (m, 2H), 5.95 (t, 2H, J = 5.2 Hz), 6.03-6.09 (m, 4H), 6.63 (d, 2H, J = 9.7 Hz), 7.40 (d, 4H, J = 8.1 Hz), 7.49 (s, 2H), 7.491 (dd, 2H, $J_1$ = 7.7Hz, $J_2$ = 2.3 Hz)
Precise mass (LC-TofMS) (m/z): 656.232 (found) 656.227 (calculated)

From the above analysis results, it was confirmed that a structure of the synthesized product did not contradict that of Ex. 4 compound.

(Example 5)

<Synthesis of Compound of Example 5 (Ex. 5 compound)>

According to the following reaction formula (scheme), Ex. 5 compound was synthesized.

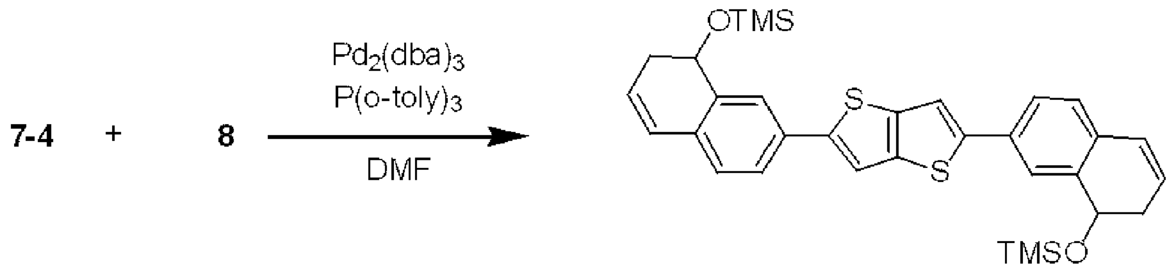


Ex. 5 compound

The procedure of Example 1 was repeated, except that Compound (7-1) was changed to Compound (7-4), to thereby obtain Ex. 5 compound (yield amount: 193 mg, yield rate: 45.5%). Here, in the above reaction formula, the TMS group is an abbreviation of a trimethylsilyl group.

The analysis results of Ex. 5 compound are shown below. [Ex. 5 compound];

$^1$H NMR (500 MHz, $CDCl_3$, TMS, δ): 0.04 (s, 18H), 2.60-2.70 (m, 4H), 5.15 (t, 2H, J =5.2 Hz), 6.00 (t, 2H, J = 5.2 Hz), 6.03-6.09 (m, 4H), 6.63 (d, 2H, J = 9.7 Hz), 7.40 (d, 4H, J = 8.1 Hz), 7.48 (s, 2H), 7.50 (dd, 2H, $J_1$ = 7.7Hz, $J_2$ = 2.3 Hz)
Precise mass (LC-TofMS) (m/z): 572.175 (found), 572.170 (calculated)

From the above analysis results, it was confirmed that a structure of the synthesized product did not contradict that of Ex. 5 compound.

(Example 6)

<Synthesis of Compound of Example 6 (Ex. 6 compound)>

According to the following reaction formula (scheme), Ex. 6 compound was synthesized.

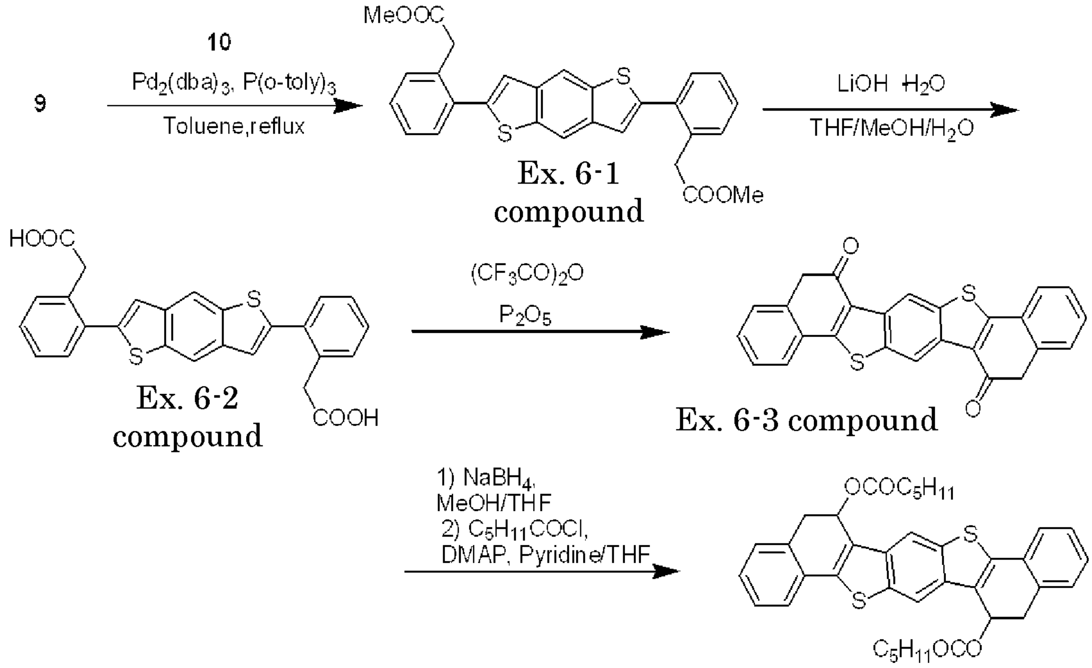


Ex. 6-1 compound

Ex. 6-2 compound

Ex. 6-3 compound

Ex. 6 compound

<Synthesis of Compound of Example 6-1 (Ex. 6-1 compound)>

A 200 mL three-neck flask was charged with Compound 9 (2.58 g, 5 mmol), Compound 10 (2.2 eq., 11 mmol, 2.52 g) and toluene (100 mL). The resultant mixture was bubbled with argon gas for 30 min. Tris(dibenzylidneacetone)dipalladium(0) (229 mg, 0.25 mmol) and tri(orthotolyl)phosphine (304 mg, 1.0 mmol) were added to the mixture, followed by refluxing for 8 hours in an argon atmosphere.

The reaction solution was filtrated with a silica gel pad (thickness: 3 cm). The filtrate was concentrated to obtain a brown solid. The obtained solid was recrystallized from toluene to obtain Ex. 6-1 compound as pale yellow crystals (yield amount: 1.85 g, yield rate: 76%).

The analysis results of Ex. 6-1 compound are shown below.

Mass spectrometry: GC-MS m/z = 486(M+)

From the above analysis results, it was confirmed that a structure of the synthesized product did not contradict that of Ex. 6-1 compound.

<Synthesis of Compound of Example 6-2 (Ex. 6-2 compound)>

A 300 mL round-bottom flask was charged with Ex. 6-1 compound (1.8 g, 3.7 mmol), water (30 mL), methanol (30 mL) and THF (90 mL). After the flask had been purged with argon gas, lithium hydroxide monohydrate (3 eq., 11.1 mmol, 466 mg) was added to the mixture, followed by stirring at 80°C for 3 hours. The mixture was cooled to room temperature, and then concentrated hydrochloric acid was added to the mixture to acidify the system. The precipitate was recovered through filtration using a PTFE filter, washed sequentially with water and hexane, and dried at 60°C under reduced pressure, to thereby obtain Ex. 6-2 compound as a pale yellow solid (yield amount: 1.6 g, yield rate: 94%).

The analysis results of Ex. 6-2 compound are shown below.

Mass spectrometry: GC-MS m/z = 458(M+)

$^1$H NMR (500 MHz, $CDCl_3$, TMS, $\delta$): 3.79 (s, 4H), 7.39-7.44 (m, 6H), 7.47 (s, 2H), 7.53 (dd, 2H, $J_1$= 6.9 Hz, J2= 1.7 Hz), 8.49 (s, 2H), 12.3-12.5 (br, 2H)

From the above analysis results, it was confirmed that a structure of the synthesized product did not contradict that of Ex. 6-2 compound.

<Synthesis of Compound of Example 6-3 (Ex. 6-3 compound)>

A 100 mL round-bottom flask was charged with Ex. 6-2 compound (2 mmol, 916 mg) and purged with argon

gas. With ice cooling, trifluoromethanesulfonic anhydride (10 mL) and phosphorus pentoxide (0.5 g) were added to the mixture, followed by stirring at the same temperature for 2 hours. The mixture was poured into ice water (200 g). The precipitate was recovered through filtration and washed sequentially with water and hexane, to thereby obtain Ex. 6-3 compound as a yellow solid, which was then used in the next reaction without any further purification.

The analysis results of Ex. 6-3 compound are shown below.

Mass spectrometry: GC-MS m/z = 422(M+)
IR: 1720 (C=O, ketone)

From the above analysis results, it was confirmed that a structure of the synthesized product did not contradict that of Ex. 6-3 compound.

<Synthesis of Compound of Example 6 (Ex. 6 compound)>

A 100 mL round-bottom flask was charged with Ex. 6-3 compound (2 mmol, 844 mg), methanol (20 mL) and THF (40 mL). With ice cooling, sodium borohydride (5 eq., 10 mmol, 378 mg) was added to the mixture, followed by stirring at the same temperature for 2 hours. The mixture was poured into ice water (200 g). The precipitate was recovered through filtration and washed sequentially with water and hexane, to thereby obtain a pale yellow solid. The obtained solid was dried under reduced pressure and used in the next reaction without any further purification.

A 100 mL round-bottom flask was charged with the above-obtained solid and DMAP (0.1 mmol, 12.2 mg). After the flask had been purged with argon gas, THF (20 mL) and pyridine (2 mL) were added to the mixture. With ice cooling, caproic acid chloride (4 eq., 8 mmol, 1.07 g) was added dropwise to the mixture for 5 min. The resultant mixture was stirred at the same temperature for 4 hours until the starting materials disappeared. Water (100 mL) and ethyl acetate (100 mL) were added to the reaction solution to separate an organic layer. The aqueous layer was extracted with ethyl acetate (30 mL) twice. The combined organic layer was washed with saturated brine and dried with sodium sulfate. The filtrate was concentrated to obtain crude Ex. 6 compound as a yellow solid. The obtained crude product was purified through column chromatography (stationary phase: silica gel, mobile phase: toluene) to obtain a pale yellow solid, which was further recrystallized from toluene/ethanol, to thereby obtain Ex. 6 compound as pale yellow crystals (yield amount: 149 mg, yield rate: 12%).

The analysis results of Ex. 6 compound are shown below.

Precise mass (LC-TofMS) (m/z): 622.228 (found), 622.221 (calculated)
Mass spectrometry: GC-MS m/z = 622(M+), 390 (thermally decomposed product)
Decomposition temperature: 200°C or lower

From the above analysis results, it was confirmed that a structure of the synthesized product did not contradict that of Ex. 6 compound.

(Example 7)

<Synthesis of Compound of Example 7 (Ex. 7 compound)>

According to the following reaction formula (scheme), Ex. 7 compound was synthesized.

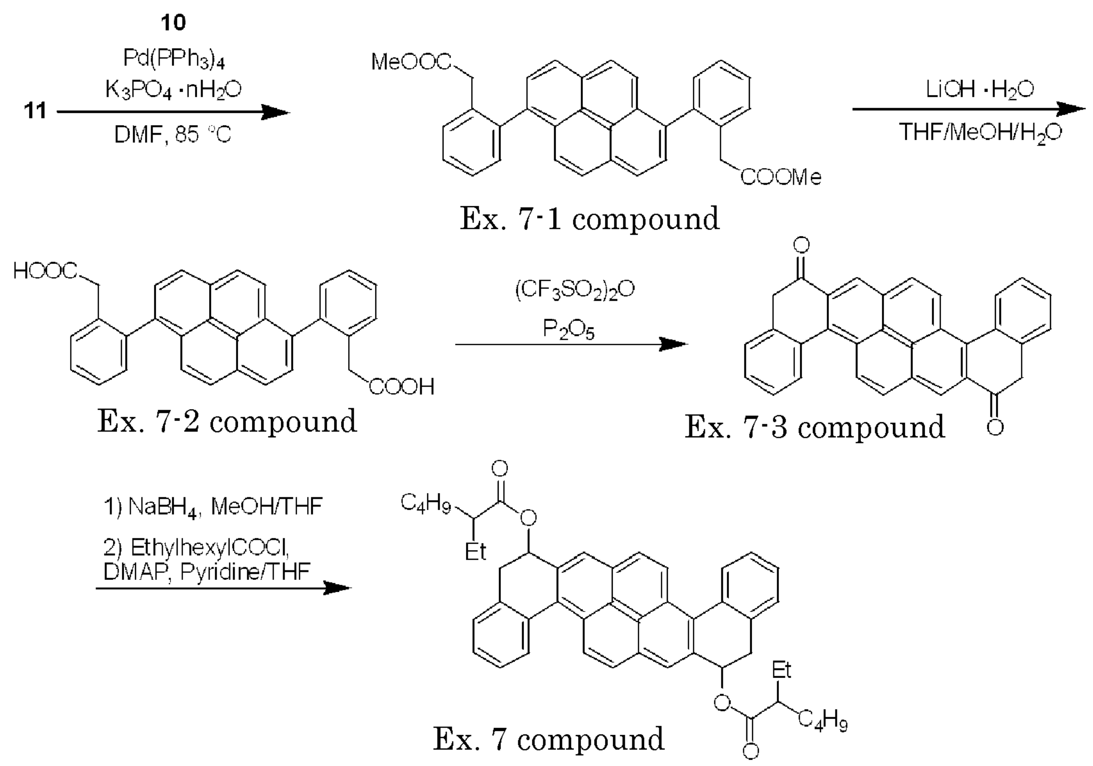


Ex. 7-1 compound

Ex. 7-2 compound

Ex. 7-3 compound

Ex. 7 compound

<Synthesis of Compound of Example 7-1 (Ex. 7-1 compound)>

A 200 mL three-neck flask was charged with Compound 10 (2.0 g, 8.7 mmol), Compound 11 (3.92 mmol, 1.78 g), potassium phosphate hydrate (13 g) and DMF/toluene (1/1, 100 mL). The resultant mixture was bubbled with argon gas for 30 min. Tris(dibenzylidneacetone)dipalladium(0) (383 mg, 0.42 mmol) and tri(orthotolyl)phosphine (510 mg, 1.67 mmol) were added to the mixture, followed by stirring at 85°C for 7 hours in an argon atmosphere. Saturated ammonium chloride solution, water and toluene were added to the reaction solution to separate an organic layer. The aqueous layer was extracted with toluene twice. The combined organic layer was washed sequentially with water and saturated brine and dried with magnesium sulfate, followed by filtration. The filtrate was concentrated to obtain a yellow solid.

The obtained solid was purified using a column (stationary phase: silica gel, mobile phase: toluene→toluene/ethyl acetate = 9/1) to obtain Ex. 7-1 compound as a yellow solid (yield amount: 1.09 g, yield rate: 56%).

The analysis results of Ex. 7-1 compound are shown below.

Mass spectrometry: GC-MS m/z = 498 (M+)

From the above analysis results, it was confirmed that a structure of the synthesized product did not contradict that of Ex. 7-1 compound.

<Synthesis of Compound of Example 7-2 (Ex. 7-2 compound)>

A 300 mL round-bottom flask was charged with Ex. 7-1 compound (498 mg, 1 mmol), water (5 mL), methanol (5 mL) and THF (15 mL). After the flask had been purged with argon gas, lithium hydroxide monohydrate (3 eq., 140 mg) was added to the mixture, followed by stirring at 80°C for 2 hours. The resultant mixture was cooled to room temperature, and then 1N hydrochloric acid was added to the mixture to acidify the system. Ethyl acetate was added to the mixture to separate an organic layer. The aqueous layer was extracted with ethyl acetate twice. The combined organic layer was washed sequentially with water and saturated brine and dried with sodium sulfate, followed by filtration. The filtrate was concentrated to obtain Ex. 7-2 compound as a yellow solid (yield amount: 447 mg, yield rate: 95%).

The analysis results of Ex. 7-2 compound are shown below.

Mass spectrometry: GC-MS m/z = 470(M+)

From the above analysis results, it was confirmed that a structure of the synthesized product did not contradict that of Ex. 7-2 compound.

<Synthesis of Compound of Example 7-3 (Ex. 7-3 compound)>

A 100 mL round-bottom flask was charged with Ex. 7-2 compound (0.8 mmol, 376 mg) and purged with argon gas. With ice cooling, trifluoromethanesufonic anhydride (10 mL) and phosphorus pentoxide (0.5 g), followed by stirring at the same temperature for 2 hours. The mixture was poured into ice water (200 g). The precipitate was recovered through filtration, and washed sequentially with water and hexane, to thereby obtain Ex. 7-3 compound as a yellow solid, which was then used in the next reaction without any further purification.

The analysis results of Ex. 7-3 compound are shown below.

Mass spectrometry: GC-MS m/z = 434(M+)
IR: 1724 (C=O, ketone)

From the above analysis results, it was confirmed that a structure of the synthesized product did not contradict that of Ex. 7-3 compound.

<Synthesis of Compound of Example 7 (Ex. 7 compound)>

A 100 mL round-bottom flask was charged with Ex. 7-3 compound (0.8 mmol, 376 mg), methanol (20 mL) and THF (40 mL). With ice cooling, sodium borohydride (5 eq., 4.0 mmol, 151 mg) was added to the resultant mixture, followed by stirring at the same temperature for 2 hours.

The mixture was poured into ice water (200 g). The precipitate was recovered through filtration and washed sequentially with water and hexane, to thereby obtain a pale yellow solid. The obtained solid was dried under reduced pressure and used in the next reaction without any further purification.

A 100 mL round-bottom flask was charged with the above-obtained solid and DMAP (0.1 mmol, 12.2 mg). After the flask had been purged with argon gas, THF (20 mL) and pyridine (2 mL) were added to the mixture. With ice cooling, 2-ethylhexanoyl chloride (4 eq., 3.2 mmol, 517 mg) was added dropwise to the mixture for 5 min. The resultant mixture was stirred at the same temperature for 6 hours until the starting materials disappeared. Water (100 mL) and ethyl acetate (100 mL) were added to the reaction solution to separate an organic layer. The aqueous layer was extracted with ethyl acetate (30 mL) twice. The combined organic layer was washed with saturated brine and dried with sodium sulfate, followed by filtration. The filtrate was concentrated to obtain crude Ex. 7 compound as a yellow solid. The obtained solid was purified through column chromatography (stationary phase: silica gel, mobile phase: toluene→toluene/ethyl acetate = 95/5) to obtain a pale yellow solid. Further, the obtained solid was recrystallized from toluene/ethanol to obtain Ex. 7 compound as a pale yellow solid (yield amount: 55.2 mg, yield rate: 10%).

The analysis results of Ex. 7 compound are shown below.

Precise mass (LC-TofMS) (m/z): 690.366 (found), 690.371 (calculated)
Mass spectrometry: GC-MS m/z = 690(M+), 402 (thermally decomposed product)
Decomposition temperature: 200°C or lower

From the above analysis results, it was confirmed that a structure of the synthesized product did not contradict that of Ex. 7 compound.

(Example 8)

<Synthesis of Compound of Example 8 (Ex. 8 compound)>

According to the following reaction formula (scheme), Ex. 8 compound was synthesized.

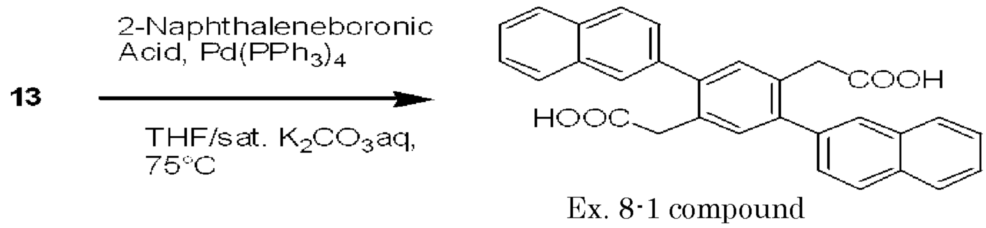


Ex. 8-1 compound

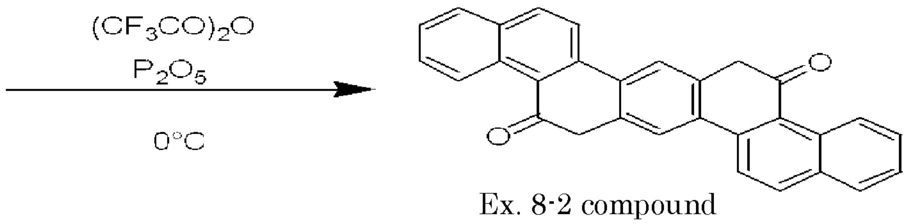


Ex. 8-2 compound

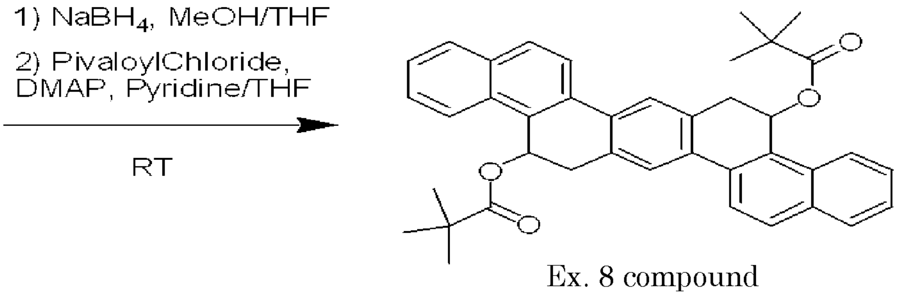


Ex. 8 compound

<Synthesis of Compound of Example 8-1 (Ex. 8-1 compound)>

A 200 mL three-neck flask was charged with Compound 13 (1.6 g, 3.6 mmol), aqueous saturated potassium carbonate solution (20 mL) and THF (40 mL). The resultant mixture was bubbled with argon for 30 min. Thereafter, 2-naphthaleneboronic acid (1.55 g, 9.0 mmol) and tetrakis(triphenylphosphine)palladium(0) (208 mg, 0.36 mM) were added to the mixture, followed by stirring under heating at 75°C for 8 hours. After confirming completion of the reaction through TLC, the mixture was cooled to room temperature. The precipitate of interest was separated through filtration and recrystallized from toluene to obtain Ex. 8-1 compound (yield amount: 800 mg, yield rate: 50%).

The analysis results of Ex. 8-1 compound are shown below.

Mass spectrometry: GC-MS m/z = 446(M+)

From the above analysis results and the Rf value obtained through TLC, it was confirmed that a structure of the synthesized product did not contradict that of Ex. 8-1 compound.

A 100 mL round-bottom flask was charged with Ex. 8-1 compound (821 mg, 2 mmol) and purged with argon gas. With ice cooling, trifluoromethanesulfonic anhydride (10 mL) and phosphorus pentoxide (0.5 g) were added to the mixture, followed by stirring at the same temperature for 2 hours. The mixture was poured into ice water (200 g). The precipitate was recovered through filtration and washed sequentially with water and hexane, to thereby obtain Ex. 8-2 compound as a yellow solid. Although the corresponding enol compound (tautomer) was observed in addition to Ex. 8-2 compound, the obtained solid was used in the next reaction without any further purification.

The analysis results of Ex. 8-2 compound are shown below.

Mass spectrometry: GC-MS m/z = 410(M+)

From the above analysis results, it was confirmed that a structure of the synthesized product did not contradict that of Ex. 8-2 compound.

<Synthesis of Compound of Example 8 (Ex. 8 compound)>

A 100 mL round-bottom flask was charged with Ex. 8-2 compound (1 mmol, 410 mg), ethanol (20 mL) and THF (40 mL). With ice cooling, sodium borohydride (5 eq., 5 mmol, 189 mg) was added to the resultant mixture, followed by stirring at the same temperature for 2 hours. The mixture was poured into ice water (200 g). The precipitate was recovered through filtration and washed sequentially with water and hexane to obtain a pale yellow solid. The obtained solid was dried under reduced pressure and used in the next reaction without any further purification.

A 100 mL round-bottom flask was charged with the above-obtained solid and DMAP (0.1 mmol, 12.2 mg). After

the flask had been purged with argon gas, THF (50 mL) and pyridine (2 mL) were added to the mixture. With ice cooling, pivaloyl chloride (4 eq., 8 mmol, 1.07 g) was added dropwise to the mixture for 5 min. The resultant mixture was stirred at the same temperature for 4 hours until the starting materials disappeared. Water (100 mL) and ethyl acetate (100 mL) were added to the reaction solution to separate an organic layer. The aqueous layer was extracted with ethyl acetate (30 mL) twice. The combined organic layer was washed with saturated brine and dried with sodium sulfate, followed by filtration. The filtrate was concentrated to obtain crude Ex. 8 compound as a yellow solid. The obtained solid was purified through column chromatography (stationary phase: silica gel, mobile phase: toluene) to obtain a pale yellow solid.

Further, the obtained solid was recrystallized from toluene/ethanol to obtain Ex. 8 compound as pale yellow crystals (yield amount: 87 mg, yield rate: 15%).

The analysis results of Ex. 8 compound are shown below.
Precise mass (LC-TofMS) (m/z): 582.273 (found), 582.277 (calculated)

From the above analysis results, it was confirmed that a structure of the synthesized product did not contradict that of Ex. 8 compound.

(Example 9)

<Synthesis of Compound of Example 9 (Ex. 9 compound)>

According to the following reaction formula (scheme), Ex. 9 compound was synthesized.

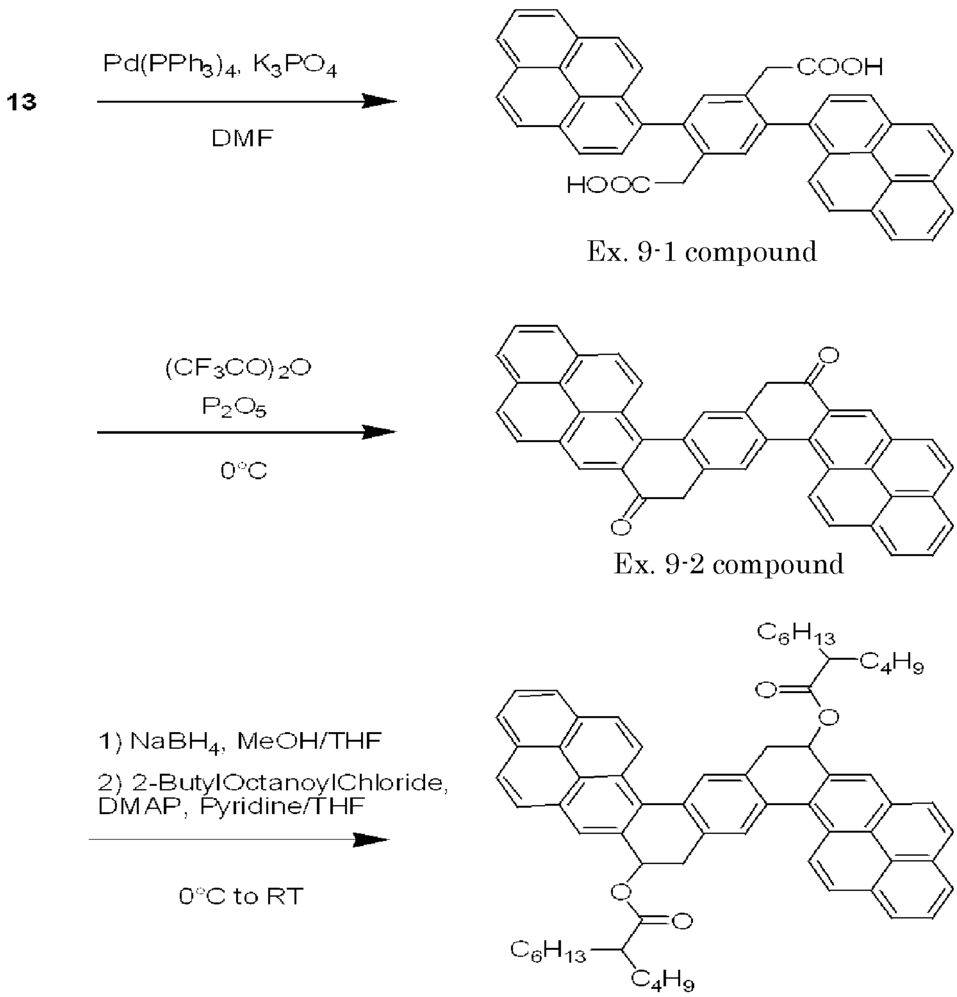


Ex. 9-1 compound

Ex. 9-2 compound

Ex. 9 compound

<Synthesis of Compound of Example 9-1 (Ex. 9-1 compound)>

A 200 mL three-neck flask was charged with Compound 13 (1.6 g, 1.0 mmol), potassium phosphate (0.5 g)

and DMF (30 mL). The resultant mixture was bubbled with argon for 30 min. Thereafter, 1-pyrenylboronic acid (0.62 g, 2.5 mmol) and tetrakis(triphenylphosphine)palladium(0) (104 mg, 0.18 mM) were added to the mixture, followed by stirring under heating at 70°C for 8 hours. The mixture was cooled to room temperature. The precipitate of interest was recovered through filtration, washed with hexane, and recrystallized from toluene, to thereby obtain Ex. 9-1 compound (yield amount: 500 mg, yield rate: 84.1%).

The analysis results of Ex. 9-1 compound are shown below.
Mass spectrometry: GC-MS m/z = 594(M+)

From the above analysis results, it was confirmed that a structure of the synthesized product did not contradict that of Ex. 9-1 compound.

<Synthesis of Compound of Example 9-2 (Ex. 9-2 compound)>

A 100 mL round-bottom flask was charged with Ex. 9-1 compound (1.49 g, 2.5 mmol) and purged with argon gas. With ice cooling, trifluoromethanesulfonic anhydride (50 mL) and phosphorus pentoxide (1 g) were added to the mixture, followed by stirring at the same temperature for 8 hours. The mixture was poured into ice water (total amount: 1 kg). The precipitate was recovered through filtration and washed sequentially with water and hexane, to thereby obtain Ex. 9-2 compound as a yellow solid. Although the corresponding enol compound (tautomer) was observed in addition to Ex. 9-2 compound, the obtained solid was used in the next reaction without any further purification.

The analysis results of Ex. 9-2 compound are shown below.
Mass spectrometry: GC-MS m/z = 558(M+)

From the above analysis results, it was confirmed that a structure of the synthesized product did not contradict that of Ex. 9-2 compound.

<Synthesis of Compound of Example 9 (Ex. 9 compound)>

A 300 mL round-bottom flask was charged with Ex. 9-2 compound (1.3 mmol, 726 mg), ethanol (50 mL) and THF (200 mL). With ice cooling, sodium borohydride (7.8 mmol, 295 mg) was added to the resultant mixture, followed by stirring at the same temperature for 2.5 hours. The mixture was poured into ice water (1 kg). The precipitate was recovered through filtration and washed sequentially with water and hexane, to thereby obtain a pale yellow solid. The obtained solid was dried under reduced pressure and used in the next reaction without any further purification.

A 300 mL round-bottom flask was charged with the above-obtained solid and DMAP (0.13 mmol, 15.9 mg). After the flask had been purged with argon gas, THF (200 mL) and pyridine (10 mL) were added to the mixture. With ice cooling, 2-butyloctanoyl chloride (1.13 g, 5.2 mmol) was added dropwise to the mixture. The resultant mixture was stirred at 0°C for 4 hours until the starting materials disappeared. The mixture was returned to room temperature. Water (100 mL) and ethyl acetate (100 mL) were added to the reaction solution to separate an organic layer. The aqueous layer was extracted with ethyl acetate (30 mL) twice. The combined organic layer was washed with saturated brine and dried with sodium sulfate, followed by filtration. The filtrate was concentrated to obtain crude Ex. 9 compound as a yellow solid. The obtained solid was purified through column chromatography (stationary phase: silica gel, mobile phase: toluene) to obtain a pale yellow solid (yield amount: 84 mg, yield rate: 7%).

The analysis results of Ex. 9 compound are shown below.
Precise mass (LC-TofMS) (m/z): 926.533 found), 926.527 (calculated)

From the above analysis results, it was confirmed that a structure of the synthesized product did not contradict that of Ex. 9 compound.

(Example 10)

<Synthesis of Compound of Example 10 (Ex. 10 compound)>

According to the following reaction formula (scheme), Ex. 10 compound was synthesized.

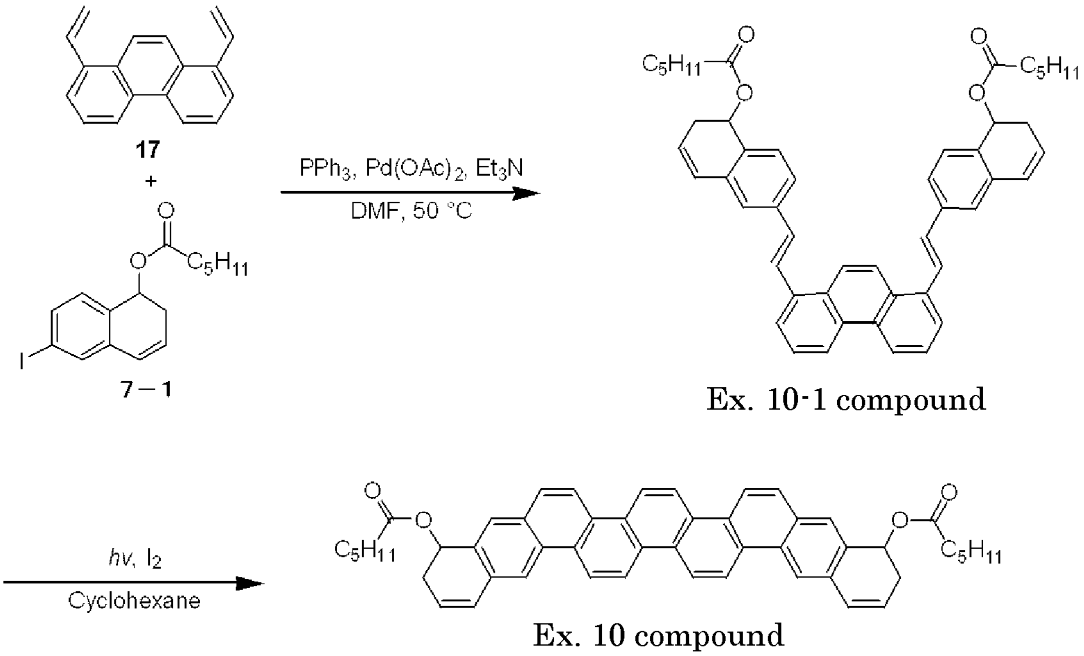


17 + 7-1

Ex. 10-1 compound

Ex. 10 compound

<Synthesis of Compound of Example 10-1 (Ex. 10-1 compound)>

A 100 mL four-neck flask was charged with DMF (30 mL), Compound (17) (0.20 g, 0.89 mmol), Compound (7-1) (2.2 eq., 1.96 mmol, 726 mg), triphenylphosphine (9.1 mg, 0.034 mol) and triethylamine (0.34 mL, 2.4 mmol). The resultant mixture was stirred for 45 min with argon gas bubbling. Palladium acetate (3.9 mg, 0.017 mmol) was added to the mixture, followed by stirring at 50°C for 24 hours.

The mixture was cooled to room temperature, followed by filtration with Celite. The obtained solution was extracted with chloroform. The organic layer was washed with saturated brine and dried with magnesium sulfate. The magnesium sulfate was removed through filtration, followed by concentration. The obtained solid was dissolved in a minimum required amount of toluene. The resultant solution was caused to pass through a silica gel pad (thickness: 2 cm), followed by concentration again. The obtained solid was separated and purified from by-products using a recycle GPC (product of Japan Analytical Industry Co., Ltd.), to thereby obtain Ex. 10-1 compound as a yellow solid (yield amount: 444 mg, yield rate: 70%).

The analysis results of Ex. 10-1 compound are shown below.

Precise mass (LC-TofMS) (m/z): 714.378 (found), 714.372 (calculated)
Mass spectrometry: GC-MS m/z = 714(M+), 483 (thermally decomposed product)

From the above analysis results, it was confirmed that a structure of the synthesized product did not contradict that of Ex. 10-1 compound.

<Synthesis of Compound of Example 10 (Ex. 10 compound)>

A 500 mL four-neck flask was charged with cyclohexane (300 mL), Ex. 10-1 compound (130 mg, 0.182 mmol) and iodine (30 mg, 0.117 mmol).

The resultant mixture was irradiated with light for 5 hours using a low-pressure mercury lamp (product of USHIO Co.). Cyclohexane was evaporated under reduced pressure, and then water and chloroform were added to the residue to separate an organic layer. The aqueous layer was extracted with chloroform twice.

The combined organic layer was washed sequentially with aqueous thiosodium sulfate solution, water and saturated brine, and dried with sodium sulfate, followed by filtration. The filtrate was concentrated to obtain a yellow oily solid. The obtained solid was separated and purified from by-products using a recycle GPC (product of Japan Analytical Industry Co., Ltd.), to thereby obtain Ex. 10 compound as a pale yellow solid (yield amount: 28.6 mg, yield rate: 22%).

The analysis results of Ex. 10 compound are shown below.

Precise mass (LC-TofMS) (m/z): 710.336 (found), 710.34 (calculated)
Mass spectrometry: GC-MS m/z =710(M+), 479 (thermally decomposed product)

Decomposition temperature: 200°C or lower

From the above analysis results, it was confirmed that a structure of the synthesized product did not contradict that of Ex. 10 compound.

(Example 11)

<Synthesis of Compound of Example 11 (Ex. 11 compound)>

According to the following reaction formula (scheme), Ex. 11 compound was synthesized.

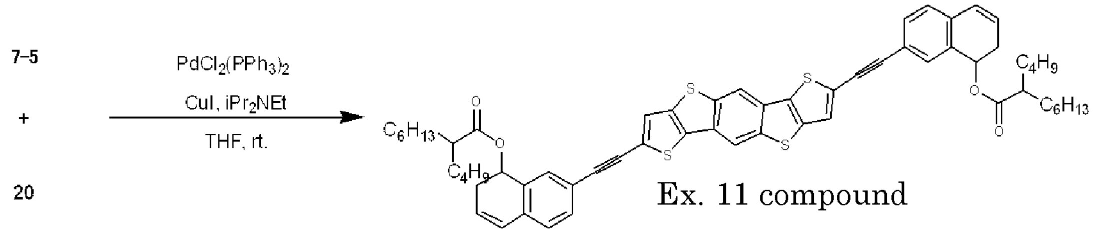


A 100 mL round-bottom flask was charged with Compound (24) (275 mg, 0.785 mmol), Compound (7-5) (750 mg, 1.65 mmol) and copper iodide (20.0 mg). THF (30 mL) and diisopropylethylamine (1.5 mL) were added to the resultant mixture. After the flask had been purged with argon gas, $PdCl_2(PPh_3)_2$ (16.6 mg) was added to the mixture, followed by stirring at room temperature for 72 hours.

Dichloromethane (100 mL) and water (100 mL) were added to the mixture to separate an organic layer. The aqueous layer was extracted with dichloromethane twice. The combined organic layer was washed sequentially with water and saturated brine and dried with sodium sulfate, followed by filtration. The filtrate was concentrated and dissolved in a minimum required amount of dichloromethane. The resultant solution was caused to pass through an alumina pad (activity II (water content: 3%)), followed by concentration again, to thereby obtain yellow oil. The obtained oil was purified using a recycle GPC (product of Japan Analytical Industry Co., Ltd.) to obtain Ex. 11 compound as a yellow solid (yield amount: 273 mg, yield rate: 34.7%).

The analysis results of Ex. 11 compound are shown below.

$^1$H NMR (500 MHz, $CDCl_3$, TMS, $\delta$): 0.74-0.83 (m, 12H), 1.10-1.32 (m, 24H) , 1.36-1.43 (m, 4H), 1.50-1.60(m, 4H), 2.2-2.32 (m, 2H), 2.56-2.62 (m, 2H), 2.65-2.71 (m, 2H), 6.03-6.08 (m, 4H), 6.56 (d, 2H, J=9.0 Hz), 7.33 (s, 2H), 7.36-7.41 (m, 4H), 7.48 (s, 2H), 8.28 (s, 2H)
Precise mass spectrometry: (LC-TofMS): 1002.379(found), 1002.384 (calculated)
Mass spectrometry: GC-MS m/z =1003(M+), 603 (thermally decomposed product)

From the above analysis results, it was confirmed that a structure of the synthesized product did not contradict that of Ex. 11 compound.

(Example 12)

According to the following reaction formula (scheme), Ex. 12 compound was synthesized.

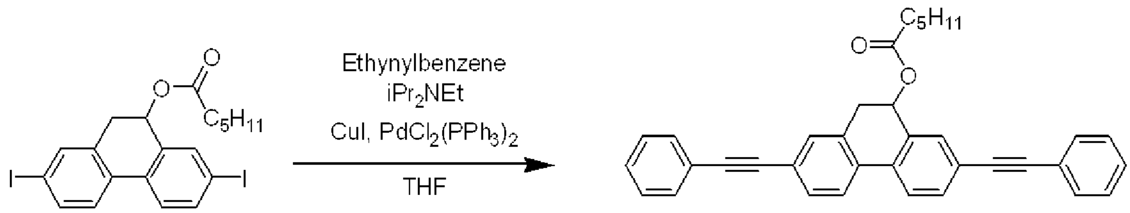


23 Ex. 12 compound

A 100 mL round-bottom flask was charged with Compound (23) (546 mg, 1.0 mmol), ethynylbenzene (224 mg, 2.2 mmol) and copper iodide (30.0 mg). THF (30 mL) and diisorpropylethylamine (2.5 mL) were added to the resultant mixture. After the flask had been purged with argon gas, $PdCl_2(PPh_3)_2$ (32.0 mg) was added to the mixture, followed by stirring at room temperature for 72 hours. Dichloromethane (100 mL) and water (100 mL) were added to the mixture to separate an organic layer. The aqueous layer was extracted with dichloromethane twice. The combined organic layer was washed sequentially with water and saturated brine and dried with sodium sulfate, followed by filtration. The filtrate was concentrated and dissolved in a minimum required amount of dichloromethane. The resultant solution was caused to pass through an alumina pad (activity II (water content: 3%)), followed by concentration again, to thereby obtain yellow oil. The obtained oil was purified using a recycle GPC (product of Japan Analytical Industry Co., Ltd.) to obtain Ex. 12 compound as a pale yellow solid (yield amount: 292 mg, yield rate: 59.0%).

The analysis results of Ex. 12 compound are shown below.

$^1$H NMR (500 MHz, $CDCl_3$, TMS, $\delta$): 0.86 (t, 3H, J =7.2 Hz), 1.21-1.30 (m, 4H) , 1.54-1.60 (m, 2H), 2.20-2.23 (m, 2H), 3.05 (d, 2H), 6.05(t, 1H), 7.2-7.95 (m, 16H)
Mass spectrometry: GC-MS m/z =495 (M+), 378(thermally decomposed product)

From the above analysis results, it was confirmed that a structure of the synthesized product did not contradict that of Ex. 12 compound.

Next, description will be given to an example of conversion of the leaving substituent-containing compound synthesized in Examples to a specific compound through elimination of the substituent.

(Example 13)

<Example of conversion through elimination of substituent>

[Conversion of leaving substituent-containing compound (7-1) to 2-iodonaphthalene through elimination of substituent]

<Synthesis of 2-iodonaphthalene>

According to the following reaction formula (scheme), 2-iodonaphthalene was synthesized.

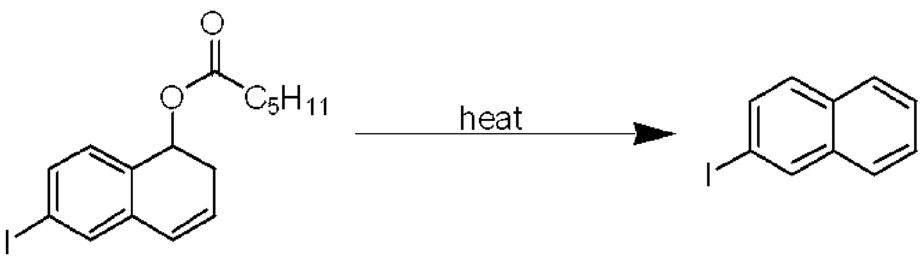


Compound (7-1) (100 mg) synthesized in Synthesis Example 1 was added to a round-bottom flask, and stirred for 1 hour with the internal temperature of the flask being maintained at 140°C. The flask was dried in vacuum at 50°C for 1 hour, and the colorless crystals remaining in the flask was scraped off (yield amount: 68.5 mg, yield rate: 99.8%).

The analysis results of the crystals are shown below.

$^1$H NMR (400 MHz, $CDCl_3$, TMS, $\delta$): 7.46-7.52(m,2H), 7.55-7.58(m,1H), 7.68-7.74(m,2H), 7.76-7.82(m,1H), 8.22-8.26(m,1H)
Elemental analysis ($C_{10}H_7I$): C,47.11; H,2.94 (found), C,47.27; H,2.78 (calculated)
Mass spectrometry: GC-MS m/z = 254 (M+)
Melting point: 50.5°C-52.0°C

From the above analysis results, it was confirmed that the colorless crystals obtained in the above reaction were 2-iodonaphthalene.

[Comparative Example 1]

[Attempt to convert leaving substituent-containing compound (7') to 2-iodonaphthalene]

The procedure of Example 13 was repeated, except that Compound (7-1) was changed to Compound (7') used in Synthesis Example 3, to thereby perform conversion to 2-iodonaphthalene through elimination of the substituent. However, through analysis of the pale yellow liquid remaining in the flask, Compound (7') was not converted to 2-iodonaphthalene through elimination of the substituent; i.e., remained unchanged.

(Example 14)

<Conversion Example 1 through elimination of substituent; [conversion of Ex. 1 leaving substituent-containing compound to Compound (24) through elimination of substituent]>

<Synthesis of Organic Semiconductor Compound [Compound (24)]>

According to the following reaction formula (scheme), Compound (24) was synthesized.

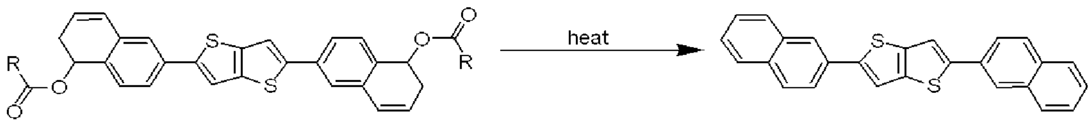


Ex. 1 compound R:n-pentyl 24

Ex. 1 compound (50 mg, 0.159 mmol) synthesized in Example 1 was added to a round-bottom flask and stirred under heating for 1 hour in an argon atmosphere at 140°C (internal temperature of the flask), to thereby obtain a bright yellow solid. The obtained solid was washed sequentially with toluene and methanol, followed by drying under vacuum, to thereby obtain Compound (24) as yellow crystals (yield amount: 30.2 mg, yield rate: 96.1%).

The analysis results of Compound (24) are shown below.

Elemental analysis ($C_{26}H_{16}S_2$): C, 79.54; H, 4.00; S, 16.20 (found) C, 79.55; H, 4.11; S, 16.34 (calculated)
Precise mass spectrometry: LC-MS (m/z) = 392.068 (found), 392.069 (calculated)
Melting point: 357.7°C

From the above analysis results, it was confirmed that a structure of the synthesized product did not contradict that of Compound (24).

(Example 15)

<Conversion Example 2 through elimination of substituent; [conversion of Ex. 2 leaving substituent-containing compound to Compound (24) through elimination of substituent]>

<Synthesis of Organic semiconductor Compound [Compound (24)]>

The procedure of Example 14 was repeated, except that Ex. 1 compound was changed to Ex. 2 compound and that the heating temperature was changed from 140°C to 130°C, to thereby obtain a product of interest (yield rate: 97.0%).

The analysis results of the obtained yellow crystals are shown below.

Elemental analysis ($C_{26}H_{16}S_2$): C, 79.50; H, 4.01; S, 16.23 (found), C, 79.55; H, 4.11; S, 16.34 (calculated)
Precise mass spectrometry: LC-MS (m/z) = 392.066 (found), 392.069 (calculated)
Melting point: 357.9°C

From the above analysis results, it was confirmed that a structure of the synthesized product did not contradict that of Compound (24).

(Example 16)

<Conversion Example 3 through elimination of substituent; [conversion of Ex. 3 leaving substituent-containing compound to Compound (24) through elimination of substituent>

The procedure of Example 14 was repeated, except that Ex. 1 compound was changed to Ex. 3 compound, that the heating temperature was changed from 140°C to 180°C, and that the heating time was changed from 1 hour to 4 hours, to thereby obtain a product of interest (yield rate: 95.5%).

The analysis results of the obtained yellow crystals are shown below.

Elemental analysis ($C_{26}H_{16}S_2$): C, 79.50; H, 4.05; S, 16.24 (found), C, 79.55; H, 4.11; S, 16.34 (calculated)
Precise mass spectrometry: LC-MS (m/z) = 392.072 (found), 392.069 (calculated)
Melting point: 358.3°C

From the above analysis results, it was confirmed that a structure of the synthesized product did not contradict that of Compound (24).

(Example 17)

<Conversion Example 4 through elimination of substituent; [conversion of Ex. 4 leaving substituent-containing compound to Compound (24) through elimination of substituent]>

The procedure of Example 14 was repeated, except that Ex. 1 compound was changed to Ex. 4 compound, to thereby obtain a product of interest (yield rate: 97.3%).

The analysis results of the obtained yellow crystals are shown below.

Elemental analysis ($C_{26}H_{16}S_2$): C, 79.51; H, 4.04; S, 16.30 (found), C, 79.55; H, 4.11; S, 16.34 (calculated)
Precise mass spectrometry: LC-MS (m/z) = 392.075 (found), 392.069 (calculated)
Melting point: 358.0°C

From the above analysis results, it was confirmed that a structure of the synthesized product did not contradict that of Compound (24).

(Example 18)

<Conversion Example 5 through elimination of substituent; [conversion of Ex. 5 leaving substituent-containing compound to Compound (24) through elimination of substituent]>

The procedure of Example 14 was repeated, except that Ex. 1 compound was changed to Ex. 5 compound, that the heating temperature was changed from 140°C to 180°C, and that the heating time was changed from 1 hour to 4 hours, to thereby obtain a product of interest (yield rate: 95.0%).

The analysis results of the obtained yellow crystals are shown below.

Elemental analysis ($C_{26}H_{16}S_2$): C, 79.51; H, 4.05; S, 16.28 (found), C, 79.55; H, 4.11; S, 16.34 (calculated)
Precise mass spectrometry: LC-MS (m/z) = 392.073 (found), 392.069 (calculated)
Melting point: 357.9°C

From the above analysis results, it was confirmed that a structure of the synthesized product did not contradict that of Compound (24).

[Comparative Example 2]

<Comparative Conversion Example 1 through elimination of substituent; [conversion of Comp. Ex. 1 leaving substituent-containing compound to Compound (24) through elimination of substituent]>

The procedure of Example 12 was repeated, except that Ex. 1 compound was changed to Comp. Ex. 1 compound, to thereby obtain a pale yellow solid instead of the yellow crystals. The obtained solid was washed sequentially with toluene and methanol. As a result, the solid was completely dissolved therein, so that crystals of interest were not

obtained. The resultant solution was concentrated to obtain a solid, which was then analyzed. From the obtained values, it was confirmed that the solid was unconverted Comp. Ex. 1 compound.

Next, the results obtained in Examples 13 to 18 and Comparative Examples 1 and 2 will be discussed.

The leaving substituent-containing compound could be converted to a sparingly-soluble organic semiconductor compound with high purity and high yield (about 95% or higher) through heating (application of energy: external stimulus) at a temperature lower than in the conventional leaving substituent-containing compounds (i.e., by about 100°C between the present compound and the comparative compound having the same skeleton).

It was indicated that the method was an effective method not only for the production of a low molecular weight compound such as naphthalene, but also for production of a normally sparingly-soluble $\pi$-electron conjugated compound, especially an organic semiconductor compound. This can be used in many molecules, such as organic pigments, in addition to the organic semiconductors.

Comparing Example 13 with Comparative Example 1 and Example 14 with Comparative Example 2, each using the compound having the same leaving substituent and skeleton, it was found that combination of the leaving group and the skeleton (cyclohexadiene skeleton) of the leaving substituent-containing compound contributes to lowering conversion temperature. Other silyl or alkyl ether groups, which conventionally required heating at 250°C to 300°C or higher for elimination thereof, were eliminated at 200°C or lower when incorporated to the main skeleton.

(Example 19)

<Conversion Example 6 through elimination of substituent; [conversion of Ex. 6 leaving substituent-containing compound to Compound (25) through elimination of substituent]>

<Synthesis of organic semiconductor compound [Compound (25)]>

According to the following reaction formula (scheme), Compound (25) was synthesized.

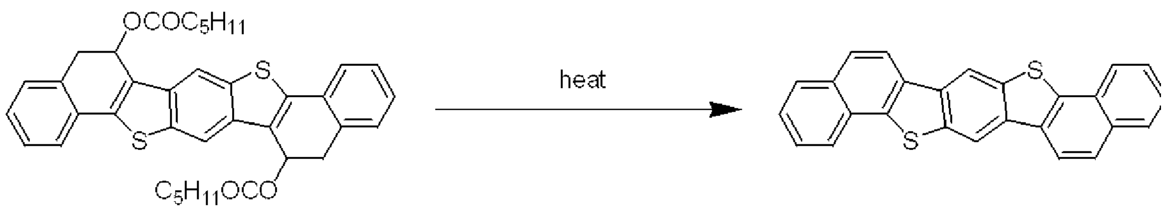


Ex. 6 compound 25

The procedure of Example 14 was repeated, except that Ex. 1 compound was changed to Ex. 6 compound, that the heating temperature was changed from 140°C to 150°C, and that the heating time was changed from 1 hour to 2 hours. The analysis results of the obtained orange crystals are shown below.

Elemental analysis ($C_{26}H_{14}S_2$): C, 79.50; H, 4.01; S, 16.23 (found), C, 79.55; H, 4.11; S, 16.34 (calculated)

Precise mass spectrometry: LC-MS (m/z) = 390.060 (found), 390.069 (calculated)

From the above analysis results, it was confirmed that a structure of the synthesized product did not contradict that of Compound (27).

(Example 20)

<Conversion Example 7 through elimination of substituent; [conversion of Ex. 7 leaving substituent-containing compound to Compound (26) through elimination of substituent]>

<Synthesis of organic semiconductor compound [Compound (26)]>

According to the following reaction formula (scheme), Compound (26) was synthesized.

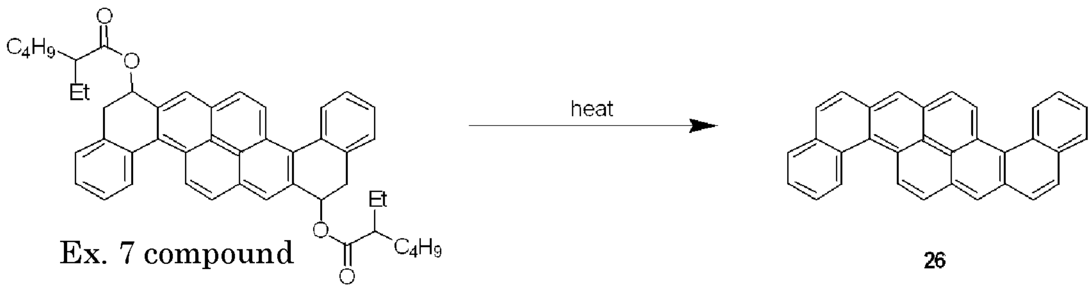


Ex. 7 compound

26

The procedure of Example 14 was repeated, except that Ex. 1 compound was changed to Ex. 7 compound, that the heating temperature was changed from 140°C to 150°C, and that the heating time was changed from 1 hour to 2 hours, to thereby obtain a product of interest (yield rate: 94.9%).

The analysis results of the obtained crystals are shown below.

Elemental analysis ($C_{32}H_{18}$): C, 95.25; H, 4.71 (found), C, 95.49; H, 4.51 (calculated)

Precise mass spectrometry: LC-MS (m/z) = 402.149 (found), 402.141 (calculated)

From the above analysis results, it was confirmed that a structure of the synthesized product did not contradict that of Compound (26).

(Example 21)

<Conversion Example 8 through elimination of substituent; [conversion of Ex. 8 leaving substituent-containing compound to Compound (27) through elimination of substituent]>

<Synthesis of organic semiconductor compound [Compound (27)]>

According to the following reaction formula (scheme), Compound (27) was synthesized.

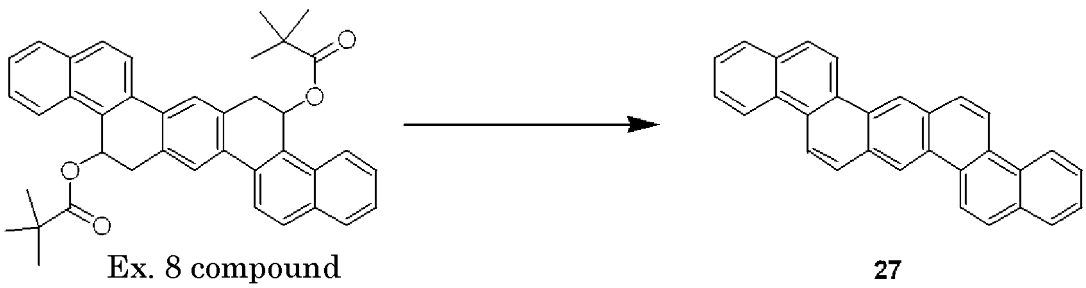

Ex. 8 compound

27

The procedure of Example 14 was repeated, except that Ex. 1 compound was changed to Ex. 8 compound, that the heating temperature was changed from 140°C to 150°C, and that the heating time was changed from 1 hour to 3 hours, to thereby obtain a product of interest (yield rate: 95.3%).

The analysis results of the obtained crystals are shown below.

Elemental analysis ($C_{30}H_{18}$): C, 95.11; H, 4.88 (found), C, 95.21; H, 4.79 (calculated)

Precise mass spectrometry: LC-MS (m/z) = 378.136 (found), 378.141 (calculated)

From the above analysis results, it was confirmed that a structure of the synthesized product did not contradict that of Compound (27).

(Example 22)

<Conversion Example 9 through elimination of substituent; [conversion of Ex. 9 leaving substituent-containing compound to Compound (28) through elimination of substituent]>

<Synthesis of organic semiconductor compound [Compound (28)]>

According to the following reaction formula (scheme), compound (28) was synthesized.

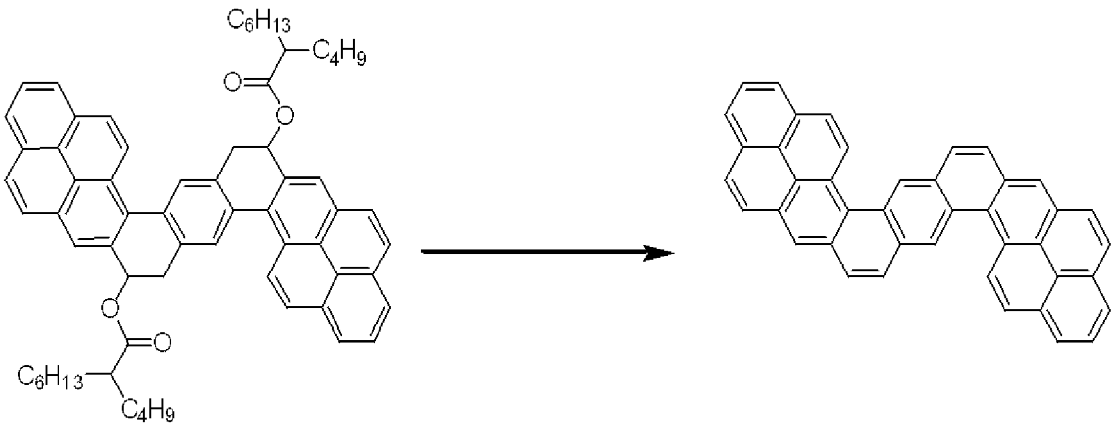

Ex. 9 compound 28

The procedure of Example 14 was repeated, except that Ex. 1 compound was changed to Ex. 9 compound, that the heating temperature was changed from 140°C to 150°C, and that the heating time was changed from 1 hour to 3 hours, to thereby obtain a product of interest (yield rate: 96.3%).

The analysis results of the obtained crystals are shown below.

Elemental analysis ($C_{42}H_{22}$): C, 95.70; H, 4.18 (found), C, 95.79; H, 4.21 (calculated)

Precise mass spectrometry: LC-MS (m/z) = 526.166 (found), 526.172 (calculated)

From the above analysis results, it was confirmed that a structure of the synthesized product did not contradict that of Compound (28).

(Example 23)

<Conversion Example 10 through elimination of substituent; [conversion of Ex. 10 leaving substituent-containing compound to Compound (29) through elimination of substituent]>

<Synthesis of organic semiconductor compound [Compound (29)]>

According to the following reaction formula (scheme), Compound (29) was synthesized.

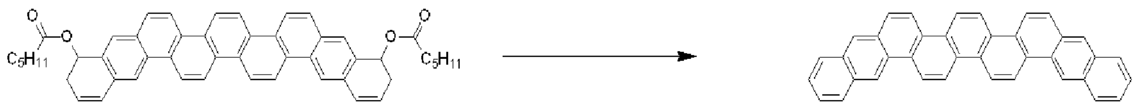

Ex. 10 compound 29

The procedure of Example 14 was repeated, except that Ex. 1 compound was changed to Ex. 10 compound, that the heating temperature was changed from 140°C to 150°C, and that the heating time was changed from 1 hour to 2 hours, to thereby obtain a product of interest (yield rate: 93.3%).

The analysis results of the obtained crystals are shown below.

Elemental analysis ($C_{38}H_{22}$): C, 95.25; H, 4.71 (found), C, 95.37; H, 4.63 (calculated)
Precise mass spectrometry: LC-MS (m/z) = 478.165 (found), 478.172 (calculated)

From the above analysis results, it was confirmed that a structure of the synthesized product did not contradict that of Compound (29).

(Example 24)

<Conversion Example 11 through elimination of substituent; [conversion of Ex. 11 leaving substituent-containing compound to Compound (30) through elimination of substituent]>

<Synthesis of organic semiconductor compound [Compound (30)]>

According to the following reaction formula (scheme), Compound (30) was synthesized.

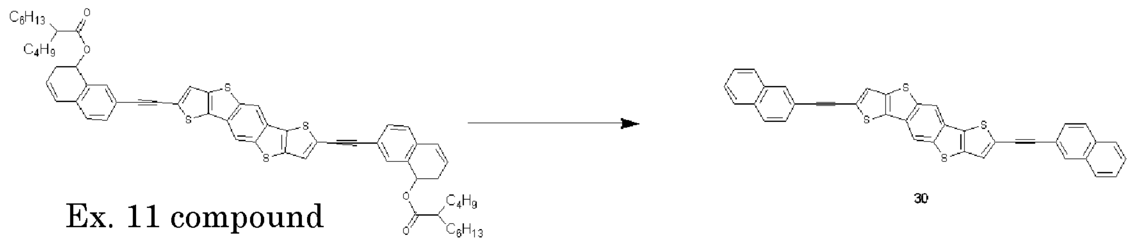

Ex. 11 compound

30

The procedure of Example 14 was repeated, except that Ex. 1 compound was changed to Ex. 11 compound, that the heating temperature was changed from 140°C to 160°C, and that the heating time was changed from 1 hour to 4 hours, to thereby obtain a product of interest (yield rate: 93.3%).
The analysis results of the obtained crystals are shown below.
Elemental analysis ($C_{38}H_{18}S_4$): C, 75.79; H, 3.11; S, 21.07 (found), C, 75.71; H, 3.01; S, 21.28 (calculated)
Precise mass spectrometry: LC-MS (m/z) = 602.023 (found), 602.029 (calculated)
From the above analysis results, it was confirmed that a structure of the synthesized product did not contradict that of Compound (30).

(Example 25)

<Conversion Example 12 through elimination of substituent; [conversion of Ex. 12 leaving substituent-containing compound to Compound (31) through elimination of substituent]>

<Synthesis of organic semiconductor compound [Compound (31)]>

According to the following reaction formula (scheme), Compound (31) was synthesized.

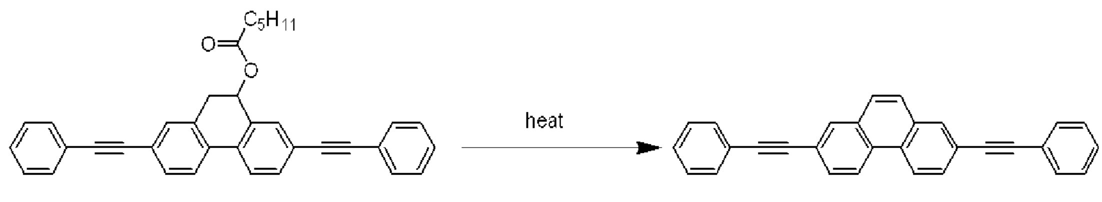


Ex. 12 compound

31

The procedure of Example 14 was repeated, except that Ex. 1 compound was changed to Ex. 12 compound, that the heating temperature was changed from 140°C to 160°C, and that the heating time was changed from 1 hour to 2 hours, to thereby obtain a product of interest (yield rate: 95.4%).
The analysis results of the obtained crystals are shown below.

Elemental analysis ($C_{38}H_{18}S_4$): C, 95.16; H, 4.60 (found), C, 95.21; H, 4.79 (calculated)
Precise mass spectrometry: LC-MS (m/z) = 378.148 (found), 378.141 (calculated)

From the above analysis results, it was confirmed that a structure of the synthesized product did not contradict that of Compound (31).
The results obtained in Examples 19 and 25 will be discussed.
The leaving substituent-containing compounds were converted with high yield to the specific compounds having

26 carbon atoms linked together via a covalent bond (as shown in Examples 13 and 14) as well as the ring-fused specific compounds having up to 42 carbon atoms, by heating the leaving substituent-containing compounds at about 150°C to 200°C.

(Example 26)

<Formation of Organic semiconductor precursor into ink (evaluation of dissolvability)>

Each of the compounds obtained in Examples 1, 3, 5, 6, 7, 8, 9, 10, 11 and 12 and the corresponding specific compounds (24) to (33) was added to toluene, THF, anisole or chloroform (each: 2.0 mg) until insoluble matter was found. The resultant mixture was stirred for 10 min under reflux of the solvent and cooled to room temperature, followed by stirring for 1 hour. After the mixture had been left to stand still for 16 hours, the supernatant was filtrated with a 0.2 μm PTFE filter to obtain a saturated solution. The obtained solution was dried under reduced pressure to calculate the dissolvability of the compound to each solvent. The results are shown in Table 1.

The evaluation criteria in Table 1 are as follows.

A: 0.5% by mass ≤ Dissolvability
B: 0.1% by mass ≤ Dissolvability < 0.5% by mass
C: 0.005% by mass ≤ Dissolvability < 0.1% by mass
D: Dissolvability < 0.005% by mass

Table 1

| Compound | Solvent | | |
|---|---|---|---|
| | THF | Toluene | Anisole |
| Ex. 1 compound | A | A | A |
| Ex. 3 compound | A | A | B |
| Ex. 5 compound | A | A | B |
| Ex. 6 compound | A | B | B |
| Ex. 7 compound | A | B | B |
| Ex. 8 compound | A | A | A |
| Ex. 9 compound | A | B | B |
| Ex. 10 compound | A | B | B |
| Ex. 11 compound | A | A | A |
| Ex. 12 compound | A | A | A |
| Compound (24) | D | D | D |
| Compound (25) | D | D | D |
| Compound (26) | D | D | D |
| Compound (27) | D | D | D |
| Compound (28) | D | D | D |
| Compound (29) | D | D | D |
| Compound (30) | D | D | D |
| Compound (31) | B | C | C |

As is clear from Table 1, the leaving substituent-containing compounds were found to have dissolvability of about 0.1% by mass or higher to different solvents with different polarities. This dissolvability is comparable to that of the conventional compound having a cyclohexene skeleton and two leaving groups introduced thereto, indicating that various solvents can be used for the present compound in coating processes.

The present compounds have such a high dissolvability, and thus are applicable to various film forming/printing methods such as inkjet coating, spin coating, solution casting, dip coating, screen printing and gravure printing.

Meanwhile, Compounds (24) to (31) obtained after conversion were found to have dissolvability of 0.005% by mass or lower to all of these solvents, indicating that the solubility-imparting groups of the leaving substituent-containing compound exhibited great contribution. In other wards, the compounds obtained after conversion through elimination reaction exhibited insolubility. Also, when the molecular size is not large like Compound (33), dissolvability is exhibited even after elimination of the substituent.

(Example 27)

<Observation of Elimination Behavior of Ex. 1 Compound>

Ex. 1 compound synthesized in Example 1 was heated at a range of 25°C to 500°C at a temperature increasing rate of 5 °C/min and the pyrolysis behavior thereof was observed by TG-DTA [reference: $Al_2O_3$, under nitrogen flow (200 mL/min), EXSTAR6000 (product name), product of Seiko Instruments Inc.].

Also, Ex. 1 compound was heated at a range of 25°C to 500°C at a temperature increasing rate of 5 °C/min and the phase transition behavior thereof was observed by DSC [reference: $Al_2O_3$, under nitrogen flow (200 mL/min), EXSTAR6000 (product name), product of Seiko Instruments Inc.].

The results are shown in Fig. 4, where the horizontal axis indicates temperature [°C], the left-hand vertical axis indicates change in mass [mg] and the right-hand vertical axis indicates heat flow [mW].

In TG-DTA, 36.4% of mass reduction was observed from 120°C to 225°C. The mass reduced coincided substantially with the mass of 2 molecules of caproic acid (theoretical value: 37.1%). It was confirmed that there was a melting point of 357.4°C. This was identified with the melting point of Compound (24).

From the above results, Ex. 1 compound was found to be converted to Compound (24) by heating.

(Example 28)

< Observation of Elimination Behavior of Ex. 2 Compound>

The procedure of Example 27 was repeated, except that Ex. 1 compound was changed to Ex. 2 compound, to thereby observe pyrolysis behavior and phase transition behavior. The results are shown in Fig. 5, where the horizontal axis indicates temperature [°C], the left-hand vertical axis indicates change in mass [mg] and the right-hand vertical axis indicates heat flow [mW].

In TG-DTA, 21.9% of mass reduction was observed from 115°C to 200°C. The mass reduced coincided substantially with the mass of 1 molecule of caproic acid (theoretical value: 22.7%). It was confirmed that there was a melting point of 357.9°C. This was identified with the melting point of Compound (24).

From the above results, Ex. 2 compound was found to be converted to Compound (24) by heating.

In the leaving substituent-containing compound (organic semiconductor precursor), the leaving group was removed at a temperature lower than in the conventional compound [e.g., Comp. Ex. compound 1] by 100°C or higher, and the molecules were crystallized (see the DSC exothermic peak). Also, the temperature for elimination and the temperature for completion of weight reduction were both lower in the organic semiconductor precursor having one soluble group (one leaving substituent) than in the organic semiconductor precursor having two soluble groups (two leaving substituents).

(Example 29)

<Production Example of Thin Film>

Each (5 mg) of Ex. 1 compound and Ex. 2 compound synthesized in Synthesis Examples 1 and 2 was dissolved in THF, so that the concentration of the compound became 0.1% by mass, and the mixture was filtrated using a 0.2 μm-filter to prepare a solution. Onto a N-type silicon substrate having a 300 nm-thick thermally-oxidized film, which had been soaked in concentrated sulfuric acid for 24 hours, 100 μL of the prepared solution was added dropwise with a pipette, and a 5 pL liquid droplet of the prepared solution was jetted 50 times using an inkjet apparatus (head; product of Ricoh Printing Systems, Ltd.). The thus-treated substrate was covered with a petri dish and left to stand still until the solvent was evaporated to thereby form a thin film. The thin film was observed with a polarization microscope and a scanning probe microscope (NANOPICS (product name), product of Seiko Instruments Inc., contact mode), and it was confirmed that a smooth and continuous amorphous film was obtained. Next, the thin film was subjected to annealing for 30 min at 150°C in an argon atmosphere. Then, the thin film was observed in the same manner as described above.

After annealing, a plurality of colored domains were observed under the polarization microscope, and it was confirmed that a smooth and crystalline film was obtained. The polarization microscope images of these films are shown in Figs. 6A and 6B. These films were obtained because each of Ex. 1 compound and Ex. 2 compound serving as precursors lost an ester group (i.e., a soluble group) and converted to Compound 24 having stronger intermolecular interaction, and became crystalline in the film. The thin film was insoluble in chloroform, THF, toluene, etc. at 25°C.

(Comparative Example 3)

The procedure of Example 29 was repeated, except that Ex. 1 compound or Ex. 2 compound was changed to Compound (24) obtained after conversion of Ex. 1 compound or Ex. 2 compound, and that THF was changed to orthodichlorobenzene heated at 150°C. In the film, crystals were precipitated in such a degree that it could be recognized by visual observation, and it was confirmed that uncontinuous film was formed. Under the polarization microscope, a plurality of uncontinuous colored domains were observed.
Under the scanning probe microscope, a surface roughness of 100 μm or more was confirmed.
These results indicate that the production method of the present invention is effective on thin film formation using compounds that are sparingly-soluble in some solvents each having a high boiling point to easily form precipitates.

(Example 30)

[Production and Evaluation of Organic Thin-film transistor through Wet Process using Solution]

A thin film containing Ex. 1 compound was formed in the same manner as in Example 29. The thin film was subjected to annealing for 60 min at 150°C in an argon atmosphere, so as to be converted to a 50 nm-thick thin film formed of Compound (24) (i.e., an organic semiconductor).
On the thin film, gold was vacuum deposited via a shadow mask under the condition of back pressure of up to $10^{-4}$ Pa, deposition rate of 1 angstrome/s to 2 angstromes/s and film thickness of 50 nm, thereby forming source and drain electrodes having a channel length of 50 μm and channel width of 2 mm. Thus, a field-effect transistor (FET) element having a structure shown in Fig. 1D was produced. The organic semiconductor layer and silicon oxide film in a region other than the gold electrode were scraped off, and a conductive paste (product of Fujikura Kasei Co., Ltd.) was applied in the region and the solvent was dried. Through the region, voltage was applied to the silicon substrate serving as the gate electrode.
The electrical property of the FET element was evaluated by a semiconductor parameter analyzer B1500A (product of Agilent Technologies) under the measurement conditions of fixed source drain voltage: -100 V, and gate voltage sweep: from -20 V to +100 V). The FET element exhibited a property as a p-type transistor element. The current and voltage (I-V) characteristics of this FET element is shown in FIG. 7.
In FIG. 7, white circles correspond to absolute values of drain current on the left-hand vertical axis, and black circles correspond to square roots of the absolute values of drain current on the right-hand vertical axis. The horizontal axis indicates the applied gate electrode.
From the saturation region of the current and voltage (I-V) characteristics of the organic thin-film transistor, a field-effect mobility was obtained.
The field-effect mobility of the organic thin-film transistor was calculated by the following equation (1).

$$Ids = \mu CinW(Vg-Vth)2/2L \cdot \cdot \cdot (1)$$

where Cin denotes a capacitance per unit area of a gate insulating film, W denotes a channel width, L denotes a channel length, Vg denotes a gate voltage, Ids denotes a source-drain current, μ denotes a mobility and Vth denotes a gate threshold voltage at which a channel begins to be formed.
Moreover, a ratio of an on-current at a gate voltage of 40 V to an off-current at a gate voltage of 0 V was obtained as a current on/off ratio. The results are shown in Table 2.

(Example 31)

The procedure of Example 30 was repeated, except that Ex. 1 compound was changed to Ex. 2 compound, which was converted to a thin film formed of Compound (24) (organic semiconductor), to thereby form and evaluate a FET. The results are shown in Table 2.

(Comparative Example 4)

The orthodichlorobenzene solution described in Comparative Example 3 was used to form a thin film of Compound (24) (organic semiconductor) on the same substrate as in Example 29. The obtained FET element was evaluated for characteristics. The results are shown in Table 2.

(Comparative Example 5)

The procedure of Example 29 was repeated, except that Ex. 1 compound was changed to Comp. Ex. 1 compound, and that annealing [treatment for conversion to a thin film of Compound (24) (organic semiconductor)] was performed to form a thin film. The obtained FET element was evaluated for characteristics. The results are shown in Table 2.

Table 2

| | Mobility ($cm^2/Vs$) | On/off ratio ($Id_{100v}/Id_{0v}$) |
|---|---|---|
| Ex. 30 | $3.8 \times 10^{-2}$ | $3.0 \times 10^{7}$ |
| Ex. 31 | $4.3 \times 10^{-2}$ | $2.0 \times 10^{7}$ |
| Comp. Ex. 4 | Not operated | Not operated |
| Comp. Ex. 5 | Not operated | Not operated |

(Example 32)

The procedure of Example 30 was repeated, except that Ex. 1 compound was changed to Ex. 6 compound, to thereby form a thin film and a transistor. The transistor containing this active layer was found to exhibit excellent p-type transistor characteristics similar to Examples 30 and 31.

(Example 33)

The procedure of Example 30 was repeated, except that Ex. 1 compound was changed to Ex. 7 compound, to thereby form a thin film and a transistor. The transistor containing this active layer was found to exhibit excellent p-type transistor characteristics similar to Examples 30 and 31.

(Example 34)

The procedure of Example 30 was repeated, except that Ex. 1 compound was changed to Ex. 8 compound, to thereby form a thin film and a transistor. The transistor containing this active layer was found to exhibit excellent p-type transistor characteristics similar to Examples 30 and 31.

(Example 35)

The procedure of Example 30 was repeated, except that Ex. 1 compound was changed to Ex. 9 compound, to thereby form a thin film and a transistor. The transistor containing this active layer was found to exhibit excellent p-type transistor characteristics similar to Examples 30 and 31.

(Example 36)

The procedure of Example 30 was repeated, except that Ex. 1 compound was changed to Ex. 10 compound, to thereby form a thin film and a transistor. The transistor containing this active layer was found to exhibit excellent p-type transistor characteristics similar to Examples 30 and 31.

(Example 37)

The procedure of Example 30 was repeated, except that Ex. 1 compound was changed to Ex. 11 compound, to thereby form a thin film and a transistor. The transistor containing this active layer was found to exhibit excellent p-type transistor characteristics similar to Examples 30 and 31.

(Example 38)

The procedure of Example 30 was repeated, except that Ex. 1 compound was changed to Ex. 12 compound, to thereby form a thin film and a transistor. The transistor containing this active layer was found to exhibit excellent p-type transistor characteristics similar to Examples 30 and 31.

As is clear from the evaluation results of characteristics shown in Table 2, when a sparingly-soluble organic semiconductor compound [Compound (24)] was dissolved in a solvent having a high boiling point and the resultant solution was used for film formation, good FET characteristics could not be attained (Comparative Example 4); and also when a precursor film containing the conventional compound [Comp. Ex. 1 compound] was converted at about 150°C, good FET characteristics could not be attained, likely because the precursor film was not sufficiently converted (Comparative Example 5).

Meanwhile, when the leaving substituent-containing compound was used as an organic semiconductor precursor, which converted to a film containing an organic semiconductor compound through wet process using a solution and treatment at a relatively low temperature of about 150°C, good FET characteristics could be attained (Examples 29 to 37).

That is, the organic thin-film transistor exhibited excellent hole mobility, current on/off ratio, and had excellent characteristics as an organic thin-film transistor. Therefore, the leaving substituent-containing compound, and the organic semiconductor compound obtained by using the leaving substituent-containing compound are useful for production of an organic electronic device, such as an organic thin-film transistor.

The leaving substituent-containing compound is excellent in solubility in various organic solvents, and can synthesize a specific compound (e.g., an organic semiconductor compound) with high yield without generating olefin end-groups by elimination reaction occurred by application of energy (external stimulus such as heat) in a dose lower than in the conventional compound [e.g., Comp. Ex. 1 compound], thereby providing excellent processability.

Since an organic semiconductor compound is a sparingly soluble, it is conventionally difficult to form a film. However, the leaving substituent-containing compound is used for film formation as an organic semiconductor compound precursor, followed by converting to an organic semiconductor compound with, for example, heat, thereby easily obtaining a continuous organic semiconductor film. Thus, the thus-formed film may be applied to organic electronic devices, particularly, applied to electronic devices such as semiconductors, and optical electronic devices such as EL light-emitting elements, electronic paper, various sensors, and radio frequency identification (RFID).

Reference Signs List

1 Organic semiconductor layer
2 Source electrode
3 Drain electrode
4 Gate electrode
5 Insulating film
6 Source electrode
7 Organic semiconductor
8 Drain electrode
9 Interlayer insulating layer
10 Pixel electrode
11 Substrate
12 Gate electrode
13 Gate insulating film
14 Through hole
15 Scanning line/gate electrode
16 Organic semiconductor
17 Source electrode
18 Drain electrode

## Claims

**1.** A method for producing a film-like product, comprising:

forming a coating film on a substrate by coating the substrate with a coating liquid containing in a solvent a π-electron conjugated compound precursor represented by A-(B)m, and
eliminating an eliminated component represented by General Formula (II) to form a π-electron conjugated compound represented by A-(C)m in the coating film,

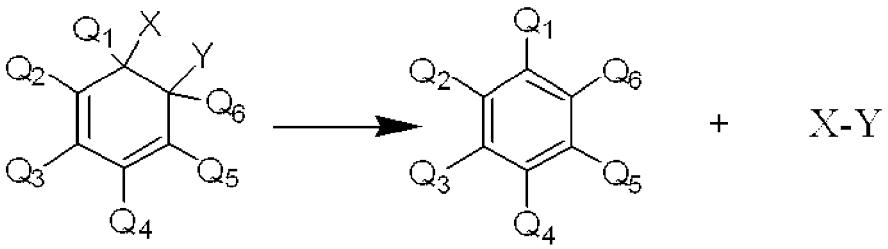

(I) (Ia) (II)

wherein in A-(B)m and A-(C)m, A represents a π-electron conjugated substituent, B represents a solvent-soluble substituent containing a structure represented by General Formula (I) as at least a partial structure, and m is a natural number,
wherein the solvent-soluble substituent represented by B is linked via a covalent bond with the π-electron conjugated substituent represented by A where the covalent bond is formed between an atom present on $Q_1$ to $Q_6$ and an atom present on the π -electron conjugated substituent represented by A or the solvent-soluble substituent represented by B is ring-fused with the π-electron conjugated substituent represented by A via atoms present on the π-electron conjugated substituent represented by A, and C represents a substituent containing a structure represented by General Formula (Ia) as at least a partial structure, and
wherein in General Formulas (I), (Ia) and (II), X and Y each represent a hydrogen atom, a substituted or unsubstituted ether group having 1 to 38 carbon atoms or a substituted or unsubstituted acyloxy group having 1 to 38 carbon atoms, where one of X and Y is the ether or acyloxy group and the other is the hydrogen atom; $Q_2$ to $Q_5$ each represent a hydrogen atom or a monovalent organic group; $Q_1$ and $Q_6$ each represent a hydrogen atom, a halogen atom, an alkyl group, an alkenyl group, an alkynyl group, an aryl group or a heteroaryl group; and among the monovalent organic groups represented by $Q_1$ to $Q_6$, adjacent monovalent organic groups may be linked together to form a ring, and wherein the monovalent organic group is any one of an alkyl group, an alkenyl group, an alkynyl group, an aryl group and a heteroaryl group, wherein the heteroaryl group is selected from a thiophene ring, a pyridine ring, a pyrazine ring, a pyrimidine ring, a triazine ring, a pyrrol ring, a pyrazole ring, an imidaziole ring, a triazole ring, an oxazole ring, a thiazole ring and a furan ring.

2. The method for producing a film-like product according to claim 1, wherein the substrate is coated with the coating liquid by a method selected from the group consisting of inkjet coating, spin coating, solution casting and dip coating.

3. The method for producing a film-like product according to claim 1 or claim 2, wherein the substituent represented by A is at least one π-electron conjugated compound selected from the group consisting of (i) compounds in which one or more aromatic hydrocarbon rings are ring-fused with one or more aromatic heterocyclic rings, compounds in which two or more aromatic hydrocarbon rings are ring-fused together, and compounds in which two or more aromatic heterocyclic rings are ring-fused together; and (ii) compounds in which one or more aromatic hydrocarbon rings are linked via a covalent bond with one or more aromatic heterocyclic rings, compounds in which two or more aromatic hydrocarbon rings are linked together via a covalent bond, and compounds in which two or more aromatic heterocyclic rings are linked together via a covalent bond.

4. The method for producing a film-like product according to any one of claims 1 to 3, wherein the eliminated component represented by General Formula (II) and eliminated from the compound represented by A-(B)m includes a hydrogen halide, a substituted or unsubstituted carboxylic acid, a substituted or unsubstituted alcohol or carbon dioxide.

5. The method for producing a film-like product according to any one of claims 1 to 4, wherein the compound represented by A-(B)m has a solvent solubility, and the compound represented by A-(C)m and formed after elimination of the leaving substituent has a solvent insolubility.

6. The method for producing a film-like product according to any one of claims 1 to 5, wherein the leaving substituent-containing compound represented by General Formula (I) does not have the structure I-(11)

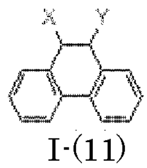

I-(11)

.

## Patentansprüche

**1.** Verfahren zur Herstellung eines filmähnlichen Produkts, Folgendes umfassend:

Bilden eines Beschichtungsfilms an einem Substrat durch Beschichten des Substrates mit einer Beschichtungsflüssigkeit, welche in einem Lösemittel eine $\pi$-Elektronen-konjugierte Vorläuferverbindung enthält, dargestellt durch A-(B)m, und

Beseitigen einer beseitigten Komponente, dargestellt durch die Allgemeine Formel (II), zum Bilden einer $\pi$-Elektronen-konjugierten Verbindung, dargestellt durch A-(C)m, in dem Beschichtungsfilm,

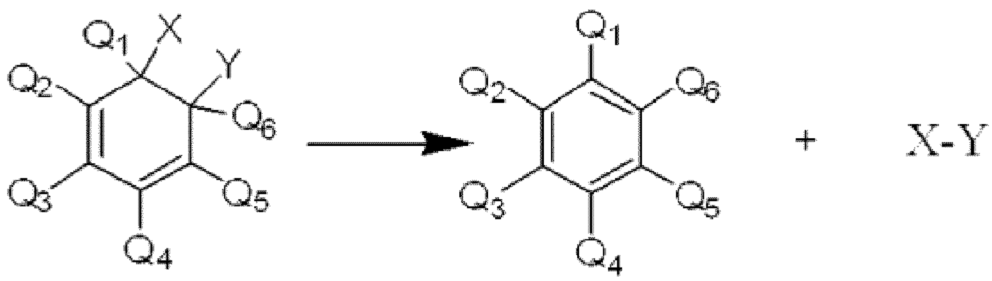

(I) (Ia) (II)

wobei in A-(B)m und A-(C)m, A einen $\pi$-Elektronen-konjugierten Substituenten darstellt, B einen lösemittellöslichen Substituenten darstellt, welcher eine Struktur, dargestellt durch die Allgemeine Formel (I), als mindestens eine Teilstruktur enthält, und m eine natürliche Zahl ist,

wobei der durch B dargestellte lösemittellösliche Substituent über eine kovalente Bindung mit dem durch A dargestellten $\pi$-Elektronen-konjugierten Substituenten verbunden ist, worin die kovalente Bindung zwischen einem an $Q_1$ bis $Q_6$ vorhandenen Atom und einem an dem durch A dargestellten $\pi$-Elektronen-konjugierten Substituenten vorhandenen Atom gebildet ist, oder der durch B dargestellte lösemittellösliche Substituent mit dem durch A dargestellten $\pi$-Elektronen-konjugierten Substituenten durch Atome ringkondensiert ist, welche an dem durch A dargestellten $\pi$-Elektronen-konjugierten Substituenten vorhanden sind, und C einen Substituenten darstellt, welcher eine durch die Allgemeine Formel (Ia) dargestellte Struktur als mindestens eine Teilstruktur enthält, und

wobei, in den Allgemeinen Formeln (I), (Ia) und (II), X und Y jeweils ein Wasserstoffatom darstellen, eine substituierte oder unsubstituierte Ethergruppe, welche 1 bis 38 Kohlenstoffatome aufweist, oder eine substituierte oder unsubstituierte Acyloxygruppe, welche 1 bis 38 Kohlenstoffatome aufweist, worin eines von X und Y die Ether- oder die Acyloxygruppe ist und das andere das Wasserstoffatom ist; $Q_2$ bis $Q_5$ jeweils ein Wasserstoffatom oder eine einwertige organische Gruppe darstellen; $Q_1$ und $Q_6$ jeweils ein Wasserstoffatom, ein Halogenatom, eine Alkylgruppe, eine Alkenylgruppe, eine Alkynylgruppe, eine Arylgruppe oder eine Heteroarylgruppe darstellen; und unter den einwertigen organischen Gruppen, welche durch $Q_1$ bis $Q_6$ dargestellt sind, angrenzende einwertige organische Gruppen miteinander zum Bilden eines Rings verbunden sein können, und

wobei die einwertige organische Gruppe eine beliebige aus einer Alkylgruppe, einer Alkenylgruppe, einer Alkynylgruppe, einer Arylgruppe und einer Heteroarylgruppe ist, wobei die Heteroarylgruppe ausgewählt ist aus einem Thiophenring, einem Pyridinring, einem Pyrazinring, einem Pyrimidinring, einem Triazinring, einem Pyrrolring, einem Pyrazolring, einem Imidaziolring, einem Triazolring, einem Oxazolring, einem Thiazolring und einem Furanring.

2. Verfahren zur Herstellung eines filmähnlichen Produkts nach Anspruch 1, wobei das Substrat mit der Beschichtungsflüssigkeit anhand eines Verfahrens beschichtet wird, ausgewählt aus der Gruppe, bestehend aus Tintenstrahlbeschichten, Rotationsbeschichten, Lösungsgießen und Tauchbeschichten.

3. Verfahren zur Herstellung eines filmähnlichen Produkts nach Anspruch 1 oder Anspruch 2, wobei der durch A dargestellte Substituent mindestens eine π-Elektronenkonjugierte Verbindung ist, ausgewählt aus der Gruppe, bestehend aus (i) Verbindungen, in welchen ein oder mehrere aromatische Kohlenwasserstoffringe mit einem oder mehreren aromatischen heterozyklischen Ringen ringkondensiert sind, Verbindungen, in welchen zwei oder mehr aromatische Kohlenwasserstoffringe miteinander ringkondensiert sind, und Verbindungen, in welchen zwei oder mehr aromatische heterozyklische Ringe miteinander ringkondensiert sind; und (ii) Verbindungen, in welchen ein oder mehrere aromatische Kohlenwasserstoffringe über eine kovalente Bindung mit einem oder mehreren aromatischen heterozyklischen Ringen verbunden sind, Verbindungen, in welchen zwei oder mehr aromatische Kohlenwasserstoffringe miteinander über eine kovalente Bindung verbunden sind, und Verbindungen, in welchen zwei oder mehr aromatische heterozyklische Ringe miteinander über eine kovalente Bindung verbunden sind.

4. Verfahren zur Herstellung eines filmähnlichen Produkts nach einem der Ansprüche 1 bis 3, wobei die beseitigte Komponente, welche durch die Allgemeine Formel (II) dargestellt ist und aus der durch A-(B)m dargestellten Verbindung beseitigt wurde, ein Wasserstoffhalogenid, eine substituierte oder unsubstituierte Karbonsäure, einen substituierten oder unsubstituierten Alkohol oder Kohlendioxid einschließt.

5. Verfahren zur Herstellung eines filmähnlichen Produkts nach einem der Ansprüche 1 bis 4, wobei die durch A-(B)m dargestellte Verbindung eine Lösemittellöslichkeit aufweist, und die durch A-(C)m dargestellte Verbindung, welche nach Beseitigung des Abgangssubstituenten gebildet wird, eine Lösemittelunlöslichkeit aufweist.

6. Verfahren zur Herstellung eines filmähnlichen Produkts nach einem der Ansprüche 1 bis 5, wobei die Verbindung, welche durch die Allgemeine Formel (I) dargestellt ist und den Abgangssubstituenten enthält, nicht die Struktur I-(11) aufweist

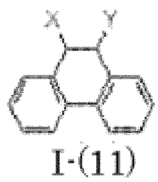

I-(11)

.

**Revendications**

1. Procédé pour produire un produit du type film, comprenant :

la formation d'un film de revêtement sur un substrat par revêtement du substrat à l'aide d'un liquide de revêtement qui contient, dans un solvant, un précurseur de composé conjugué à électrons π qui est représenté par A-(B)m ; et l'élimination d'un composant éliminé qui est représenté par la Formule Générale (II) pour former un composé conjugué à électrons π qui est représenté par A-(C)m dans le film de revêtement,

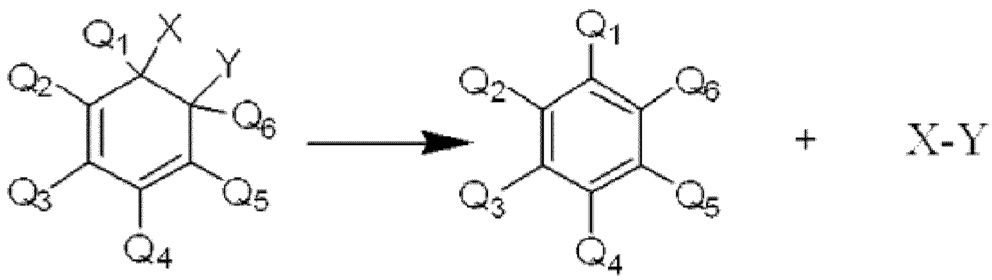

(I) (Ia) (II)

dans lequel, dans A-(B)m et A-(C)m, A représente un substituant conjugué à électrons $\pi$, B représente un substituant soluble dans un solvant qui contient une structure qui est représentée par la Formule Générale (I) en tant qu'au moins une structure partielle et m est un nombre naturel ;
dans lequel le substituant soluble dans un solvant qui est représenté par B est lié via une liaison covalente au substituant conjugué à électrons $\pi$ qui est représenté par A, où la liaison covalente est formée entre un atome qui est présent sur $Q_1$ à $Q_6$ et un atome qui est présent sur le substituant conjugué à électrons $\pi$ qui est représenté par A, ou le substituant soluble dans un solvant qui est représenté par B est fusionné en termes de cycle avec le substituant conjugué à électrons $\pi$ qui est représenté par A via des atomes qui sont présents sur le substituant conjugué à électrons $\pi$ qui est représenté par A, et C représente un substituant qui contient une structure qui est représentée par la Formule Générale (Ia) en tant qu'au moins une structure partielle ; et
dans lequel, dans les Formules Générales (I), (Ia) et (II), X et Y représentent chacun un atome d'hydrogène, un groupe éther substitué ou non substitué qui comporte de 1 à 38 atomes de carbone ou un groupe acyloxy substitué ou non substitué qui comporte de 1 à 38 atomes de carbone, où l'un de X et Y est le groupe éther ou acyloxy et l'autre est l'atome d'hydrogène ; $Q_2$ à $Q_5$ représentent chacun un atome d'hydrogène ou un groupe organique monovalent ; $Q_1$ et $Q_6$ représentent chacun un atome d'hydrogène, un atome d'halogène, un groupe alkyle, un groupe alkényle, un groupe alkynyle, un groupe aryle ou un groupe hétéroaryle ; et parmi les groupes organiques monovalents qui sont représentés par $Q_1$ à $Q_6$, des groupes organiques monovalents adjacents peuvent être liés ensemble pour former un cycle ;
et
dans lequel le groupe organique monovalent est un quelconque groupe parmi un groupe alkyle, un groupe alkényle, un groupe alkynyle, un groupe aryle et un groupe hétéroaryle, dans lequel le groupe hétéroaryle est sélectionné parmi un cycle thiophène, un cycle pyridine, un cycle pyrazine, un cycle pyrimidine, un cycle triazine, un cycle pyrrole, un cycle pyrazole, un cycle imidaziole, un cycle triazole, un cycle oxazole, un cycle thiazole et un cycle furane.

**2.** Procédé pour produire un produit du type film selon la revendication 1, dans lequel le substrat est revêtu du liquide de revêtement au moyen d'un procédé qui est sélectionné parmi le groupe qui est constitué par un revêtement par jet d'encre, un revêtement par centrifugation, une coulée d'une solution et un revêtement par immersion.

**3.** Procédé pour produire un produit du type film selon la revendication 1 ou la revendication 2, dans lequel le substituant qui est représenté par A est au moins un composé conjugué à électrons $\pi$ qui est sélectionné parmi le groupe qui est constitué par (i) des composés dans lesquels un ou plusieurs cycles hydrocarbone aromatiques sont fusionnés en termes de cycle avec un ou plusieurs cycles hétérocycliques aromatiques, des composés dans lesquels deux cycles hydrocarbone aromatiques ou plus sont fusionnés en termes de cycle ensemble et des composés dans lesquels deux cycles hétérocycliques aromatiques ou plus sont fusionnés en termes de cycle ensemble ; et (ii) des composés dans lesquels un ou plusieurs cycles hydrocarbone aromatiques sont liés via une liaison covalente avec un ou plusieurs cycles hétérocycliques aromatiques, des composés dans lesquels deux cycles hydrocarbone aromatiques ou plus sont liés ensemble via une liaison covalente et des composés dans lesquels deux cycles hétérocycliques aromatiques ou plus sont liés ensemble via une liaison covalente.

**4.** Procédé pour produire un produit du type film selon l'une quelconque des revendications 1 à 3, dans lequel le composant éliminé qui est représenté par la Formule Générale (II) et qui est éliminé du composé qui représenté par A-B(m) inclut un halogénure d'hydrogène, un acide carboxylique substitué ou non substitué, un alcool substitué ou non substitué ou du dioxyde de carbone.

**5.** Procédé pour produire un produit du type film selon l'une quelconque des revendications 1 à 4, dans lequel le composé qui est représenté par A-(B)m présente une solubilité dans un solvant et le composé qui est représenté par A-(C)m et qui est formé après élimination du substituant partant présente une non solubilité dans un solvant.

**6.** Procédé pour produire un produit du type film selon l'une quelconque des revendications 1 à 5, dans lequel le composé contenant le substituant partant et qui est représenté par la Formule Générale (I) ne présente pas la structure I-(11)

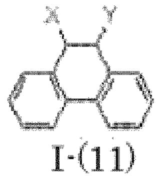

I-(11)

.

FIG. 1A

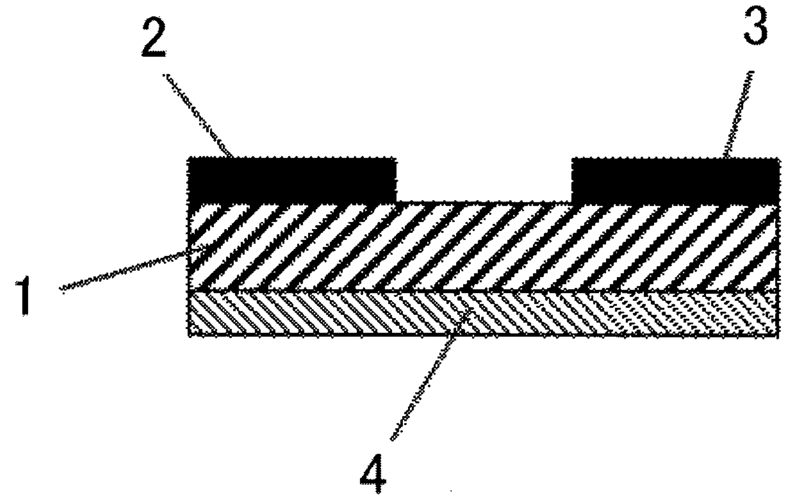


FIG. 1B

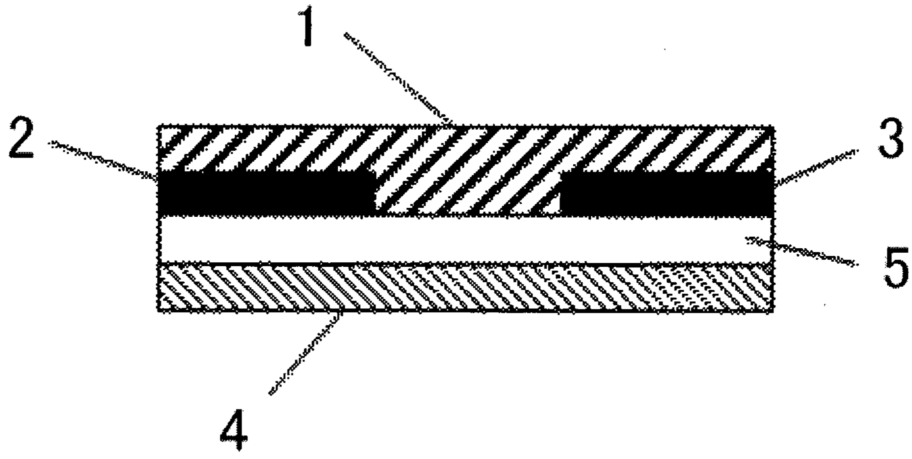

FIG. 1C

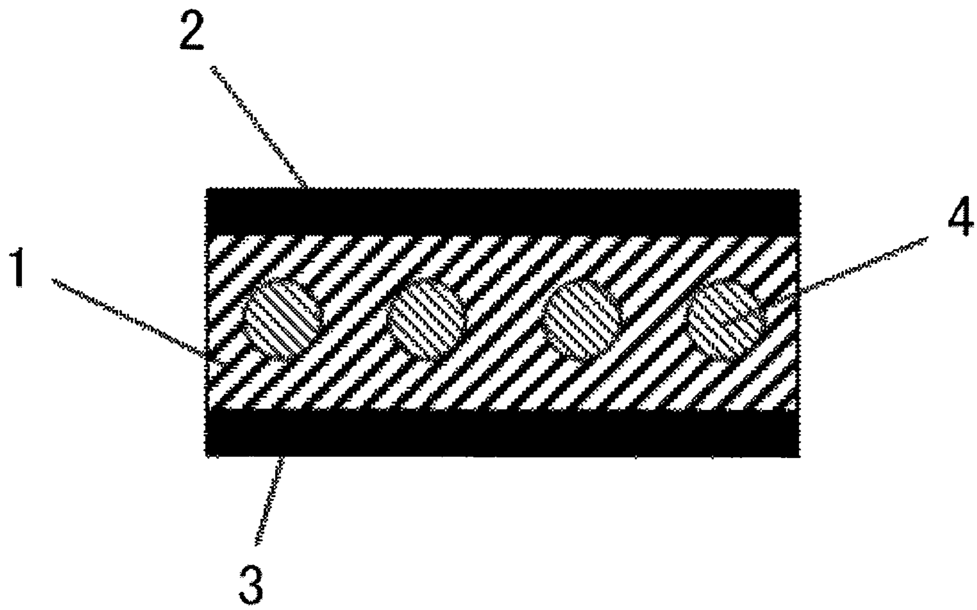


FIG. 1D

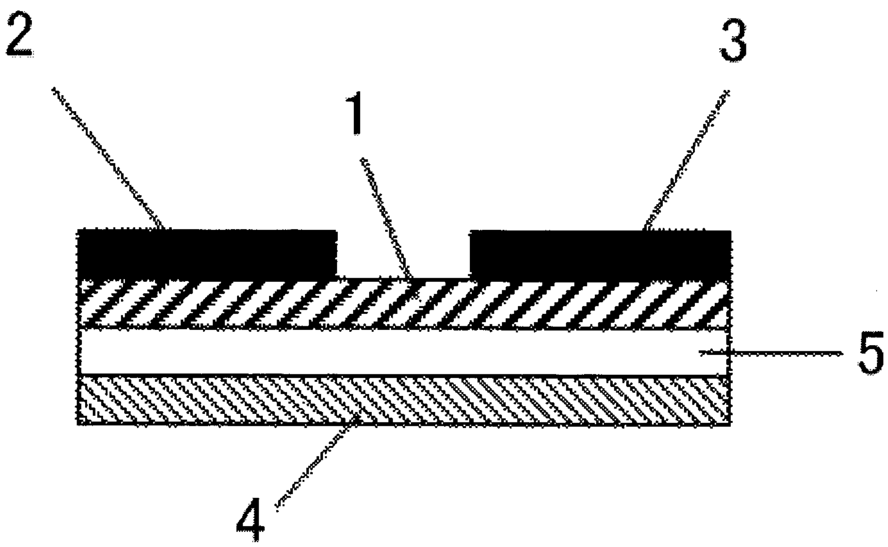

FIG. 2

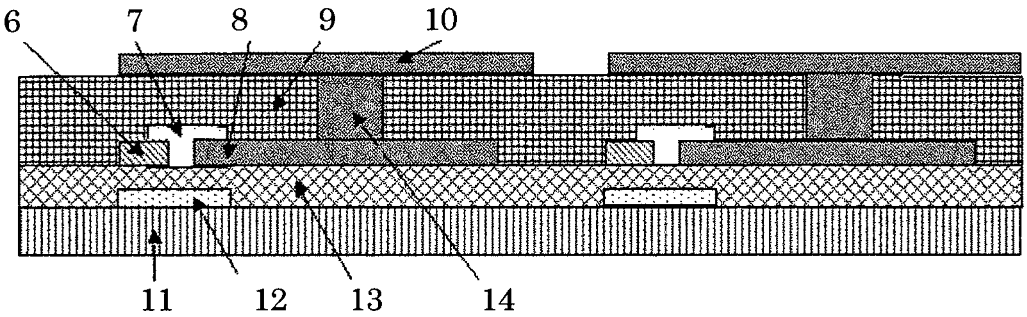


FIG. 3

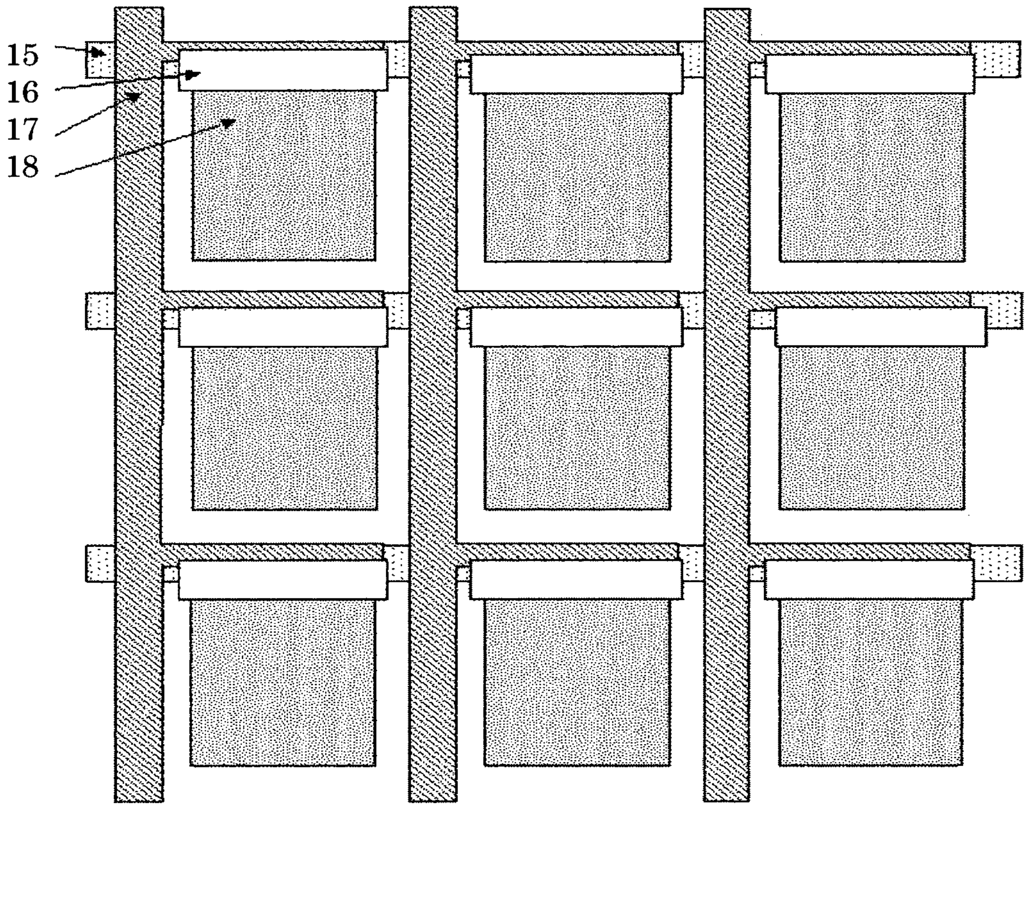

FIG. 4

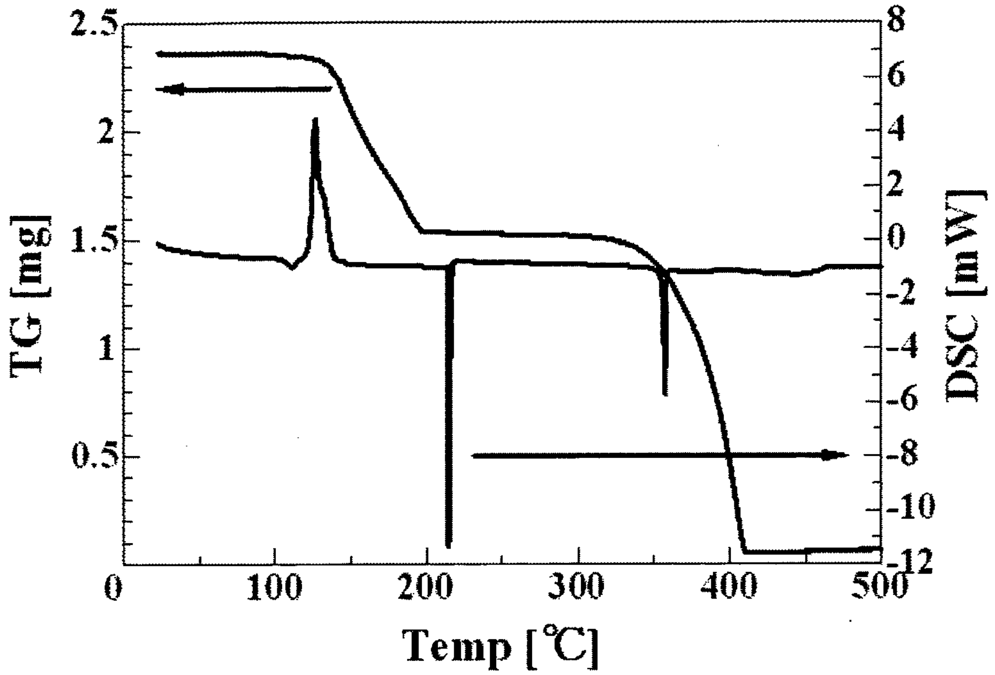
2.5
2
1.5
1
0.5
0
100
200
300
400
500
8
6
4
2
0
-2
-4
-6
-8
-10
-12
TG [mg]
DSC [mW]
Temp [℃]

FIG. 5

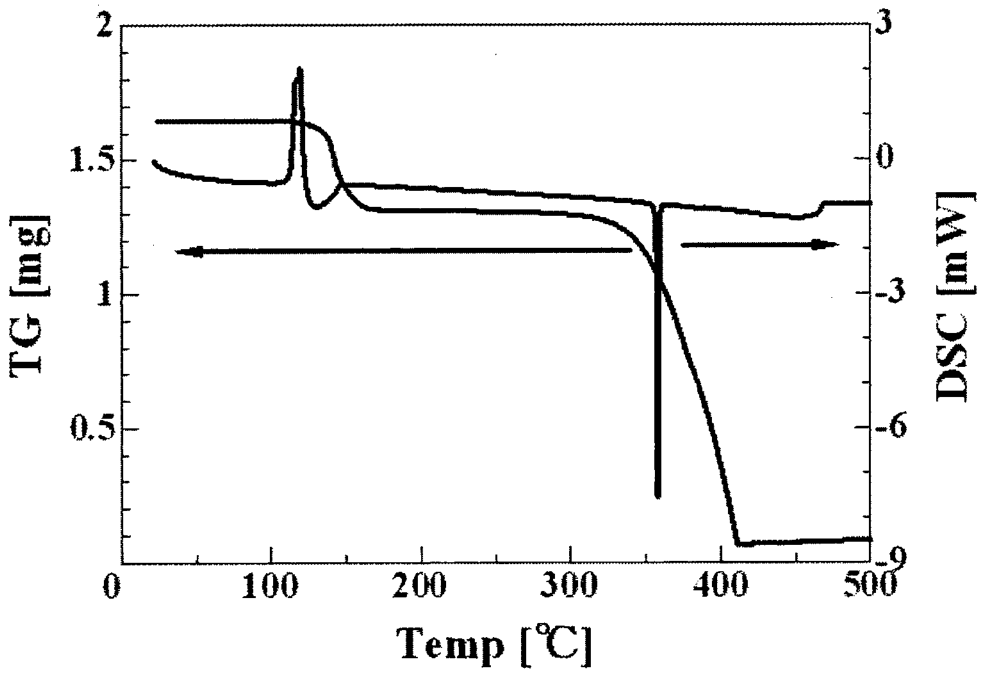
2
1.5
1
0.5
0
100
200
300
400
500
3
0
-3
-6
-9
TG [mg]
DSC [mW]
Temp [℃]

FIG. 6A

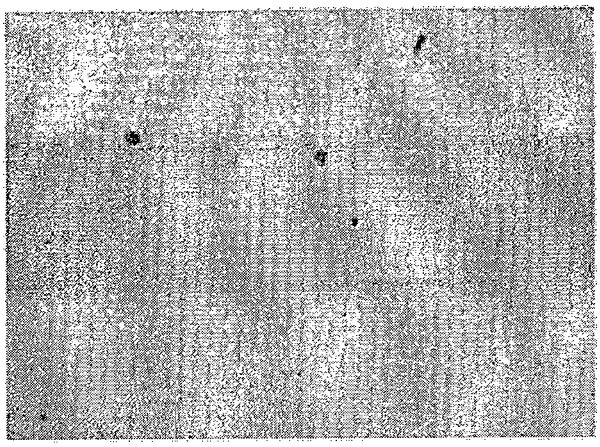

FIG. 6B

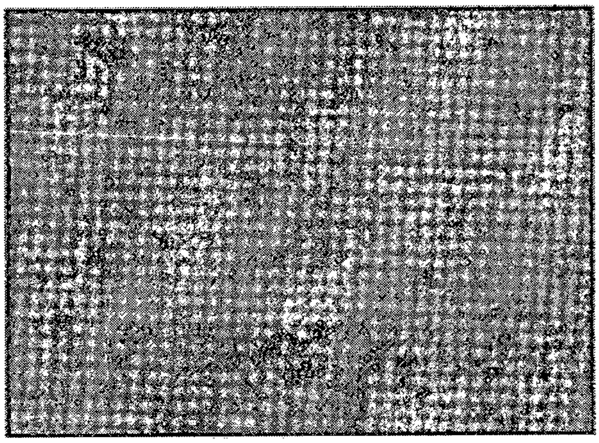

FIG. 7

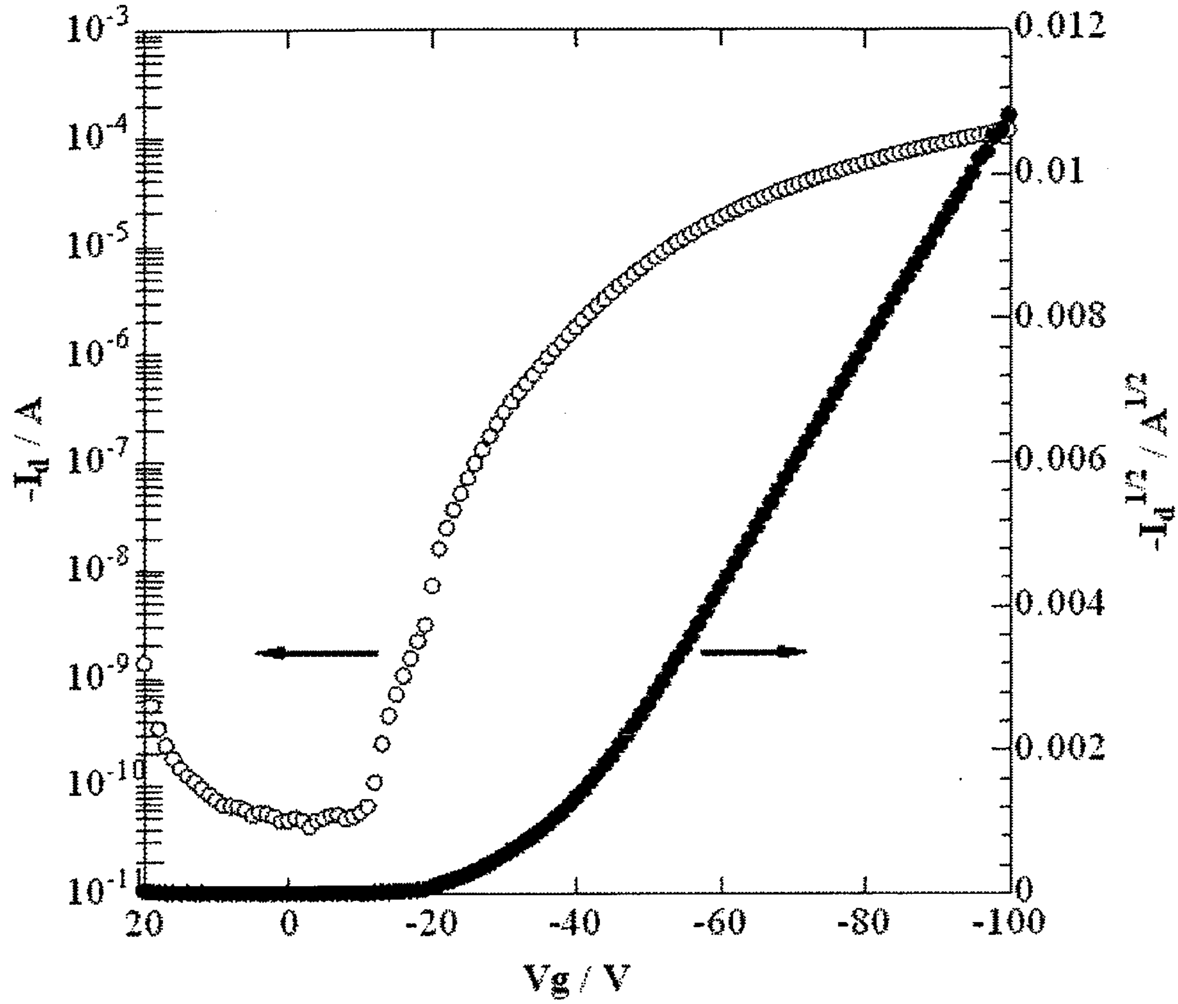

10-3
10-4
10-5
10-6
10-7
10-8
10-9
10-10
10-11
-Id / A
0.012
0.01
0.008
0.006
0.004
0.002
0
-Id1/2 / A1/2
20
0
-20
-40
-60
-80
-100
Vg / V

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2011030918 A
- JP 5055568 A
- WO 2006077888 A
- JP 2007224019 A
- JP 2008270843 A
- JP 2009105336 A
- JP 2009188386 A
- JP 2009215547 A
- JP 2009239293 A
- JP 2009283943 A
- JP 2009084555 A
- JP 2009088483 A
- JP 2006352143 A
- JP 2009275032 A
- JP 7188234 A
- JP 2008226959 A
- JP 2009302407 A


### Non-patent literature cited in the description


- *Appl. Phys. Lett.,* 1998, vol. 72, 1854
- *J. Am. Chem. Soc.,* 2006, vol. 128, 12604
- *J. Am. Chem. Soc.,* 2007, vol. 129, 15732
- *Adv. Mater.,* 1999, vol. 11, 480
- *J. Appl. Phys.,* 2006, vol. 100, 034502
- *Appl. Phys. Lett.,* 2004, vol. 84 (12), 2085
- *J. Am. Chem. Soc.,* 2004, vol. 126, 1596
- *Nature,* 1997, vol. 388, 131
- *J. Am. Chem. Soc.,* 2007, vol. 129, 2224-2225
- *J. Am. Chem. Soc.,* 2007, vol. 129, 15752
- **HIROFUSA SHIRAI ; NAGAO KOBAYASHI.** *Phthalocyanine -chemical and function,* 1997, 1-62
- **RYO HIROHASHI ; KEIICHI SAKAMOTO ; EIKO OKUMURA.** *Phthalocyanine as a functional dye,* 2004, 29-77
- *Advanced Materials,* 2009, vol. 21, 213-216
- *Chem. Mater.,* 2008, vol. 20 (20), 6289-6291